# EUROPÄISCHE PATENTSCHRIFT

(11) **EP 3 371 274 B1**
(45) Veröffentlichungstag und Bekanntmachung des Hinweises auf die Patenterteilung: **21.08.2019**
(21) Anmeldenummer: 16781677.6
(22) Anmeldetag: 11.10.2016
(51) Int. Cl.: C09K 11/06, H01L 51/00, C07D 405/14, C07D 409/14, C07D 487/04, C07D 491/048, C07D 495/04, H01L 51/50

(54) **MATERIALIEN FÜR ORGANISCHE ELEKTROLUMINESZENZVORRICHTUNGEN**
MATERIALS FOR ORGANIC ELECTROLUMINESCENT DEVICES
MATÉRIAUX POUR DISPOSITIFS ÉLECTROLUMINESCENTS ORGANIQUES

(30) Priorität: 02.11.2015 EP 15192535
(43) Veröffentlichungstag der Anmeldung: 12.09.2018
(73) Patentinhaber: Merck Patent GmbH, 64293 Darmstadt (DE)
(72) Erfinder: PARHAM, Amir Hossain, 60486 Frankfurt am Main (DE); LUDEMANN, Aurélie, 60322 Frankfurt am Main (DE); JOOSTEN, Dominik, 60487 Frankfurt am Main (DE); GROSSMANN, Tobias, 64297 Darmstadt (DE); KROEBER, Jonas Valentin, 60311 Frankfurt am Main (DE)
(86) Internationale Anmeldenummer: PCT/EP2016/001678
(87) Internationale Veröffentlichungsnummer: WO 2017/076485

(56) Entgegenhaltungen:
- WO-A1-2014/046494
- JP-A- 2009 263 579
- JP-A- 2010 135 467
- JP-A- 2012 049 518
- US-A1- 2009 167 162

## Beschreibung

Die vorliegende Erfindung beschreibt Carbazolylverbindungen, welche mit elektronenarmen Heteroarylgruppen substituiert sind, insbesondere zur Verwendung als Triplettmatrixmaterialien in organischen Elektrolumineszenzvorrichtungen. Die Erfindung betrifft ferner ein Verfahren zur Herstellung der erfindungsgemäßen Verbindungen sowie elektronische Vorrichtungen, enthaltend diese.

In organischen Elektrolumineszenzvorrichtungen (OLEDs) werden als emittierende Materialien häufig metallorganische Komplexe eingesetzt, die Phosphoreszenz zeigen. Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, insbesondere auch bei OLEDs, die Phosphoreszenz zeigen, immer noch Verbesserungsbedarf, beispielsweise im Hinblick auf Effizienz, Betriebsspannung und Lebensdauer.

Die Eigenschaften phosphoreszierender OLEDs werden nicht nur von den eingesetzten Triplettemittern bestimmt. Hier sind insbesondere auch die anderen verwendeten Materialien wie zum Beispiel Matrixmaterialien von besonderer Bedeutung. Verbesserungen dieser Materialien können somit auch zu deutlichen Verbesserungen der OLED-Eigenschaften führen. Gemäß dem Stand der Technik werden unter anderem Carbazolderivate und Dibenzofuranderivate als Matrixmaterialien für phosphoreszierende Emitter in organischen Elektrolumineszenzvorrichtungen eingesetzt. Aus JP 2012-049518, US 7,935,434 und US 8,221,908 sind Dibenzofuranderivate bekannt, die mit zwei N-Phenylcarbazolylgruppen substituiert sind. Aus WO 2014/081206 sind Verbindungen bekannt, in denen zwei Carbazolylgruppen, von denen eine am Stickstoffatom mit einer elektronenarmen Heteroarylgruppe substituiert sind, über eine Arylengruppe miteinander verknüpft sind. Aus JP2012049518 und JP2009263579 sind Verbindungen bekannt, die Formel (1) aus Anspruch 1 ähnlich sind.

Generell besteht bei diesen Materialien für die Verwendung als Matrixmaterialien noch Verbesserungsbedarf, unter anderem in Bezug auf die externe Quanteneffizienz (EQE). Aufgabe der vorliegenden Erfindung ist daher die Bereitstellung von Verbindungen, welche sich für den Einsatz in einer phosphoreszierenden oder fluoreszierenden OLED eignen, insbesondere als Matrixmaterial. Insbesondere ist es die Aufgabe der vorliegenden Erfindung, Matrixmaterialien bereitzustellen, welche sich für rot, gelb und grün phosphoreszierende OLEDs und gegebenenfalls auch für blau phosphoreszierende OLEDs eignen und die zu hoher Lebensdauer, guter Effizienz und geringer Betriebsspannung führen. Insbesondere ist es die Aufgabe der vorliegenden Erfindung, Materialien bereitzustellen, die zu einer verbesserten externen Quanteneffizienz führen.

Es wurde überraschend gefunden, dass Elektrolumineszenzvorrichtungen, die Verbindungen gemäß der folgenden Formel (1) enthalten, Verbesserungen gegenüber dem Stand der Technik aufweisen, insbesondere beim Einsatz als Matrixmaterial für phosphoreszierende Dotanden.

Gegenstand der vorliegenden Erfindung ist daher eine Verbindung gemäß der folgenden Formel (1), 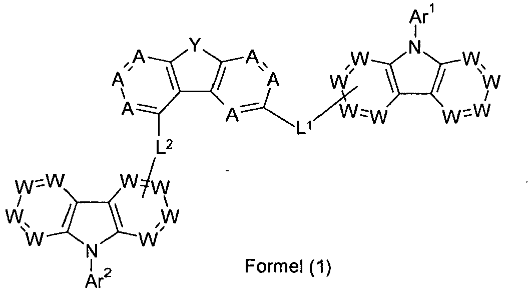 wobei für die verwendeten Symbole gilt:
- A: ist bei jedem Auftreten gleich oder verschieden CR oder N, wobei maximal zwei Gruppen A pro Cyclus, bevorzugt maximal eine Gruppe A pro Cyclus, für N stehen; besonders bevorzugt stehen alle Gruppen A für CR;
- Y: ist O oder S;
- W: ist bei jedem Auftreten gleich oder verschieden CR oder N, wobei maximal zwei Gruppen W pro Cyclus für N stehen und wobei W für C steht, wenn an diese Position eine Gruppe L¹ bzw. L² gebunden ist, oder zwei benachbarte Gruppen W stehen zusammen für eine Gruppe der folgenden Formel (2) oder (3), wobei jede der beiden Carbazolylderivat-Gruppen in der Verbindung der Formel (1) maximal zwei Gruppen der Formel (2) bzw. Formel (3) aufweist, 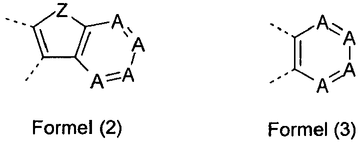 wobei die gestrichelten Bindungen die Verknüpfung dieser Gruppe andeuten, A die oben genannten Bedeutungen aufweist und Z für NR, CR₂, O oder S steht;
- Ar¹, Ar²: ist ein aromatisches Ringsystem mit 5 bis 30 aromatischen Ringatomen oder eine Dibenzofuran- oder Dibenzothiophengruppe, wobei das aromatische Ringsystem bzw. die Dibenzofuran- oder Dibenzothiophengruppe jeweils durch einen oder mehrere nichtaromatische Reste R substituiert sein kann, oder ist eine Gruppe gemäß einer der folgenden Formeln (4) oder (5), 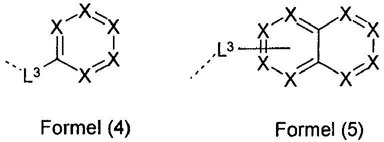 wobei die gestrichelte Bindung die Bindung an das Stickstoffatom darstellt;
mit der Maßgabe, dass genau eine der Gruppen Ar¹ oder Ar² für eine Gruppe gemäß einer der Formeln (4) oder (5) steht;
- X: ist bei jedem Auftreten gleich oder verschieden CR oder N, wobei X in Formel (5) für C steht, wenn die Gruppe der Formel (5) in dieser Position mit L³ verknüpft ist, mit der Maßgabe, dass in Formel (4) zwei oder drei Gruppen X für N stehen und in Formel (5) eine, zwei oder drei Gruppen X für N stehen;
- L¹, L², L³: ist bei jedem Auftreten gleich oder verschieden eine Einfachbindung oder ein aromatisches oder heteroaromatisches Ringsystem mit 5 bis 30 aromatischen Ringatomen, das durch einen oder mehrere Reste R substituiert sein kann;
- R: ist bei jedem Auftreten gleich oder verschieden ausgewählt aus der Gruppe bestehend aus H, D, F, Cl, Br, I, CN, NO₂, N(R¹)₂, C(=O)R¹, P(=O)(R¹)₂ P(R¹)₂, B(R¹)₂, Si(R¹)₃, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R¹ substituiert sein kann, wobei eine oder mehrere nicht-benachbarte CH₂-Gruppen durch R¹C=CR¹, Si(R¹)₂, C=O, C=S, C=NR¹, P(=O)(R¹), SO, SO₂, NR1, O, S oder CONR¹ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R¹ substituiert sein kann, oder einer Aryloxy- oder Heteroaryloxygruppe mit 5 bis 40 aromatischen Ringatomen, die mit einem oder mehreren Resten R¹ substituiert sein kann; dabei können optional zwei Substituenten R, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten R¹ substituiert sein kann;
- R¹: ist bei jedem Auftreten gleich oder verschieden ausgewählt aus der Gruppe bestehend aus H, D, F, Cl, Br, I, CN, N(R²)₂, C(=O)R¹, einer geradkettigen Alkylgruppe mit 1 bis 10 C-Atomen oder einer verzweigten oder cyclischen Alkylgruppe mit 3 bis 10 C-Atomen oder einer Alkenylgruppe mit 2 bis 10 C-Atomen, die jeweils mit einem oder mehreren Resten R² substituiert sein kann, wobei ein oder mehrere H-Atome durch D, F, Cl, Br, I oder CN ersetzt sein können, oder einem aromatischen oder heteroaromatischen Ringsystem mit 5 bis 40 aromatischen Ringatomen, das jeweils mit einem oder mehreren Resten R² substituiert sein kann; dabei können optional zwei Substituenten R¹, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten R² substituiert sein kann;
- R²: ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, CN ersetzt sein können und das durch ein oder mehrere Alkylgruppen mit jeweils 1 bis 4 Kohlenstoffatomen substituiert sein kann; dabei können zwei oder mehr benachbarte Substituenten R² miteinander ein mono- oder polycyclisches, aliphatisches Ringsystem bilden.

Benachbarte Kohlenstoffatome im Sinne der vorliegenden Erfindung sind Kohlenstoffatome, die direkt miteinander verknüpft sind.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

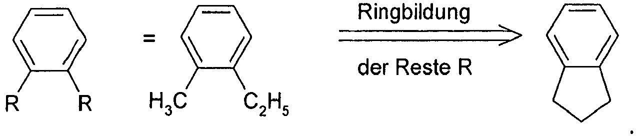

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

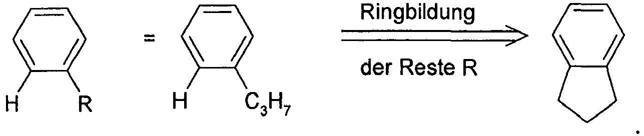

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S, wobei die Heteroarylgruppe bevorzugt nicht mehr als drei Heteroatome enthält. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Eine kondensierte Arylgruppe im Sinne der vorliegenden Erfindung ist eine Gruppe, in der zwei oder mehr aromatische Gruppen über eine gemeinsame Kante aneinander ankondensiert, d. h. anelliert, sind, wie beispielsweise im Naphthalin. Dagegen ist beispielsweise Fluoren keine kondensierte Arylgruppe im Sinne der vorliegenden Erfindung, da im Fluoren die beiden aromatischen Gruppen keine gemeinsame Kante aufweisen.

Ein aromatisches Ringsystem im Sinne dieser Erfindung enthält 6 bis 40 C-Atome im Ringsystem. Ein heteroaromatisches Ringsystem im Sinne dieser Erfindung enthält 1 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S, wobei das heteroaromatische Ringsystem bevorzugt nicht mehr als vier Heteroatome, besonders bevorzugt nicht mehr als drei Heteroatome enthält. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe unterbrochen sind. Weiterhin sollen Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer C₁- bis C₂₀-Alkylgruppe, in der auch einzelne H-Atome oder CH₂-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden: Unter einer Alkinylgruppe werden beispielsweise Ethinyl, Propinyl, Butinyl, Pentinyl, Hexinyl, Heptinyl oder Octinyl verstanden. Unter einer C₁- bis C₄₀-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 - 40 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Bevorzugt sind die Verbindungen der folgenden Formel (1a), 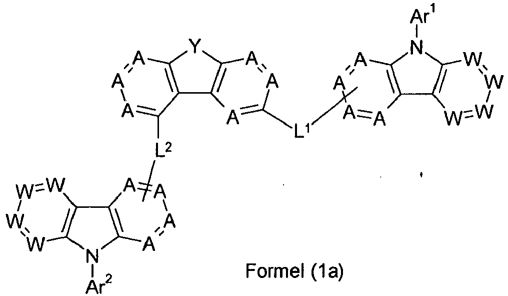 wobei die verwendeten Symbole die oben genannten Bedeutungen aufweisen und A für C steht, wenn an diese Position eine Gruppe L¹ bzw L² gebunden ist.

In einer bevorzugten Ausführungsform der Erfindung steht die Gruppe L² für eine Einfachbindung. Eine bevorzugte Ausführungsform der Verbindung der Formel (1) ist also eine Verbindung der folgenden Formel (6), und eine bevorzugte Ausführungsform der Verbindung der Formel (1a) ist eine Verbindung der Formel (6a), 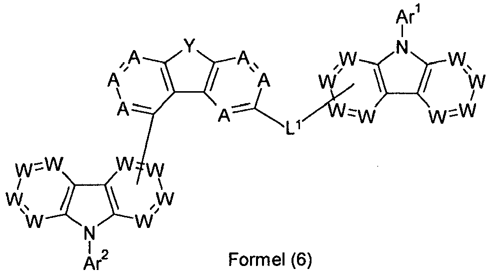 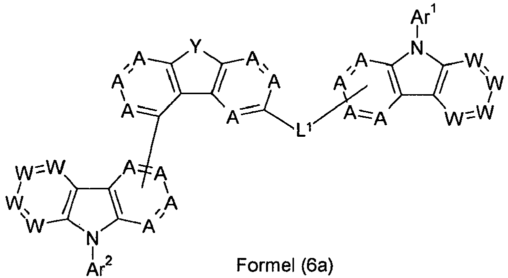 wobei die verwendeten Symbole die oben genannten Bedeutungen aufweisen.

In einer bevorzugten Ausführungsform der Erfindung steht W gleich oder verschieden bei jedem Auftreten für CR oder zwei W stehen für eine Gruppe der Formel (2a) oder (3a) und die verbleibenden W stehen für CR, und A steht gleich oder verschieden bei jedem Auftreten für CR. Bevorzugt sind also die Verbindungen der folgenden Formeln (7a), (7b), (7c), (7d) bzw. (7e), 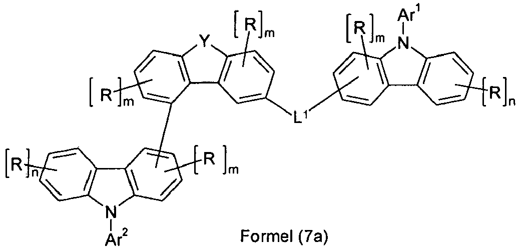 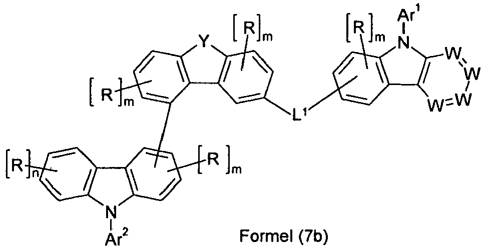 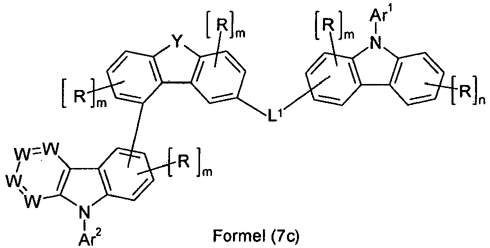 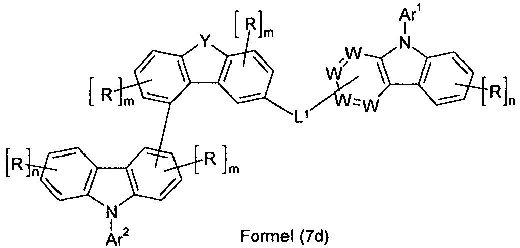 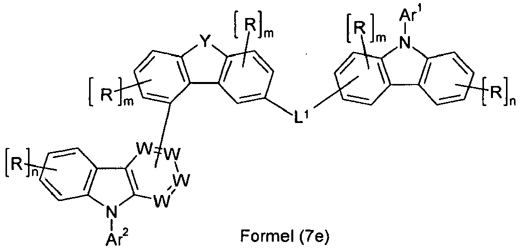 wobei gilt:
- W: zwei benachbarte Gruppen W stehen zusammen für eine Gruppe der folgenden Formel (2a) oder (3a) und die anderen beiden Gruppen W stehen für CR und bevorzugt für CH, wobei W in Formel (7d) für C steht, wenn an diese Position eine Gruppe L¹ gebunden ist, und in Formel (7e) für C steht, wenn an diese Position das Dibenzofuran bzw. Dibenzothiophen gebunden ist, 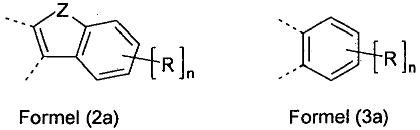 wobei die gestrichelten Bindungen die Verknüpfung dieser Gruppe andeuten;
- n: ist bei jedem Auftreten gleich oder verschieden 0, 1, 2, 3 oder 4;
- m: ist bei jedem Auftreten gleich oder verschieden 0, 1, 2 oder 3;
die weiteren verwendeten Symbole weisen die oben genannten Bedeutungen auf.

In einer weiteren bevorzugten Ausführungsform der Erfindung stehen L¹ und L² beide für eine Einfachbindung. Bevorzugt sind somit die Verbindungen der folgenden Formeln (8a), (8b), (8c), (8d) und (8e), 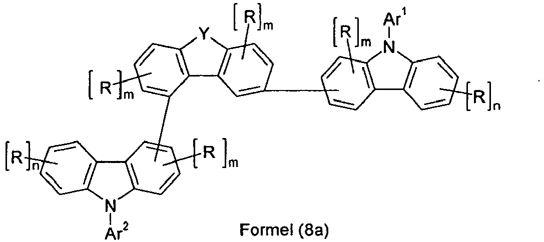 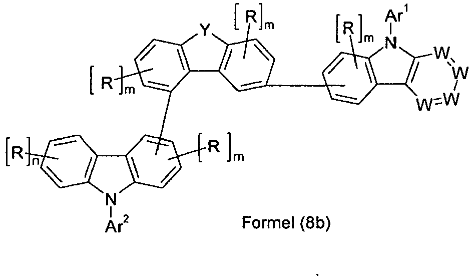 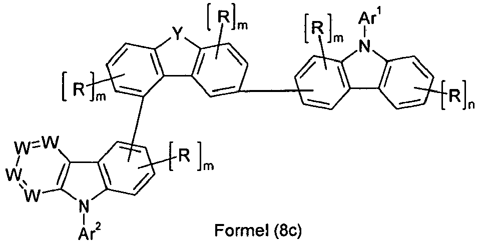 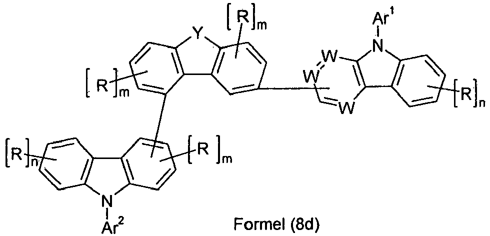 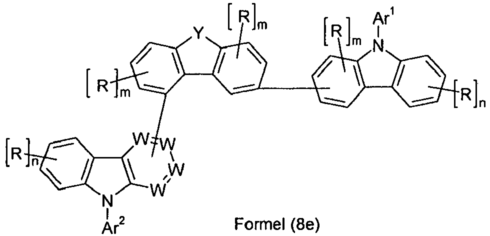 wobei die verwendeten Symbole und Indizes die oben genannten Bedeutungen aufweisen.

In einer besonders bevorzugten Ausführungsform der Erfindung erfolgt die Verknüpfung der beiden Carbazolgruppen bzw. Carbazolderivate über die 3-Position, d. h. über die Position para zu den Stickstoffatomen. Besonders bevorzugt sind also die Verbindungen der folgenden Formeln (9a), (9b), (9c), (9d) und (9e), 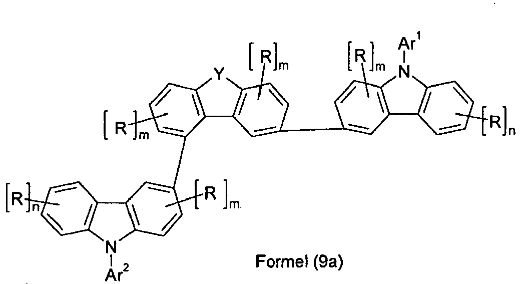 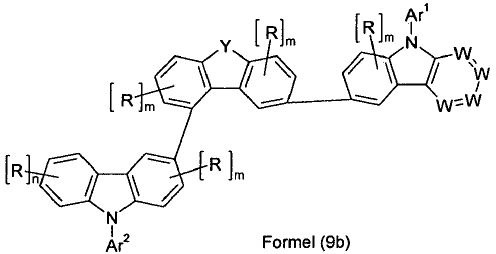 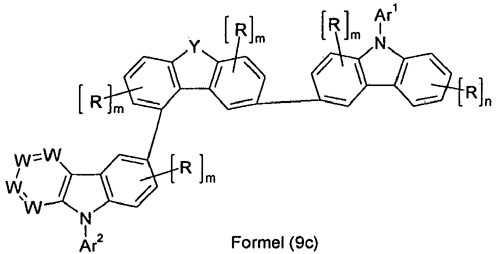 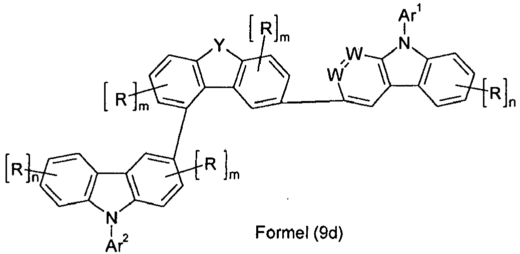 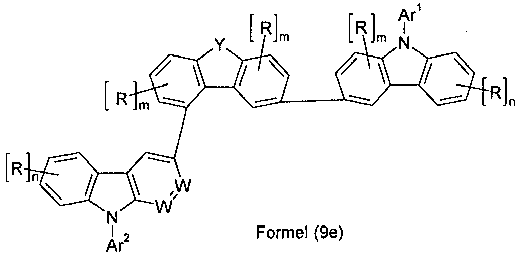 wobei die verwendeten Symbole und Indizes die oben genannten Bedeutungen aufweisen.

Ganz besonders bevorzugt sind Verbindungen, in denen alle Gruppen W für CR bzw. C stehen, also die Verbindungen der Formeln (7a), (8a) und (9a).

Bevorzugt sind weiterhin Verbindungen gemäß den oben genannten Formeln, in denen Y für O steht.

In einer weiteren bevorzugten Ausführungsform der Erfindung steht Z, falls die Verbindung eine Gruppe der Formel (2) enthält, für O, NR, wobei der an den Stickstoff gebundene Rest R ungleich H ist, oder C(R)₂, besonders bevorzugt für NR, wobei der an den Stickstoff gebundene Rest R ungleich H ist, oder C(R)₂ und ganz besonders bevorzugt für C(R)₂.

In einer bevorzugten Ausführungsform der Erfindung enthält jede der Carbazolylderivatgruppen maximal eine Gruppe der Formel (2) bzw. Formel (3).

Wenn die erfindungsgemäße Verbindung eine Gruppe der Formel (2) enthält, so kann diese in verschiedenen Positionen gebunden sein. Dies wird im Folgenden schematisch anhand von bevorzugten Ausführungsformen, in denen die Gruppen A und die anderen Gruppen W für CR stehen, durch die Formeln (A) bis (F) dargestellt: 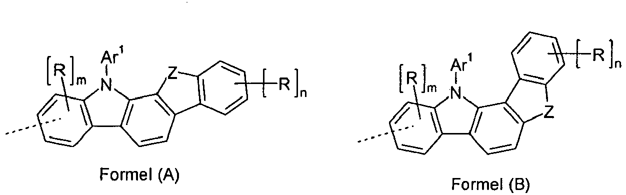 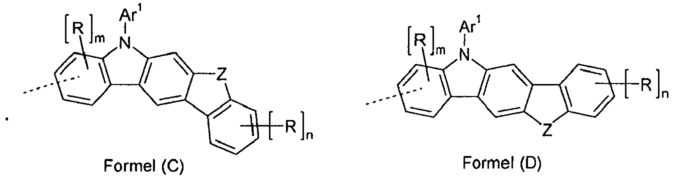 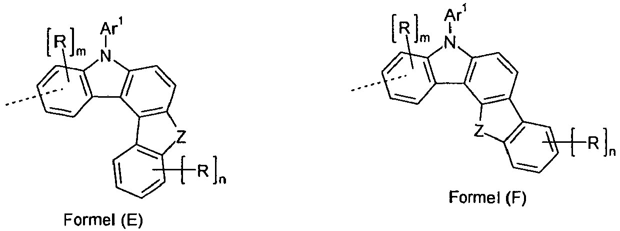 wobei die verwendeten Symbole und Indizes die oben genannten Bedeutungen aufweisen und die gestrichelte Bindung die Verknüpfung in der erfindungsgemäßen Verbindung darstellt. Entsprechendes gilt für das andere Carbazolderivat, welches statt der Gruppe Ar¹ eine Gruppe Ar² am Stickstoff gebunden enthält.

Wenn die erfindungsgemäße Verbindung eine Gruppe der Formel (3) enthält, so kann diese in verschiedenen Positionen gebunden sein. Dies wird im Folgenden schematisch anhand von bevorzugten Ausführungsformen, in denen die Gruppen A und die anderen Gruppen W für CR stehen, durch die Formeln (G) bis (K) dargestellt: 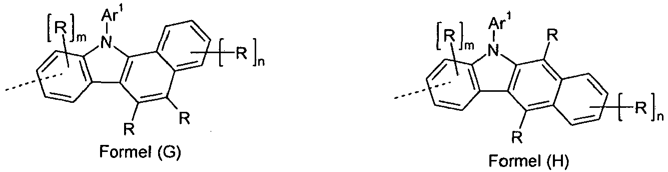 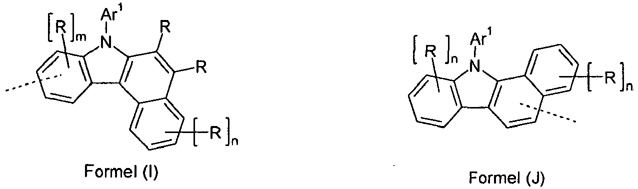 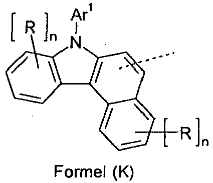 wobei die verwendeten Symbole und Indizes die oben genannten Bedeutungen aufweisen und die gestrichelte Bindung die Verknüpfung in der erfindungsgemäßen Verbindung darstellt. Entsprechendes gilt für das andere Carbazolderivat, welches eine Gruppe Ar² gebunden enthält.

Im Folgenden werden bevorzugte Ausführungsformen für die Gruppen Ar¹ und Ar² beschrieben. Wie oben beschrieben, steht eine der Gruppen Ar¹ und Ar² für ein aromatisches Ringsystem mit 5 bis 30 aromatischen Ringatomen oder für eine Dibenzofuran- oder Dibenzothiophengruppe, welche jeweils durch einen oder mehrere nicht-aromatische Reste R substituiert sein können, und die andere der Gruppen Ar¹ und Ar² steht für eine Heteroarylgruppe gemäß einer der Formeln (4) oder (5).

In einer Ausführungsform der Verbindungen gemäß Formel (1) bzw. (1a) bzw. den Verbindungen gemäß Formel (6), (7a) bis (7e), (8a) bis (8e) und (9a) bis (9e) steht Ar¹ für ein aromatisches Ringsystem mit 5 bis 30 aromatischen Ringatomen oder eine Dibenzofuran- oder Dibenzothiophengruppe, wobei das aromatische Ringsystem bzw. die Dibenzofuran- oder Dibenzothiophengruppe jeweils durch einen oder mehrere nicht-aromatische Reste R substituiert sein kann, und Ar² steht für eine Heteroarylgruppe gemäß einer der Formeln (4) oder (5).

In einer weiteren Ausführungsform der Verbindungen gemäß Formel (1) bzw. (1a) bzw. den Verbindungen gemäß Formel (6), (7a) bis (7e), (8a) bis (8e) und (9a) bis (9e) steht Ar² für ein aromatisches Ringsystem mit 5 bis 30 aromatischen Ringatomen oder eine Dibenzofuran- oder Dibenzothiophengruppe, wobei das aromatische Ringsystem bzw. die Dibenzofuran- oder Dibenzothiophengruppe jeweils durch einen oder mehrere nicht-aromatische Reste R substituiert sein kann, und Ar¹ steht für eine Heteroarylgruppe gemäß einer der Formeln (4) oder (5).

In einer bevorzugten Ausführungsform der Erfindung ist die Gruppe der Formel (4) ausgewählt aus den Strukturen der folgenden Formeln (4-1) bis (4-3), 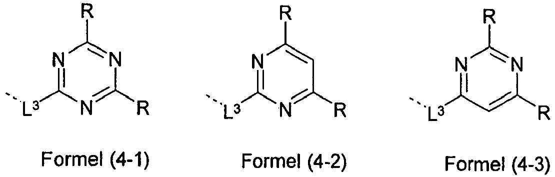 wobei die gestrichelte Bindung die Bindung an das Stickstoffatom darstellt und weiterhin gilt:
- R: ist bei jedem Auftreten gleich oder verschieden H, eine Alkylgruppe mit 1 bis 10 C-Atomen, welche mit einem oder mehreren Resten R¹ substituiert sein kann, bevorzugt aber unsubstituiert ist, oder ein aromatisches oder heteroaromatisches Ringsystem mit 5 bis 40 aromatischen Ringatomen, das jeweils mit einem oder mehreren Resten R¹ substituiert sein kann, bevorzugt aber unsubstituiert ist; bevorzugt ist R ungleich H.

In einer besonders bevorzugten Ausführungsform der Erfindung ist die Gruppe der Formel (4) ausgewählt aus den Strukturen der folgenden Formeln (4-1a) bis (4-3a), 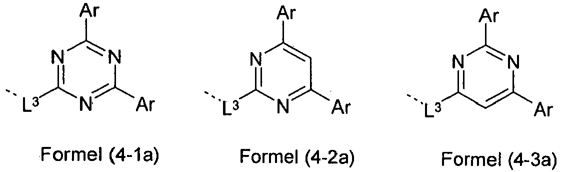 wobei die gestrichelte Bindung die Bindung an das Stickstoffatom darstellt und weiterhin gilt:
- Ar: ist bei jedem Auftreten gleich oder verschieden ein aromatisches oder heteroaromatisches Ringsystem mit 5 bis 40 aromatischen Ringatomen, das jeweils mit einem oder mehreren Resten R¹ substituiert sein kann, bevorzugt aber unsubstituiert ist.

In einer bevorzugten Ausführungsform der Gruppe der Formel (5) stehen ein oder zwei Symbole X für N, besonders bevorzugt zwei Symbole X.

Bevorzugt ist die Gruppe der Formel (5) ausgewählt aus den Strukturen der folgenden Formeln (5-1) bis (5-18), 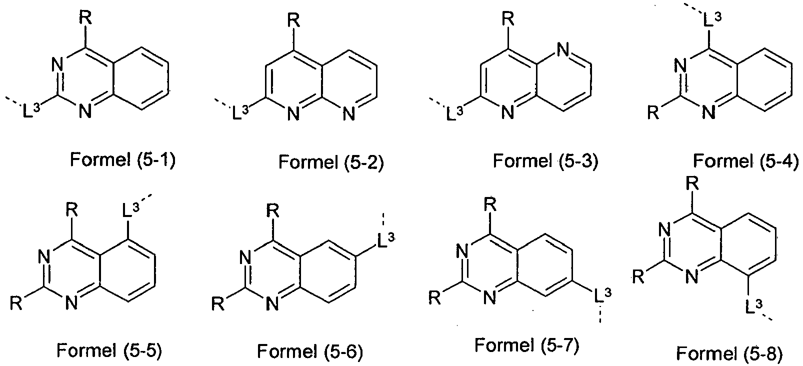 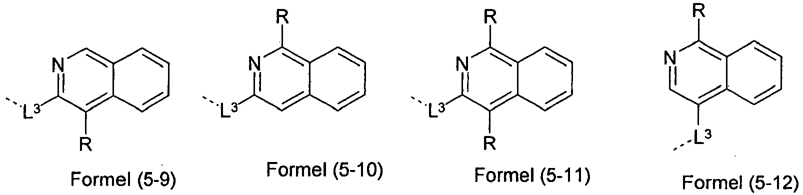 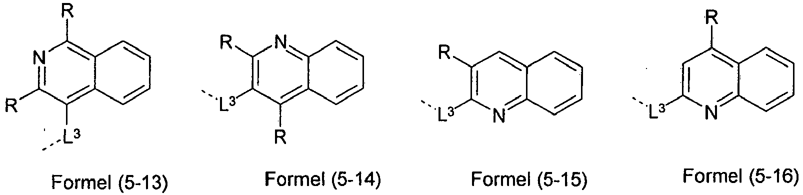 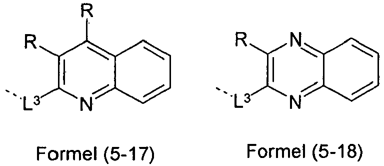 wobei die gestrichelte Bindung die Bindung an das Stickstoffatom darstellt und weiterhin gilt:
- R: ist bei jedem Auftreten gleich oder verschieden H, eine Alkylgruppe mit 1 bis 10 C-Atomen, welche mit einem oder mehreren Resten R¹ substituiert sein kann, bevorzugt aber unsubstituiert ist, oder ein aromatisches oder heteroaromatisches Ringsystem mit 5 bis 40 aromatischen Ringatomen, das jeweils mit einem oder mehreren Resten R¹ substituiert sein kann, bevorzugt aber unsubstituiert ist; bevorzugt ist R ungleich H.

Besonders bevorzugt ist die Gruppe der Formel (5) ausgewählt aus den Strukturen der folgenden Formeln (5-1a) bis (5-18a), 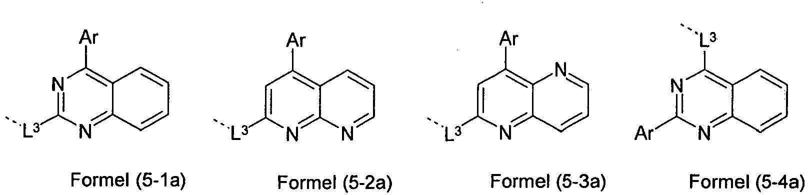 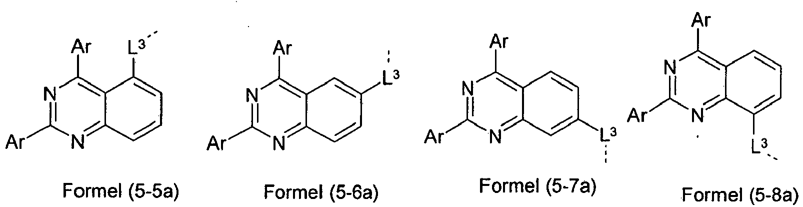 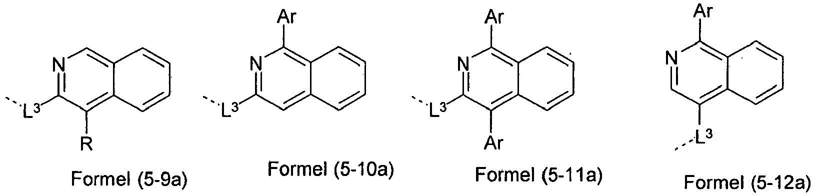 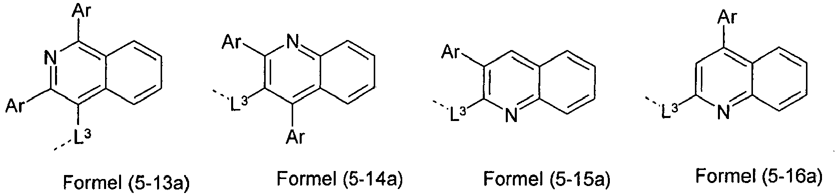 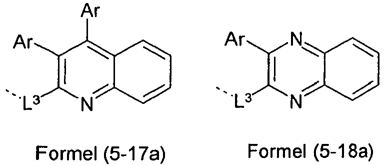 wobei die gestrichelte Bindung die Bindung an das Stickstoffatom darstellt und Ar die oben genannten Bedeutungen aufweist.

In einer bevorzugten Ausführungsform der Erfindung steht L³ gleich oder verschieden bei jedem Auftreten für eine Einfachbindung oder für ein aromatisches Ringsystem mit 6 bis 12 aromatischen Ringatomen, besonders bevorzugt für eine Einfachbindung oder para- oder meta-Phenylen und ganz besonders bevorzugt für eine Einfachbindung.

In einer weiteren bevorzugten Ausführungsform der Erfindung steht Ar in den Gruppen der Formeln (4-1a) bis (4-3a) und (5-1a) bis (5-18a) gleich oder verschieden bei jedem Auftreten für ein aromatisches oder heteroaromatisches Ringsystem mit 6 bis 24 aromatischen Ringatomen, bevorzugt mit 6 bis 18 aromatischen Ringatomen, besonders bevorzugt für ein aromatisches Ringsystem mit 6 bis 12 aromatischen Ringatomen bzw. ein heteroaromatisches Ringsystem mit 6 bis 13 aromatischen Ringatomen, das jeweils durch einen oder mehrere Reste R¹ substituiert sein kann, bevorzugt aber unsubstituiert ist. Beispiele für geeignete Gruppen Ar sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste R¹ substituiert sein können, bevorzugt aber unsubstituiert sind.

Beispiele für geeignete Gruppen Ar sind die im Folgenden aufgeführten Strukturen Ar-1 bis Ar-19, 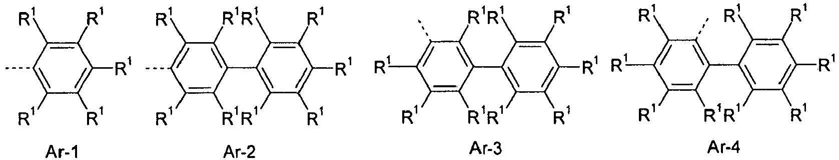 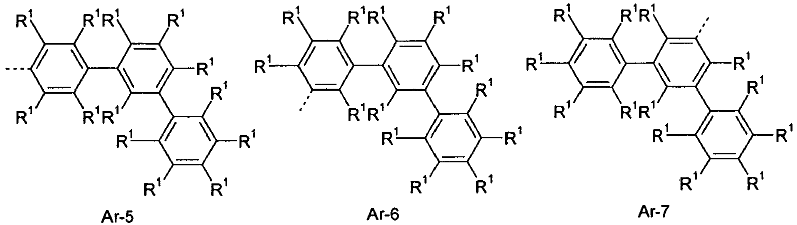 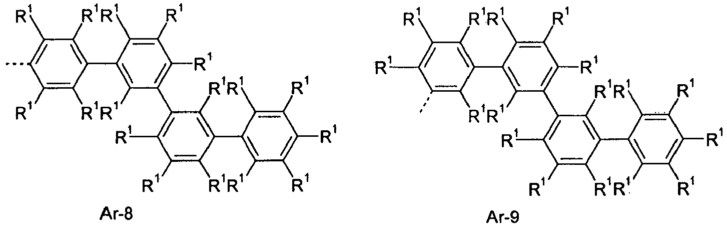 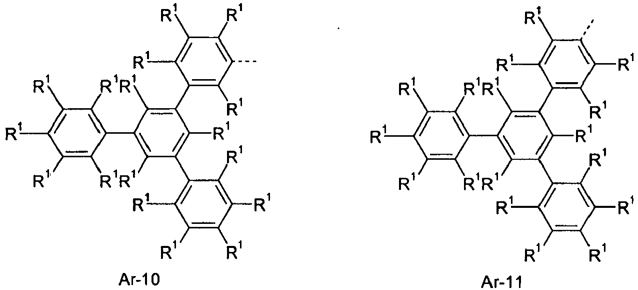 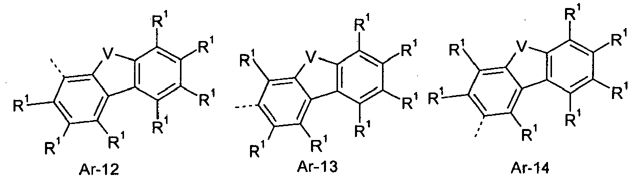 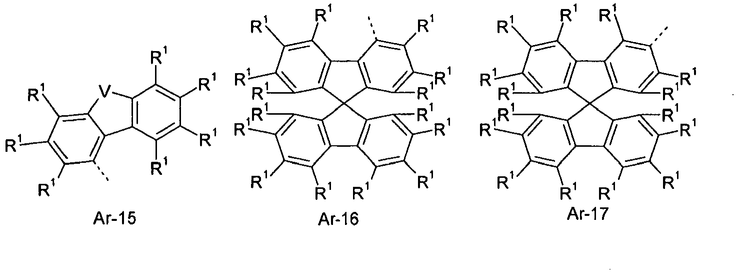 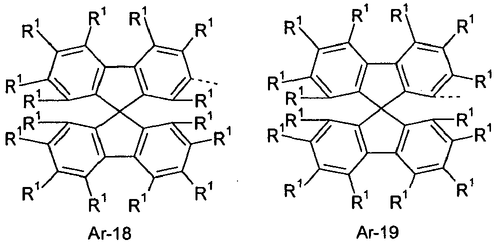 wobei R¹ die oben genannten Bedeutungen aufweist und bevorzugt für H steht, die gestrichelte Bindung die Anknüpfung dieser Gruppe darstellt und V für NR1, O, S oder C(R¹)₂ steht.

In einer weiteren bevorzugten Ausführungsform der Erfindung steht die Gruppe Ar¹ bzw. Ar², die nicht für eine Gruppe der Formel (4) oder (5) steht, für ein aromatisches Ringsystem mit 6 bis 24 aromatischen Ringatomen, bevorzugt mit 6 bis 18 aromatischen Ringatomen, besonders bevorzugt mit 6 bis 12 aromatischen Ringatomen bzw. für eine Dibenzofuran- bzw. Dibenzothiophengruppe, wobei diese Gruppen jeweils durch einen oder mehrere nicht-aromatische Reste R substituiert sein können, bevorzugt aber unsubstituiert sind. Beispiele für geeignete Gruppen Ar¹ bzw. Ar², die nicht für ene Gruppe der Formel (4) oder (5) stehen, sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, 1-, 2-, 3- oder 4-Dibenzofuranyl und 1-, 2-, 3- oder 4-Dibenzothienyl, die jeweils durch einen oder mehrere nicht-aromatische Reste R substituiert sein können, bevorzugt aber unsubstituiert sind.

Beispiele für geeignete Gruppen Ar¹ bzw. Ar² sind die im Folgenden aufgeführten Strukturen Ar¹-1 bis Ar¹-19 bzw. Ar²-1 bis Ar²-19, 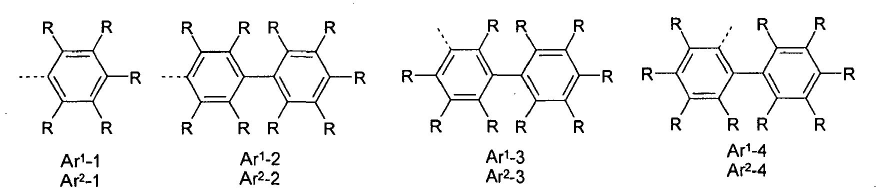 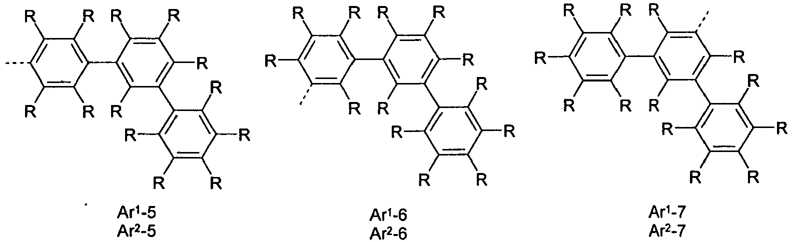 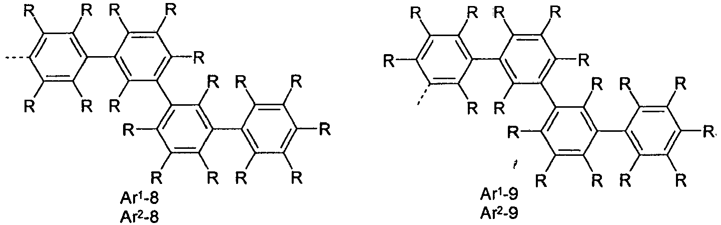 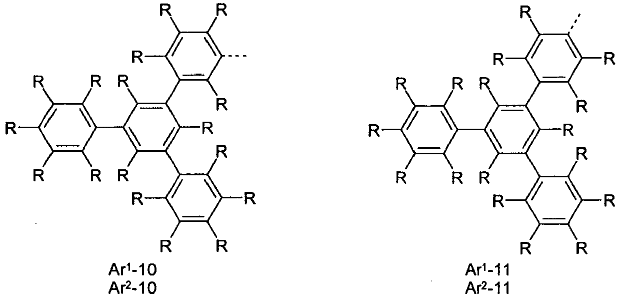 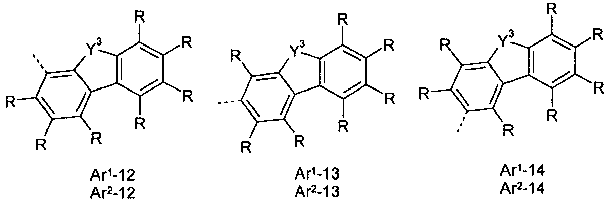 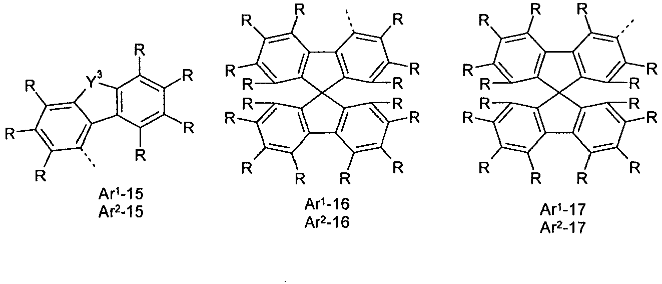 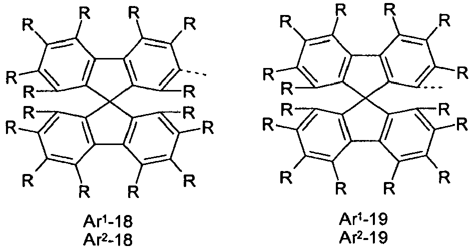 wobei R die oben genannten Bedeutungen aufweist und bevorzugt für H steht, die gestrichelte Bindung die Bindung an das Stickstoffatom darstellt und Y³ gleich oder verschieden bei jedem Auftreten für CR₂, O oder S steht.

In einer weiteren bevorzugten Ausführungsform der Erfindung ist in Verbindungen der Formeln (7a) bis (7e), (8a) bis (8e) und (9a) bis (9e) der Index n gleich oder verschieden bei jedem Auftreten 0, 1, 2 oder 3 ist, besonders bevorzugt 0, 1 oder 2 und ganz besonders bevorzugt 0 oder 1 und insbesondere gleich 0.

In nochmals einer weiteren bevorzugten Ausführungsform der Erfindung ist in Verbindungen der Formeln (7a) bis (7e), (8a) bis (8e) und (9a) bis (9e) der Index m gleich oder verschieden bei jedem Auftreten 0, 1 oder 2 ist, besonders bevorzugt 0 oder 1 und ganz besonders bevorzugt 0.

Im Folgenden werden bevorzugte Substituenten R beschrieben. R ist bevorzugt gewählt aus der Gruppe bestehend aus H, D, F, CN, N(R¹)₂, C(=O)R¹, P(=O)(R¹)₂, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, wobei die Alkyl-, Alkoxy- bzw. Alkenylgruppe jeweils mit einem oder mehreren Resten R¹ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten R¹ substituiert sein kann, bevorzugt aber unsubstituiert ist; dabei können optional zwei Substituenten R, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten R¹ substituiert sein kann.

Besonders bevorzugt sind diese Substituenten R ausgewählt aus der Gruppe bestehend aus H, D, F, CN, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3 oder 4 C-Atomen, oder einer Alkenylgruppe mit 2 bis 8 C-Atomen, bevorzugt mit 2, 3 oder 4 C-Atomen, wobei die Alkyl- bzw. Alkenylgruppe jeweils mit einem oder mehreren Resten R¹ substituiert sein kann, bevorzugt aber unsubstituiert ist, oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren nicht-aromatischen Resten R¹ substituiert sein kann, bevorzugt aber unsubstituiert ist; dabei können optional zwei Substituenten R, die an dasselbe Kohlenstoffatom oder an benachbarte Kohlenstoffatome gebunden sind, ein monocyclisches oder polycyclisches, aliphatisches Ringsystem bilden, das mit einem oder mehreren Resten R¹ substituiert sein kann, bevorzugt aber unsubstituiert ist.

Ganz besonders bevorzugt sind die Substituenten R ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten R¹ substituiert sein kann, bevorzugt aber unsubstituiert ist. Beispiele für geeignete Substituenten R sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste R¹ substituiert sein können, bevorzugt aber unsubstituiert sind. Dabei sind geeignete Strukturen R die gleichen Strukturen, wie sie vorne für Ar-1 bis Ar-19 abgebildet sind.

Wenn Z in der Struktur der Formel (2) für NR steht, ist es bevorzugt, wenn der Rest R, der an dieses Stickstoffatom gebunden ist, bei jedem Auftreten gleich oder verschieden für ein aromatisches oder heteroaromatisches Ringsystem mit 5 bis 24 aromatischen Ringatomen steht, das jeweils mit einem oder mehreren Resten R¹ substituiert sein kann, besonders bevorzugt für ein aromatisches oder heteroaromatisches Ringsystem mit 6 bis 18 aromatischen Ringatomen, das mit einem oder mehreren Resten R¹ substituiert sein kann. Beispiele für geeignete Substituenten R sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1,3,5-Triazinyl, 4,6-Diphenyl-1,3,5-triazinyl, 1-, 2-, 3- oder 4-Dibenzofuranyl, 1-, 2-, 3- oder 4-Dibenzothienyl und 1-, 2-, 3- oder 4-Carbazolyl, wobei die Carbazolylgruppe am Stickstoffatom durch einen Rest R¹ ungleich H oder D substituiert ist. Dabei können diese Gruppen jeweils durch einen oder mehrere Reste R¹ substituiert sein, sind bevorzugt aber unsubstituiert. Dabei sind geeignete Strukturen R die gleichen Strukturen, wie sie vorne für Ar-1 bis Ar-19 abgebildet sind.

In einer weiteren bevorzugten Ausführungsform der Erfindung ist R¹ bei jedem Auftreten gleich oder verschieden ausgewählt aus der Gruppe bestehend aus H, D, F, CN, einem aliphatischen Kohlenwasserstoffrest mit 1 bis 6 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch eine oder mehrere Alkylgruppen mit jeweils 1 bis 4 Kohlenstoffatomen substituiert sein kann, bevorzugt aber unsubstituiert ist.

Es ist weiterhin bevorzugt, wenn die aromatischen bzw. heteroaromatischen Gruppen R bzw. R¹ bzw. R² bzw. Ar¹ bzw. Ar² in der erfindungsgemäßen Verbindung keine Aryl- oder Heteroarylgruppen mit mehr als zwei direkt aneinander kondensierten aromatischen Sechsringen aufweisen. Kondensierte Arylgruppen mit mehr als zwei direkt aneinander kondensierten aromatischen Sechsringen, die dennoch auch erfindungsgemäß geeignet sind, sind Phenanthren und Triphenylen, da auch diese ein hohes Triplettniveau aufweisen.

Die oben genannten Bevorzugungen können einzeln oder gemeinsam auftreten. Es ist bevorzugt, wenn die oben genannten Bevorzugungen gemeinsam auftreten.

Ganz besonders bevorzugt ist sind die Verbindungen gemäß den folgenden Formeln (10) und (11), 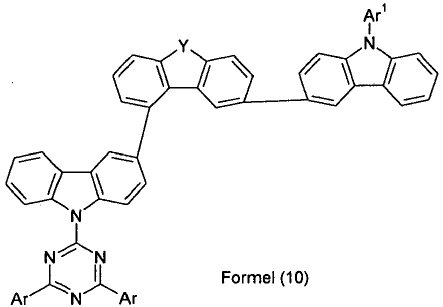 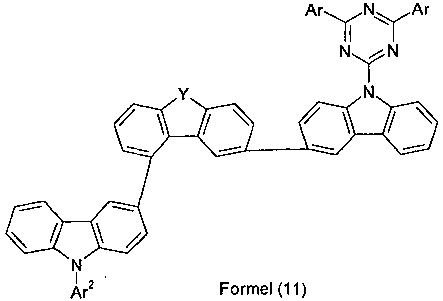 wobei die verwendeten Symbole die oben genannten Bedeutungen und insbesondere die oben genannten bevorzugten Bedeutungen aufweisen.

Beispiele für geeignete erfindungsgemäße Verbindungen sind die nachstehend abgebildeten Strukturen.

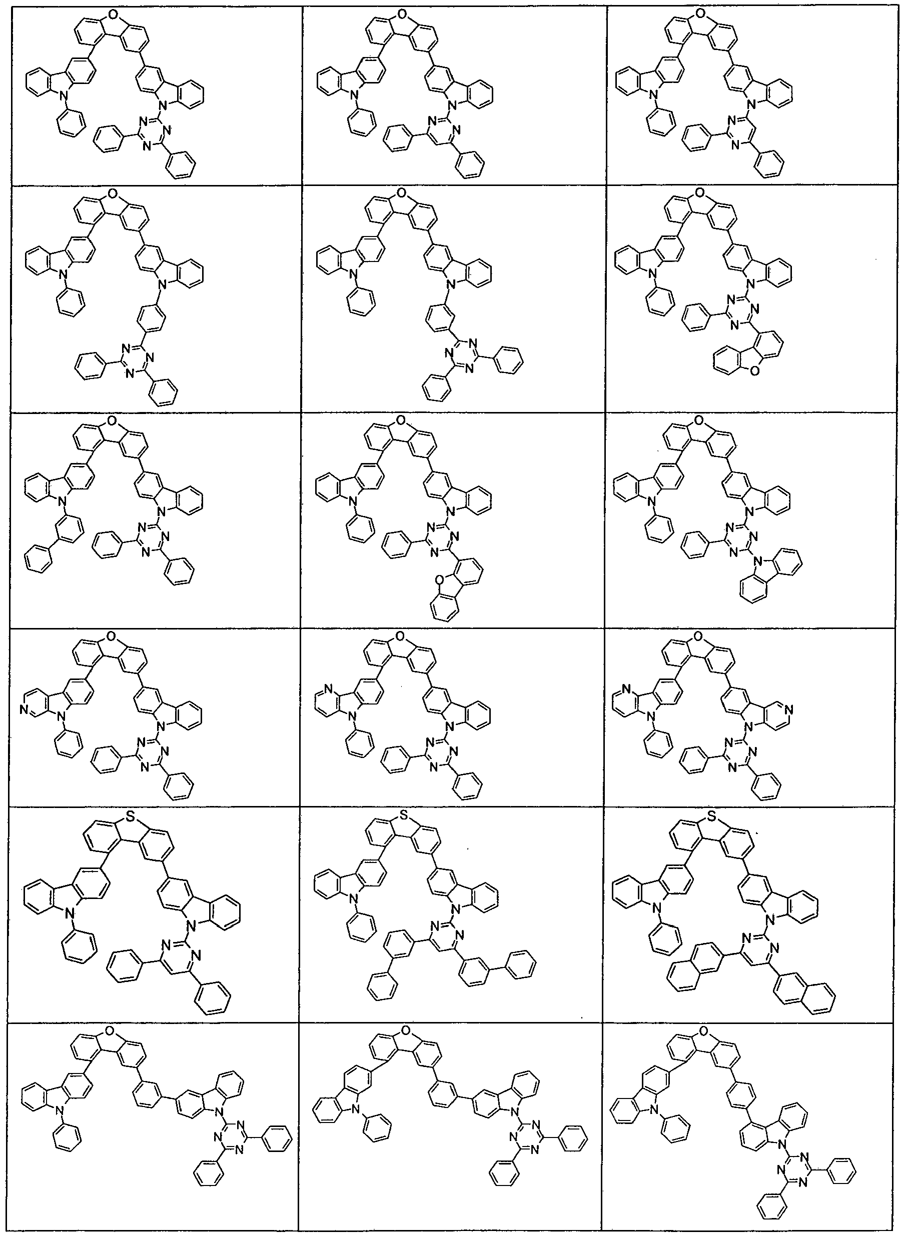

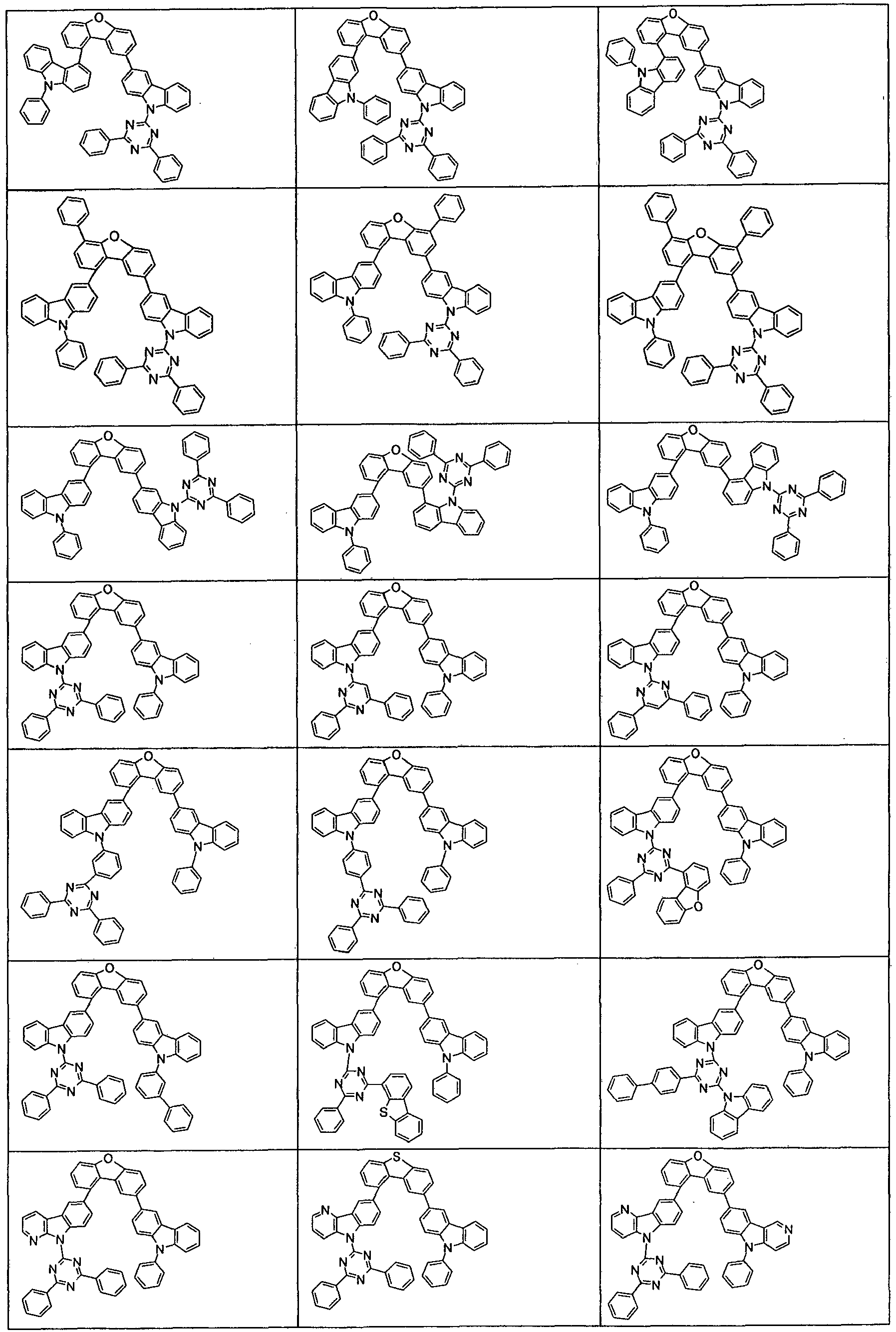

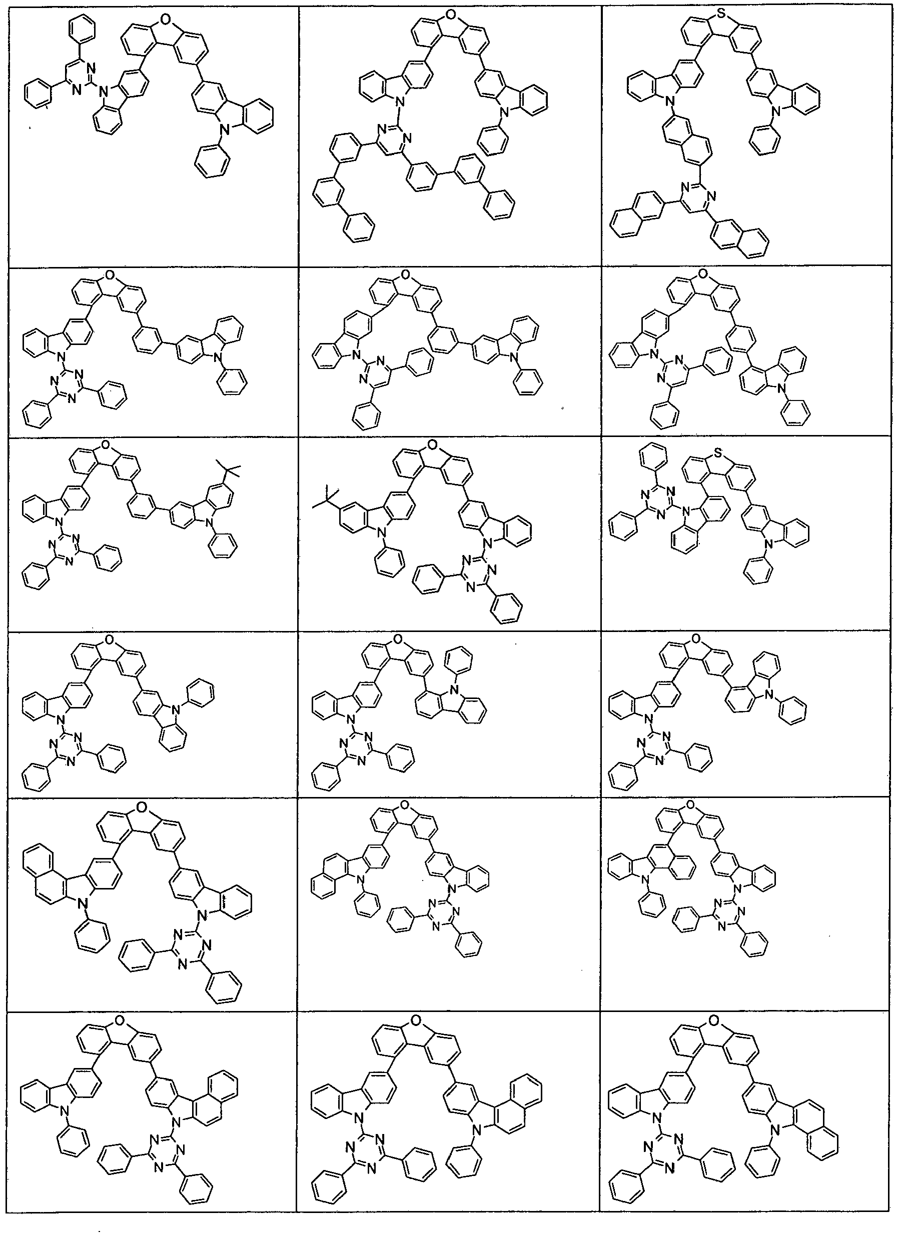

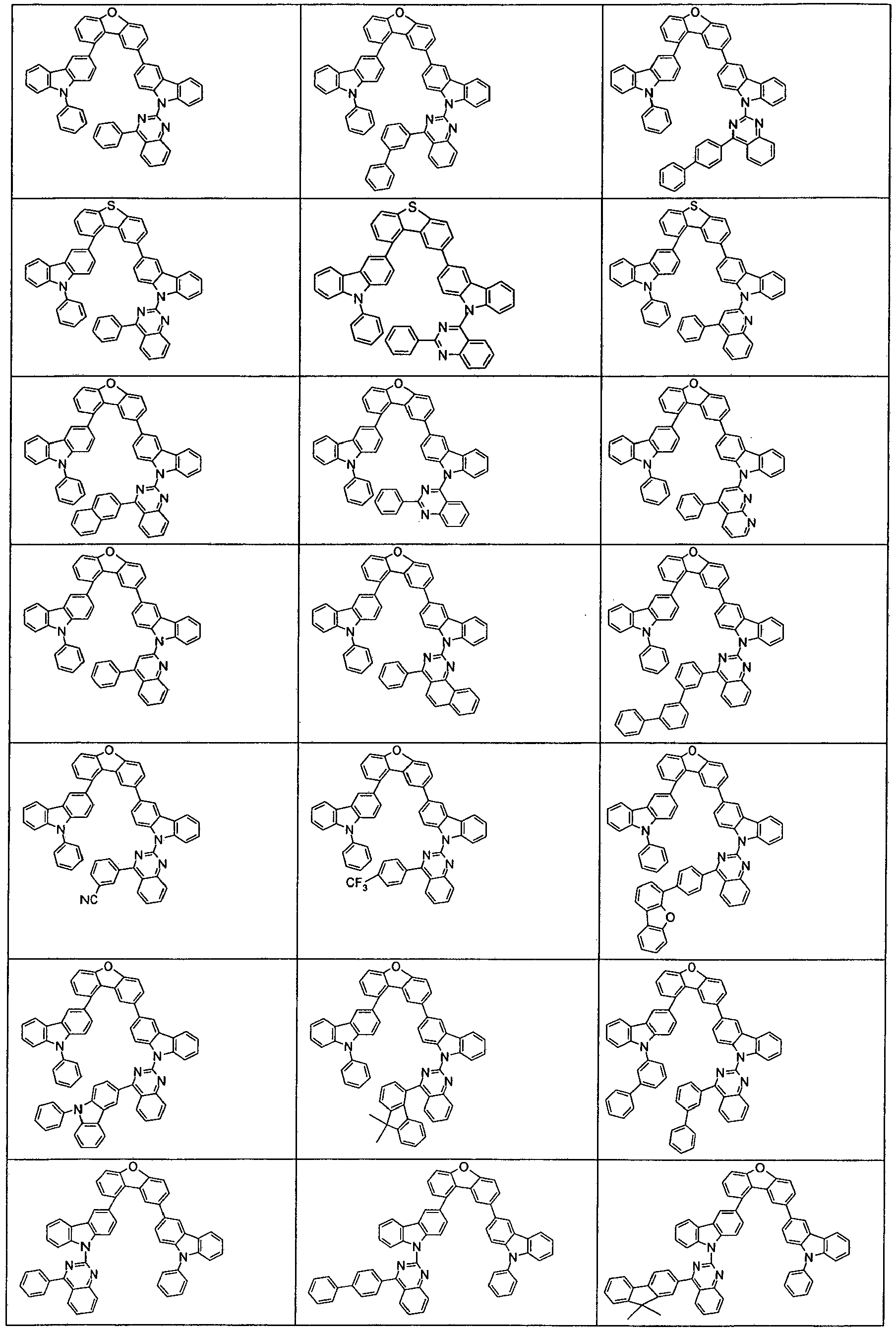

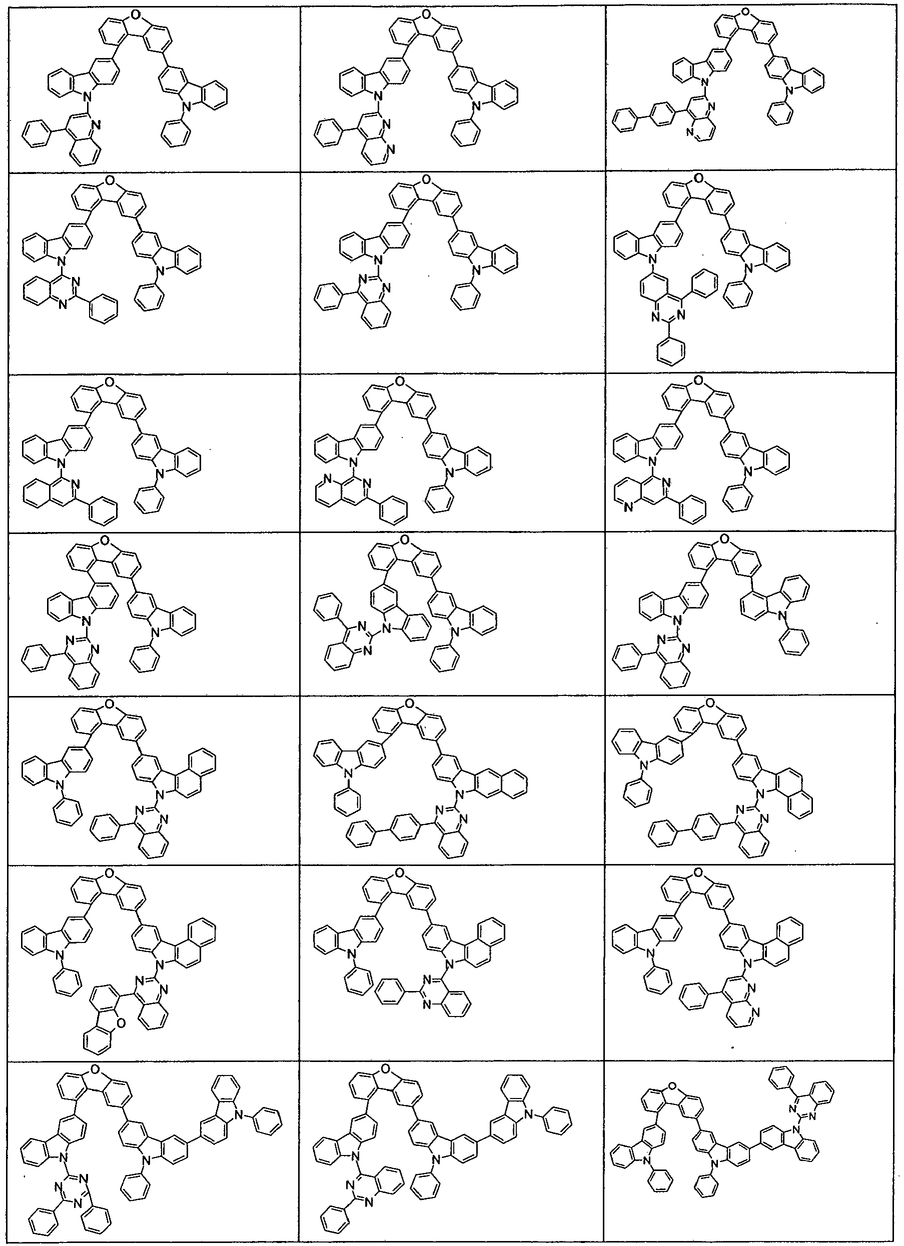

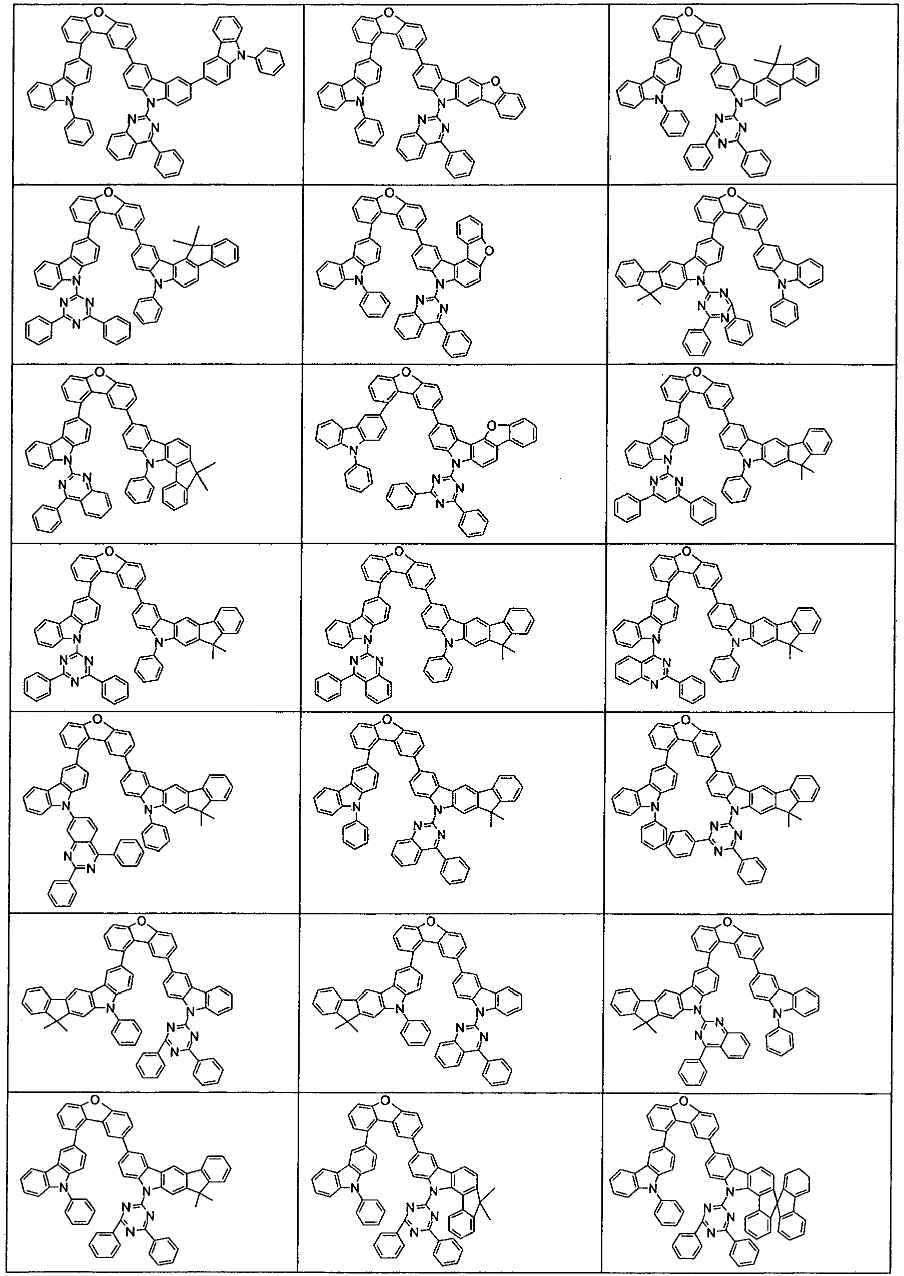

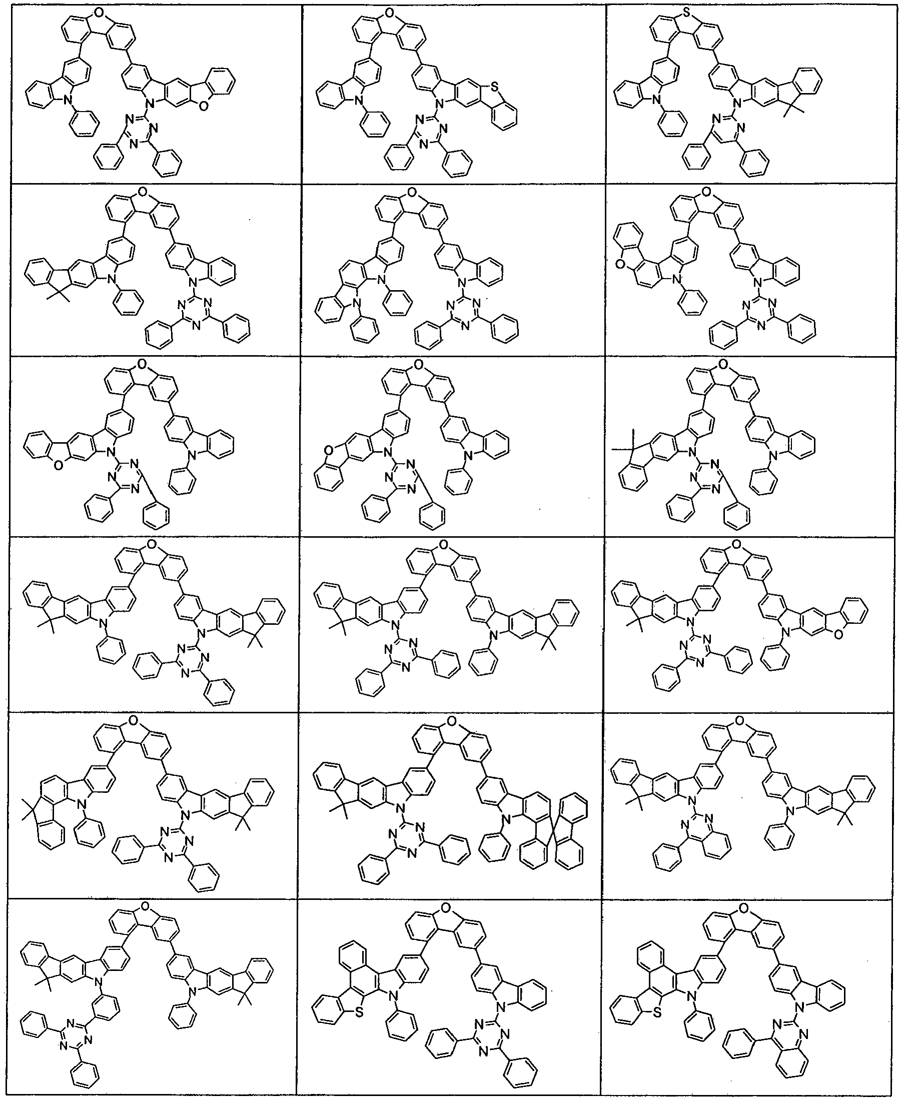

Die erfindungsgemäßen Verbindungen können nach dem Fachmann bekannten Syntheseschritten, wie z. B. Bromierung, Suzuki-Kupplung, Ullmann-Kupplung, Hartwig-Buchwald-Kupplung, etc., dargestellt werden. Ein geeignetes Syntheseverfahren ist allgemein im folgenden Schema 2 dargestellt. Schema 1 zeigt die Synthese des 1-Brom-substituierten Dibenzofurans, welches als Edukt eingesetzt wird. In Schema 2 wird die Funktionalisierung des Dibenzofurans in 8-Position sowie die Umsetzung zu den erfindungsgemäßen Verbindungen gezeigt.

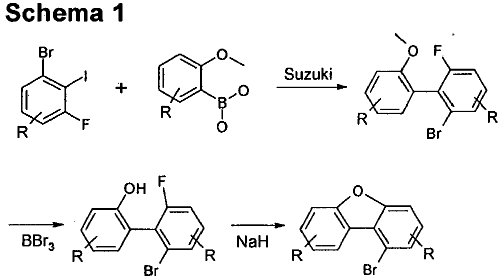

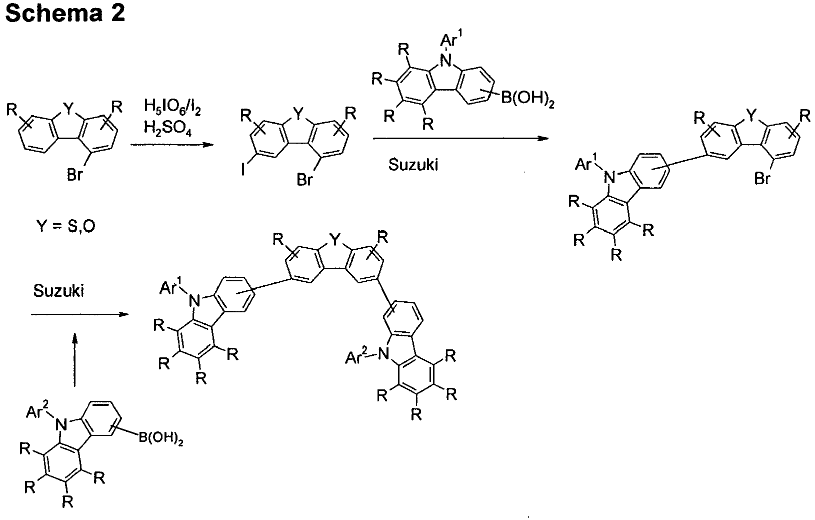

Ein weiterer Gegenstand der vorliegenden Erfindung ist daher ein Verfahren zur Herstellung der erfindungsgemäßen Verbindungen durch Umsetzung eines gegebenenfalls substituierten 1,8-Dihalogendibenzofurans bzw. 1,8-Dihalogendibenzothiophens bzw. einem entsprechenden Derivat, welches ein oder mehrere Stickstoffatome im Grundkörper aufweist, mit einem Carbazolderivat, gefolgt durch Umsetzung mit dem anderen Carbazolderivat, wobei die Umsetzungen mit den Carbazolderivaten jeweils C-C-Kupplungen darstellen, insbesondere Suzuki-Kupplungen.

Für die Verarbeitung der erfindungsgemäßen Verbindungen aus flüssiger Phase, beispielsweise durch Spin-Coating oder durch Druckverfahren, sind Formulierungen der erfindungsgemäßen Verbindungen erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropylenglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)-ethan, Hexamethylindan oder Mischungen dieser Lösemittel.

Ein weiterer Gegenstand der vorliegenden Erfindung ist daher eine Formulierung, enthaltend eine erfindungsgemäße Verbindung und mindestens eine weitere Verbindung. Die weitere Verbindung kann beispielsweise ein Lösemittel sein, insbesondere eines der oben genannten Lösemittel oder eine Mischung dieser Lösemittel. Die weitere Verbindung kann aber auch mindestens eine weitere organische oder anorganische Verbindung sein, die ebenfalls in der elektronischen Vorrichtung eingesetzt wird, beispielsweise eine emittierende Verbindung, insbesondere ein phosphoreszierender Dotand, und/oder ein weiteres Matrixmaterial. Geeignete emittierende Verbindungen und weitere Matrixmaterialien sind hinten im Zusammenhang mit der organischen Elektrolumineszenzvorrichtung aufgeführt. Diese weitere Verbindung kann auch polymer sein.

Die erfindungsgemäßen Verbindungen und Mischungen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Ein weiterer Gegenstand der vorliegenden Erfindung ist daher die Verwendung der erfindungsgemäßen Verbindungen oder Mischungen in einer elektronischen Vorrichtung, insbesondere in einer organischen Elektrolumineszenzvorrichtung.

Nochmals ein weiterer Gegenstand der vorliegenden Erfindung ist eine elektronische Vorrichtung enthaltend mindestens eine der oben ausgeführten erfindungsgemäßen Verbindungen oder Mischungen. Dabei gelten die oben für die Verbindung ausgeführten Bevorzugungen auch für die elektronischen Vorrichtungen.

Die elektronische Vorrichtung ist bevorzugt ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen farbstoff-sensibilisierten Solarzellen, organischen optischen Detektoren, organischen Photorezeptoren, organischen Feld-Quench-Devices (O-FQDs), lichtemittierenden elektrochemischen Zellen (LECs), organischen Laserdioden (O-Laser) und "organic plasmon emitting devices" (D. M. Koller et al., Nature Photonics 2008, 1-4), bevorzugt organischen Elektrolumineszenzvorrichtungen (OLEDs, PLEDs), insbesondere phosphoreszierenden OLEDs.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten und/oder Ladungserzeugungsschichten (Charge-Generation Layers). Ebenso können zwischen zwei emittierende Schichten Zwischenschichten (Interlayer) eingebracht sein, welche beispielsweise eine exzitonenblockierende Funktion aufweisen. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Systeme mit drei emittierenden Schichten, wobei die drei Schichten blaue, grüne und orange oder rote Emission zeigen (für den prinzipiellen Aufbau siehe z. B. WO 2005/011013). Es kann sich dabei um fluoreszierende oder um phosphoreszierende Emissionsschichten handeln oder um Hybrid-Systeme, bei denen fluoreszierende und phosphoreszierende Emissionsschichten miteinander kombiniert werden. Eine weiß emittierende Elektrolumineszenzvorrichtung kann beispielsweise für Beleuchtungsanwendungen, aber auch in Kombination mit einem Farbfilter für Vollfarb-Displays verwendet werden.

Die erfindungsgemäße Verbindung gemäß den oben aufgeführten Ausführungsformen kann dabei in unterschiedlichen Schichten eingesetzt werden, je nach genauer Struktur. Bevorzugt ist eine organische Elektrolumineszenzvorrichtung, enthaltend eine Verbindung gemäß Formel (1) oder gemäß den bevorzugten Ausführungsformen als Matrixmaterial für fluoreszierende oder phosphoreszierende Emitter, insbesondere für phosphoreszierende Emitter, und/oder als Elektronetransport- bzw. Lochblockiermaterial in einer Elektronentransportschicht und/oder in einer lochblockierenden Schicht, je nach genauer Substitution. Dabei gelten die oben aufgeführten bevorzugten Ausführungsformen auch für die Verwendung der Materialien in organischen elektronischen Vorrichtungen.

In einer bevorzugten Ausführungsform der Erfindung wird die Verbindung gemäß Formel (1) bzw. gemäß den bevorzugten Ausführungsformen als Matrixmaterial für eine phosphoreszierende Verbindung in einer emittierenden Schicht eingesetzt. Dabei kann die organische Elektrolumineszenzvorrichtung eine emittierende Schicht enthalten, oder sie kann mehrere emittierende Schichten enthalten, wobei mindestens eine emittierende Schicht mindestens eine erfindungsgemäße Verbindung als Matrixmaterial enthält.

Wenn die Verbindung gemäß Formel (1) bzw. gemäß den bevorzugten Ausführungsformen als Matrixmaterial für eine emittierende Verbindung in einer emittierenden Schicht eingesetzt wird, wird sie bevorzugt in Kombination mit einem oder mehreren phosphoreszierenden Materialien (Triplettemitter) eingesetzt. Unter Phosphoreszenz im Sinne dieser Erfindung wird die Lumineszenz aus einem angeregten Zustand mit Spinmultiplizität > 1, insbesondere aus einem angeregten Triplettzustand. Im Sinne dieser Anmeldung sollen alle lumineszierenden Übergangsmetallkomplexe und lumineszierenden Lanthanidkomplexe, insbesondere alle Iridium-, Platin- und Kupferkomplexe als phosphoreszierende Verbindungen angesehen werden.

Die Mischung aus der Verbindung gemäß Formel (1) bzw. gemäß den bevorzugten Ausführungsformen und der emittierenden Verbindung enthält zwischen 99 und 1 Vol.-%, vorzugsweise zwischen 98 und 10 Vol.-%, besonders bevorzugt zwischen 97 und 60 Vol.-%, insbesondere zwischen 95 und 80 Vol.-% der Verbindung gemäß Formel (1) bzw. gemäß den bevorzugten Ausführungsformen bezogen auf die Gesamtmischung aus Emitter und Matrixmaterial. Entsprechend enthält die Mischung zwischen 1 und 99 Vol.-%, vorzugsweise zwischen 2 und 90 Vol.-%, besonders bevorzugt zwischen 3 und 40 Vol.-%, insbesondere zwischen 5 und 20 Vol.-% des Emitters bezogen auf die Gesamtmischung aus Emitter und Matrixmaterial. Werden die Verbindungen aus Lösung verarbeitet, so werden statt der oben angegebenen Mengen in Vol.-% bevorzugt die entsprechenden Mengen in Gew.-% verwendet.

Als phosphoreszierende Verbindungen (= Triplettemitter) eignen sich insbesondere Verbindungen, die bei geeigneter Anregung Licht, vorzugsweise im sichtbaren Bereich, emittieren und außerdem mindestens ein Atom der Ordnungszahl größer 20, bevorzugt größer 38 und kleiner 84, besonders bevorzugt größer 56 und kleiner 80 enthalten, insbesondere ein Metall mit dieser Ordnungszahl. Bevorzugt werden als Phosphoreszenzemitter Verbindungen, die Kupfer, Molybdän, Wolfram, Rhenium, Ruthenium, Osmium, Rhodium, Iridium, Palladium, Platin, Silber, Gold oder Europium enthalten, verwendet, insbesondere Verbindungen, die Iridium oder Platin enthalten. Im Sinne der vorliegenden Erfindung werden alle lumineszierenden Verbindungen, die die oben genannten Metalle enthalten, als phosphoreszierende Verbindungen angesehen.

Beispiele der oben beschriebenen Emitter können den Anmeldungen WO 00/70655, WO 2001/41512, WO 2002/02714, WO 2002/15645, EP 1191613, EP 1191612, EP 1191614, WO 05/033244, WO 05/019373, US 2005/0258742, WO 2009/146770, WO 2010/015307, WO 2010/031485, WO 2010/054731, WO 2010/054728, WO 2010/086089, WO 2010/099852, WO 2010/102709, WO 2011/032626, WO 2011/066898, WO 2011/157339, WO 2012/007086, WO 2014/008982, WO 2014/023377, WO 2014/094961, WO 2014/094960, WO 2015/036074, WO 2015/104045, WO 2015/117718, WO 2016/015815 und den noch nicht offen gelegten Anmeldungen EP 15000307.7, EP 15182264.0 und EP 16179378.1 entnommen werden. Generell eignen sich alle phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind, und der Fachmann kann ohne erfinderisches Zutun weitere phosphoreszierende Komplexe verwenden.

Eine weitere bevorzugte Ausführungsform der vorliegenden Erfindung ist der Einsatz der Verbindung gemäß Formel (1) bzw. gemäß den bevorzugten Ausführungsformen als Matrixmaterial für einen phosphoreszierenden Emitter in Kombination mit einem weiteren Matrixmaterial. In einer bevorzugten Ausführungsform der Erfindung ist das weitere Matrixmaterial eine lochtransportierende Verbindung. In einer weiteren bevorzugten Ausführungsform der Erfindung ist das weitere Matrixmaterial eine elektronentransportierende Verbindung. In nochmals einer weiteren bevorzugten Ausführungsform ist das weitere Matrixmaterial eine Verbindung mit großem Bandabstand, das nicht oder nicht in wesentlichem Umfang am Loch- und Elektronentransport in der Schicht beteiligt ist.

Geeignete Matrixmaterialien für die erfindungsgemäßen Verbindungen sind Ketone, Phosphinoxide, Sulfoxide und Sulfone, z. B. gemäß WO 2004/013080, WO 2004/093207, WO 2006/005627 oder WO 2010/006680, Triarylamine, Carbazolderivate, z. B. CBP (N,N-Bis-carbazolylbiphenyl), m-CBP oder die in WO 2005/039246, US 2005/0069729, JP 2004/288381, EP 1205527, WO 2008/086851 oder US 2009/0134784 offenbarten Carbazolderivate, Kombinationen aus Triazinen und Carbazolen, z. B. gemäß WO 2011/057706 oder WO 2014/015931, Indolocarbazolderivate, z. B. gemäß WO 2007/063754 oder WO 2008/056746, Indenocarbazolderivate, z. B. gemäß WO 2010/136109, WO 2011/000455, WO 2013/041176 oder WO 2013/056776, Spiroindenocarbazolderivate, z. B. gemäß WO 2014/094963 oder WO 2015/124255, Azacarbazole, z. B. gemäß EP 1617710, EP 1617711, EP 1731584, JP 2005/347160, bipolare Matrixmaterialien, z. B. gemäß WO 2007/137725, Lactame, z. B. gemäß WO 2011/116865, WO 2011/137951, WO 2013/064206 oder WO 2014/056567, Silane, z. B. gemäß WO 2005/111172, Azaborole oder Boronester, z. B. gemäß WO 2006/117052 oder WO 2013/091762, Diazasilolderivate, z. B. gemäß WO 2010/054729, Diazaphospholderivate, z. B. gemäß WO 2010/054730, Triazinderivate, z. B. gemäß WO 2010/015306, WO 2007/063754, WO 2008/056746 oder WO 2014/023388, Zinkkomplexe, z. B. gemäß EP 652273 oder WO 2009/062578, Dibenzofuranderivate, z. B. gemäß WO 2009/148015, WO 2015/169412, WO 2016/015810 oder WO 2016/023608, verbrückte Carbazolderivate, z. B. gemäß US 2009/0136779, WO 2010/050778, WO 2011/042107 oder WO 2011/088877, oder Triphenylenderivate, z. B. gemäß WO 2012/048781. Ebenso kann ein weiterer phosphoreszierender Emitter, welcher kürzerwellig als der eigentliche Emitter emittiert, als Co-Host in der Mischung vorhanden sein.

Bevorzugte Co-Host-Materialien sind Triarylaminderivate, insbesondere Monoamine, Indenocarbazolderivate, 4-Spirocarbazolderivate, Lactame, Triazinderivate und Carbazolderivate.

Bevorzugte Triarylaminderivate, die als Co-Host-Materialien zusammen mit den erfindungsgemäßen Verbindungen eingesetzt werden, sind ausgewählt aus den Verbindungen der folgenden Formel (12), 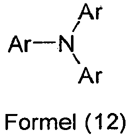 wobei Ar gleich oder verschieden bei jedem Auftreten die oben genannten Bedeutungen aufweist. Bevorzugt sind die Gruppen Ar gleich oder verschieden bei jedem Auftreten ausgewählt aus den oben genannten Gruppen Ar-1 bis Ar-19.

In einer bevorzugten Ausführungsform der Verbindungen der Formel (12) ist mindestens eine Gruppe Ar ausgewählt aus einer Biphenylgruppe, wobei es sich um eine ortho-, meta- oder para-Biphenylgruppe handeln kann. In einer weiteren bevorzugten Ausführungsform der Verbindungen der Formel (12) ist mindestens eine Gruppe Ar ausgewählt aus einer Fluorengruppe oder Spirobifluorengruppe, wobei diese Gruppen jeweils in 1-, 2-, 3- oder 4-Position an das Stickstoffatom gebunden sein können. In nochmals einer weiteren bevorzugten Ausführungsform der Verbindungen der Formel (12) ist mindestens eine Gruppe Ar ausgewählt aus einer Phenylen- oder Biphenylgruppe, wobei es sich um eine ortho-, meta- oder para-verknüpfte Gruppe handelt, die mit einer Dibenzofurangruppe, einer Dibenzothiophengruppe oder einer Carbazolgruppe, insbesondere einer Dibenzofurangruppe, substituiert ist, wobei die Dibenzofuran- bzw. Dibenzothiophengruppe über die 1-, 2-, 3- oder 4-Position mit der Phenylen- bzw. Biphenylgruppe verknüpft ist und wobei die Carbazolgruppe über die 1-, 2-, 3- oder 4-Position oder über das Stickstoffatom mit der Phenylen- bzw. Biphenylgruppe verknüpft ist.

In einer besonders bevorzugten Ausführungsform der Verbindungen der Formel (12) ist eine Gruppe Ar ausgewählt aus einer Fluoren- oder Spirobifluorengruppe, insbesondere einer 4-Fluoren- bzw. 4-Spirobifluorengruppe, und eine Gruppe Ar ist ausgewählt aus einer Biphenylgruppe, insbesondere einer para-Biphenylgruppe, oder einer Fluorengruppe, insbesondere einer 2-Fluorengruppe, und die dritte Gruppe Ar ist ausgewählt aus einer para-Phenylengruppe oder einer para-Biphenylgruppe, die mit einer Dibenzofurangruppe, insbesondere einer 4-Dibenzofurangruppe, oder einer Carbazolgruppe, insbesondere einer N-Carbazolgruppe oder einer 3-Carbazolgruppe, substituiert ist.

Bevorzugte Indenocarbazlderivate, die als Co-Host-Materialien zusammen mit den erfindungsgemäßen Verbindungen eingesetzt werden, sind ausgewählt aus den Verbindungen der folgenden Formel (13), 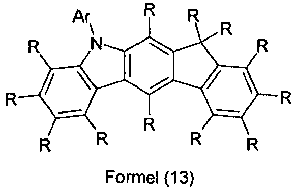 wobei Ar und R die oben aufgeführten Bedeutungen aufweisen. Dabei sind bevorzugte Ausführungsformen der Gruppe Ar die oben aufgeführten Strukturen Ar-1 bis Ar-19.

Eine bevorzugte Ausführungsform der Verbindungen der Formel (13) sind die Verbindungen der folgenden Formel (13a), 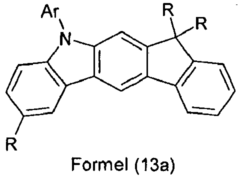 wobei Ar und R die oben genannten Bedeutungen aufweisen. Dabei stehen die beiden Gruppen R, die an das Indenokohlenstoffatom gebunden sind, bevorzugt gleich oder verschieden für eine Alkylgruppe mit 1 bis 4 C-Atomen, insbesondere für Methylgruppen, oder für ein aromatisches Ringsystem mit 6 bis 12 C-Atomen, insbesondere für Phenylgruppen. Besonders bevorzugt stehen die beiden Gruppen R, die an das Indenokohlenstoffatom gebunden sind, für Methylgruppen. Weiterhin bevorzugt steht der Substituent R, der in Formel (13a) an den Indenocarbazolgrundkörper gebunden ist, für H oder für eine Carbazolgruppe, die über die 1-, 2-, 3- oder 4-Position oder über das N-Atom an den Indenocarbazolgrundkörper gebunden sein kann, insbesondere über die 3-Position.

Bevorzugte 4-Spirocarbazolderivate, die als Co-Host-Materialien zusammen mit den erfindungsgemäßen Verbindungen eingesetzt werden, sind ausgewählt aus den Verbindungen der folgenden Formel (14), 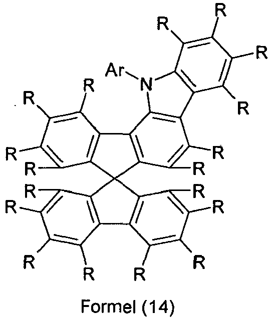 wobei Ar und R die oben aufgeführten Bedeutungen aufweisen. Dabei sind bevorzugte Ausführungsformen der Gruppe Ar die oben aufgeführten Strukturen Ar-1 bis Ar-19.

Eine bevorzugte Ausführungsform der Verbindungen der Formel (14) sind die Verbindungen der folgenden Formel (14a), 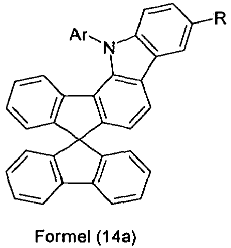 wobei Ar und R die oben genannten Bedeutungen aufweisen.

Bevorzugte Lactame, die als Co-Host-Materialien zusammen mit den erfindungsgemäßen Verbindungen eingesetzt werden, sind ausgewählt aus den Verbindungen der folgenden Formel (15), 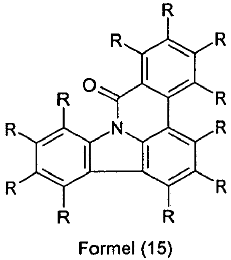 wobei R die oben aufgeführten Bedeutungen aufweist.

Eine bevorzugte Ausführungsform der Verbindungen der Formel (15) sind die Verbindungen der folgenden Formel (15a), 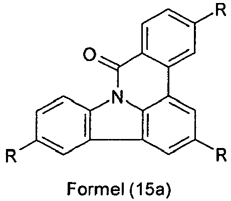 wobei R die oben genannten Bedeutungen aufweist. Dabei steht R bevorzugt gleich oder verschieden bei jedem Auftreten für H oder ein aromatisches oder heteroaromatisches Ringsystem mit 5 bis 40 aromatischen Ringatomen, das mit einem oder mehreren Resten R¹ substituiert sein kann. Ganz besonders bevorzugt sind die Substituenten R ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten R¹ substituiert sein kann, bevorzugt aber unsubstituiert ist. Beispiele für geeignete Substituenten R sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste R¹ substituiert sein können, bevorzugt aber unsubstituiert sind. Dabei sind geeignete Strukturen R die gleichen Strukturen, wie sie vorne für Ar-1 bis Ar-19 abgebildet sind, wobei diese Strukturen durch R¹ statt R substituiert sind.

In einer weiteren Ausführungsform der Erfindung enthält die erfindungsgemäße organische Elektrolumineszenzvorrichtung keine separate Lochinjektionsschicht und/oder Lochtransportschicht und/oder Lochblockierschicht und/oder Elektronentransportschicht, d. h. die emittierende Schicht grenzt direkt an die Lochinjektionschicht oder die Anode an, und/ oder die emittierende Schicht grenzt direkt an die Elektronentransportschicht oder die Elektroneninjektionsschicht oder die Kathode an, wie zum Beispiel in WO 2005/053051 beschrieben. Weiterhin ist es möglich, einen Metallkomplex, der gleich oder ähnlich dem Metallkomplex in der emittierenden Schicht ist, direkt angrenzend an die emittierende Schicht als Lochtransport- bzw. Lochinjektionsmaterial zu verwenden, wie z. B. in WO 2009/030981 beschrieben.

Weiterhin ist es möglich, die erfindungsgemäßen Verbindungen in einer Lochblockier- oder Elektronentransportschicht einzusetzen.

In den weiteren Schichten der erfindungsgemäßen organischen Elektrolumineszenzvorrichtung können alle Materialien verwendet werden, wie sie üblicherweise gemäß dem Stand der Technik eingesetzt werden. Der Fachmann kann daher ohne erfinderisches Zutun alle für organische Elektrolumineszenzvorrichtungen bekannten Materialien in Kombination mit den erfindungsgemäßen Verbindungen gemäß Formel (1) bzw. gemäß den bevorzugten Ausführungsformen verwenden.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck kleiner 10⁻⁵ mbar, bevorzugt kleiner 10⁻⁶ mbar aufgedampft. Es ist aber auch möglich, dass der Anfangsdruck noch geringer oder höher ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Ink-Jet Druck (Tintenstrahldruck), LITI (Light Induced Thermal Imaging, Thermotransferdruck), Siebdruck, Flexodruck, Offsetdruck oder Nozzle-Printing hergestellt werden. Hierfür sind lösliche Verbindungen nötig, welche beispielsweise durch geeignete Substitution erhalten werden.

Weiterhin sind Hybridverfahren möglich, bei denen beispielsweise eine oder mehrere Schichten aus Lösung aufgebracht werden und eine oder mehrere weitere Schichten aufgedampft werden. So ist es beispielsweise möglich, die emittierende Schicht aus Lösung aufzubringen und die Elektronentransportschicht aufzudampfen.

Diese Verfahren sind dem Fachmann generell bekannt und können von ihm ohne erfinderisches Zutun auf organische Elektrolumineszenzvorrichtungen enthaltend die erfindungsgemäßen Verbindungen angewandt werden.

Die erfindungsgemäßen Verbindungen weisen bei Verwendung in organischen Elektrolumineszenzvorrichtungen generell sehr gute Eigenschaften auf. Insbesondere ist bei Verwendung der erfindungsgemäßen Verbindungen in organischen Elektrolumineszenzvorrichtungen die Lebensdauer wesentlich besser im Vergleich zu ähnlichen Verbindungen gemäß dem Stand der Technik. Dabei sind die weiteren Eigenschaften der organischen Elektrolumineszenzvorrichtung, insbesondere die Effizienz und die Spannung, ebenfalls besser oder zumindest vergleichbar.

Die Erfindung wird nun durch die nachfolgenden Beispiele näher erläutert, ohne sie dadurch einschränken zu wollen.

### Beispiele

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Zu den literaturbekannten Verbindungen sind jeweils auch die entsprechenden CAS-Nummern angegeben.

### a) 6-Brom-2-fluor-2'-methoxy-biphenyl

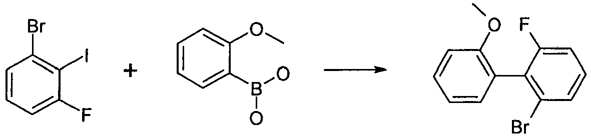

200 g (664 mmol) 1-Brom-3-fluor-2-iodbenzol, 101 g (664 mmol) 2-Methoxyphenylboronsäure und 137.5 g (997 mmol) Natriumtetraborat werden in 1000 mL THF und 600 ml Wasser gelöst und entgast. Es wird mit 9.3 g (13.3 mmol) Bis(triphenylphosphin)palladium(II)chlorid und 1 g (20 mmol) Hydraziniumhydroxid versetzt. Die Reaktionsmischung wird bei 70 °C für 48 h unter Schutzgasatmosphäre gerührt. Die abgekühlte Lösung wird mit Toluol aufgestockt, mehrmals mit Wasser gewaschen, getrocknet und eingeengt. Das Produkt wird via Säulenchromatographie an Kieselgel mit Toluol/Heptan (1:2) gereinigt. Ausbeute: 155 g (553 mmol), 83 % der Theorie.

Analog dazu wird die folgende Verbindung hergestellt:

| | **Edukt 1** | **Edukt 2** | **Produkt** | **Ausbeute** |
|---|---|---|---|---|
| a1 | 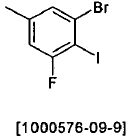 | 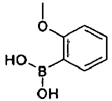 | 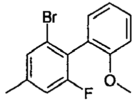 | 92% |

### b) 6'-Bromo-2'-fluoro-biphenyl-2-ol

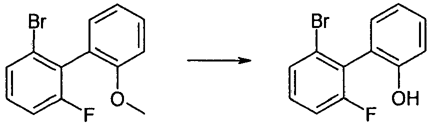

112 g (418 mmol) 6-Bromo-2-fluoro-2'-methoxy-biphenyl werden in 2 L Dichloromethan gelöst und auf 5 °C gekühlt. Zu dieser Lösung werden innerhalb von 90 min. 41.01 ml (431 mmol) Bortribromid zugetropft und über Nacht weiter gerührt. Das Gemisch wird im Anschluss langsam mit Wasser versetzt, die organische Phase dreimal mit Wasser gewaschen, über Na₂SO₄ getrocknet und einrotiert und chromatographisch gereinigt. Ausbeute: 104 g (397 mmol), 98 % der Theorie.

Analog dazu wird die folgende Verbindung hergestellt:

| | **Edukt 1** | **Produkt** | **Ausbeute** |
|---|---|---|---|
| b1 | 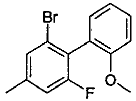 | 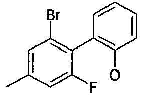 | 92% |

### c) 1-Brom-dibenzofuran

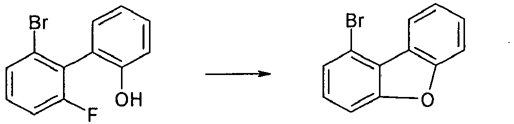

111 g (416 mmol) 6'-Brom-2'-fluor-biphenyl-2-ol werden in 2 L DMF (max. 0.003 % H₂O) SeccoSolv® gelöst und auf 5 °C gekühlt. Zu dieser Lösung werden portionsweise 20 g (449 mmol) Natriumhydrid (60% Suspension in Paraffinöl) zugegeben, nach beendeter Zugabe 20 min. nachgerührt und dann für 45 min. auf 100 °C erhitzt. Das Gemisch wird nach dem Abkühlen langsam mit 500 ml Ethanol versetzt, einrotiert und dann chromatographisch gereinigt. Ausbeute: 90 g (367 mmol), 88.5 % der Theorie.

Analog dazu wird die folgende Verbindung hergestellt:

| | **Edukt 1** | **Produkt** | **Ausbeute** |
|---|---|---|---|
| c1 | 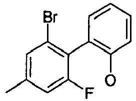 | 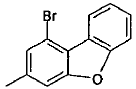 | 81% |

### d) 1-Bromo-8-iodo-dibenzofuran

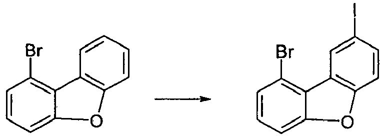

20 g (80 mmol) 1-Bromo-dibenzofuran, 2.06 g (40.1 mmol) Iod, 3.13 g (17.8 mmol) Iodsäure, 80 ml Essigsäure und 5 ml Schwefelsäure und 5 ml Wasser und 2 ml Chloroform werden 3 h bei 65 °C gerührt. Nach Erkalten wird die Mischung mit Wasser versetzt, der ausgefallene Feststoff abgesaugt und dreimal mit Wasser gewaschen. Der Rückstand wird aus Toluol und aus Dichlormethan /Heptan umkristallisiert. Die Ausbeute beträgt 25.6 g (68 mmol), entsprechend 85 % der Theorie.

Analog dazu werden die folgenden Verbindungen hergestellt:

| | **Edukt 1** | **Produkt** | **Ausbeute** |
|---|---|---|---|
| d1 | 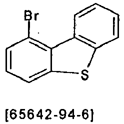 | 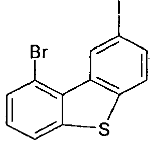 | 81% |
| d2 | 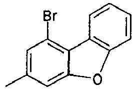 | 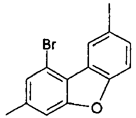 | 67% |

### e) 3-(9-Bromo-dibenzofuran-2-yl)-9-phenyl-9H-carbazol

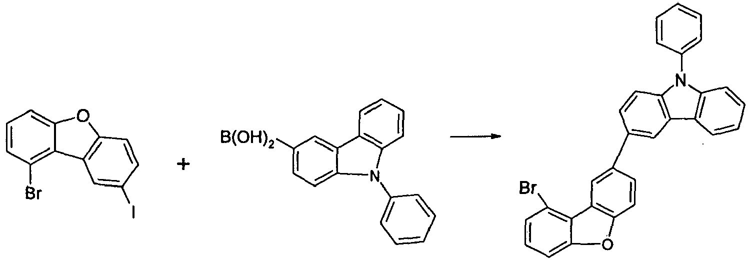

58 g (156 mmol) 1-Bromo-8-iodo-dibenzofuran, 50 g (172 mmol) N-Phenyl-carbazol-3-boronsäure und 36 g (340 mmol) Natriumcarbonat werden in 1000 mL Ethylenglycoldimethylether und 280 mL Wasser suspendiert. Zu dieser Suspension werden 1.8 g (1.5 mmol) Tetrakis(triphenylphosphin)-palladium(0) zugegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt. Die Ausbeute beträgt 48 g (89 mmol), entsprechend 64 % der Theorie.

Analog dazu werden die folgenden Verbindungen hergestellt:

| | **Edukt 1** | **Edukt 2** | **Produkt** | **Ausbeute** |
|---|---|---|---|---|
| e1 | 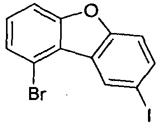 | 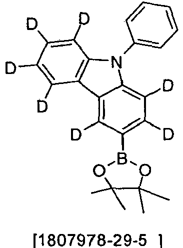 | 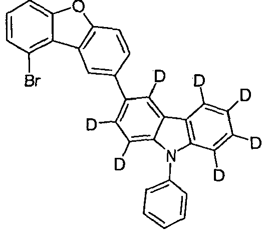 | 67% |
| e2 | 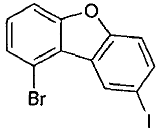 | 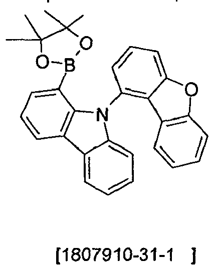 | 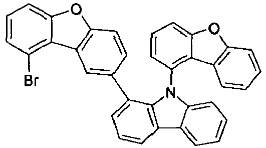 | 65% |
| e3 | 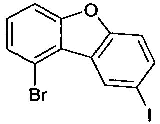 | 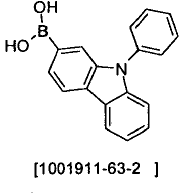 | 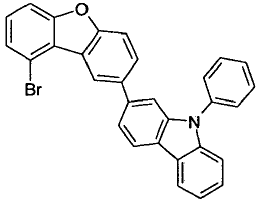 | 62% |
| e4 | 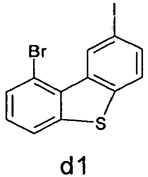 | 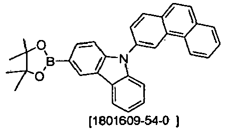 | 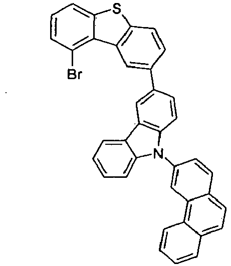 | 63% |
| e5 | 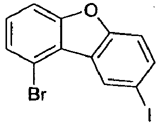 | 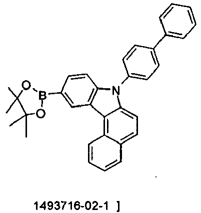 | 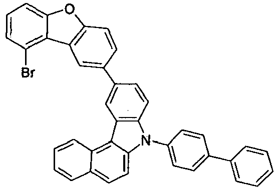 | 61% |
| e6 | 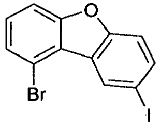 | 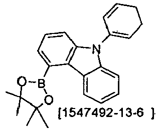 | 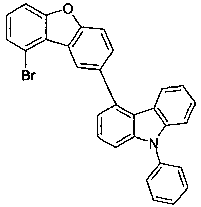 | 60% |
| e7 | 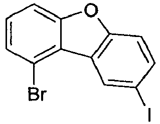 | 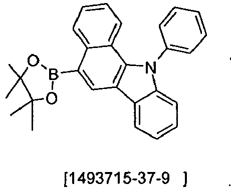 | 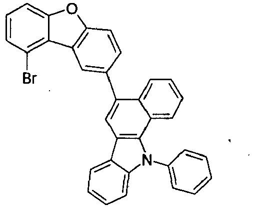 | 56% |
| e8 | 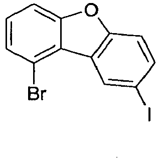 | 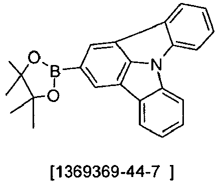 | 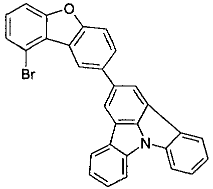 | 54% |
| e9 | 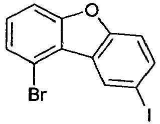 | 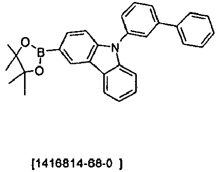 | 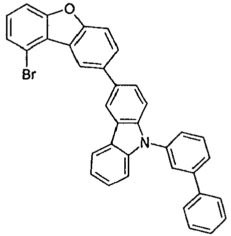 | 68% |
| e10 | 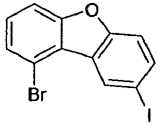 | 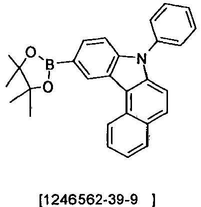 | 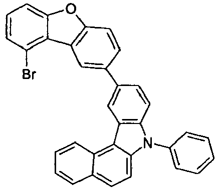 | 67% |
| e11 | 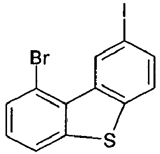 | 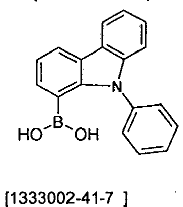 | 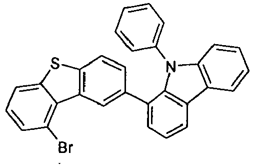 | 57% |
| e12 | 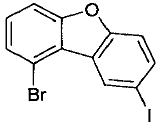 | 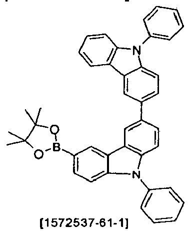 | 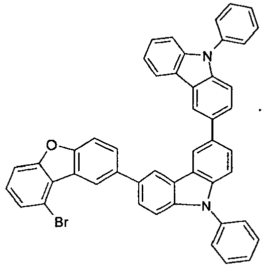 | 60% |
| e13 | 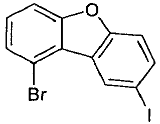 | 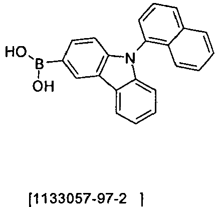 | 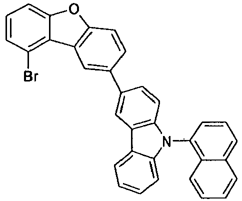 | 63% |
| e14 | 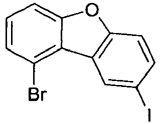 | 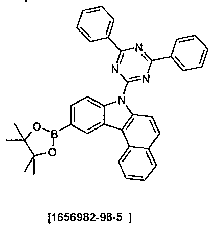 | 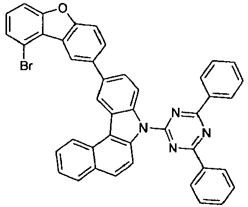 | 68% |
| e15 | 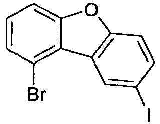 | 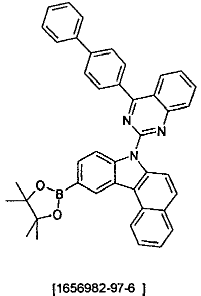 | 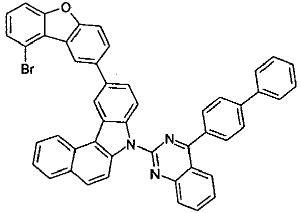 | 63% |
| e16 | 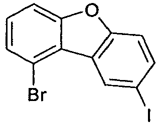 | 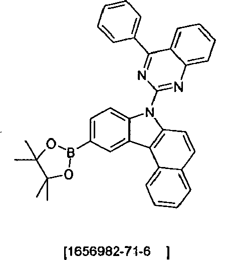 | 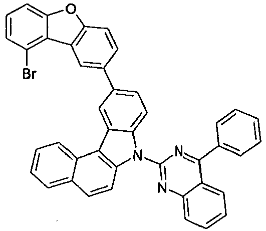 | 65% |
| e17 | 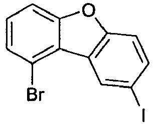 | 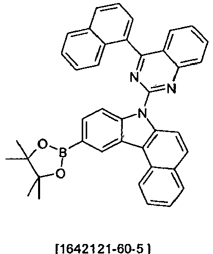 | 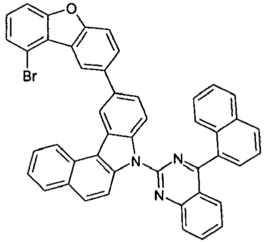 | 60% |
| e18 | 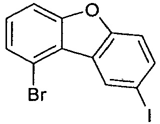 | 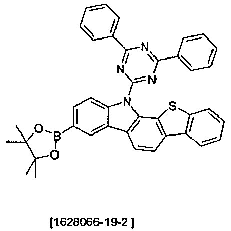 | 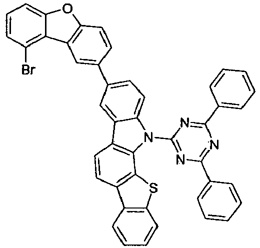 | 58% |
| e19 | 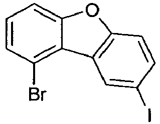 | 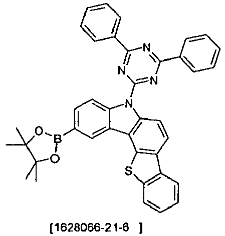 | 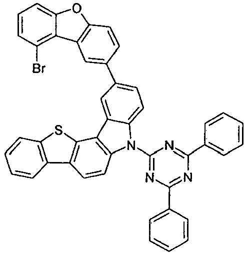 | 59% |
| e20 | 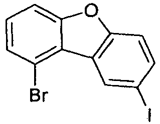 | 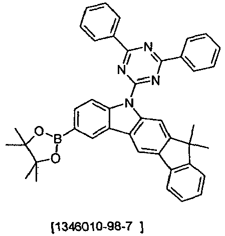 | 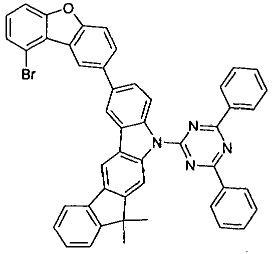 | 56% |
| e21 | 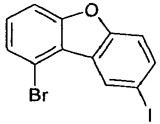 | 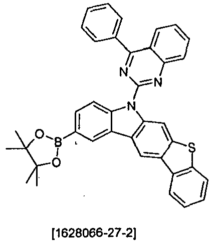 | 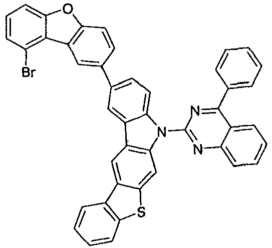 | 52% |
| e22 | 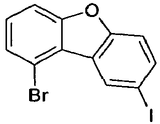 | 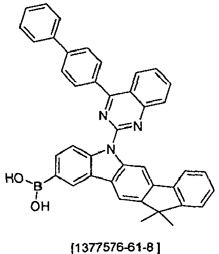 | 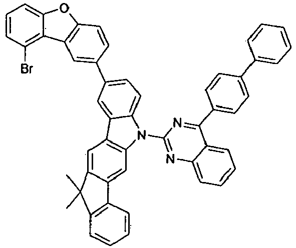 | 55% |
| e23 | 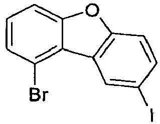 | 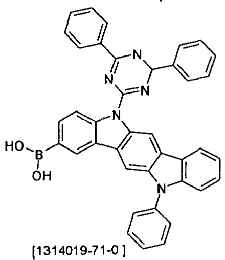 | 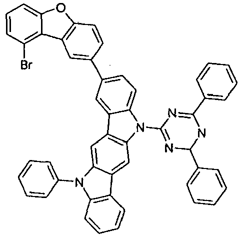 | 57% |
| e24 | 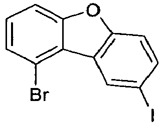 | 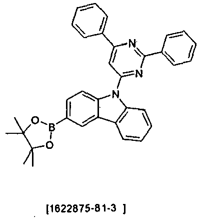 | 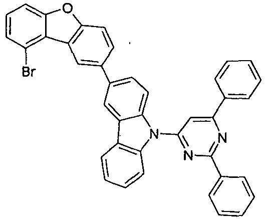 | 56% |
| e25 | 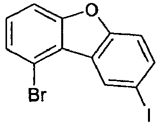 | 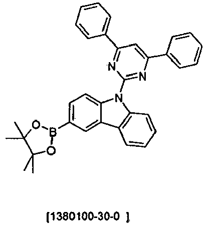 | 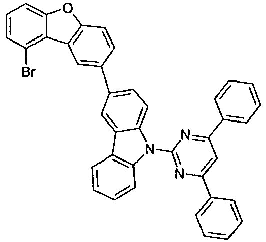 | 70% |
| e26 | 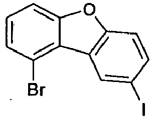 | 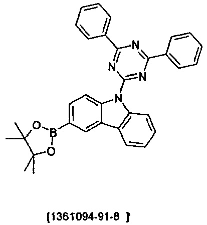 | 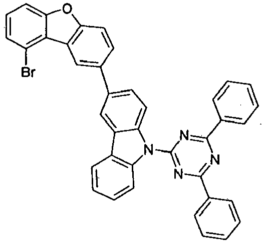 | 75% |
| e27 | 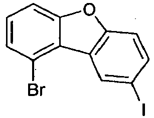 | 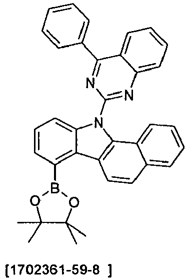 | 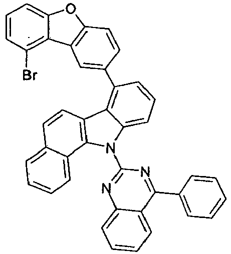 | 72% |
| e28 | 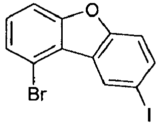 | 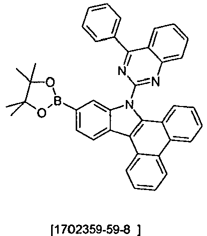 | 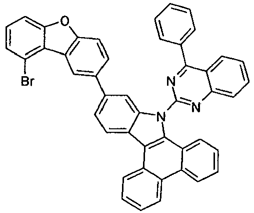 | 56% |
| e29 | 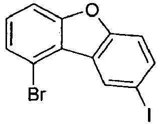 | 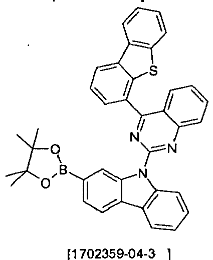 | 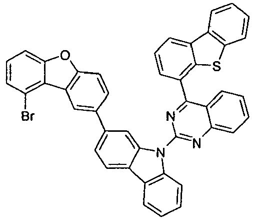 | 57% |
| e30 | 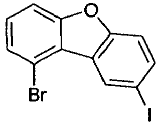 | 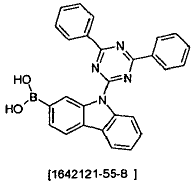 | 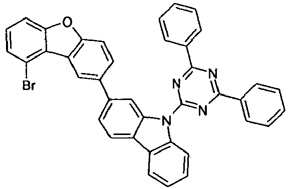 | 62% |
| e31 | 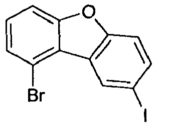 | 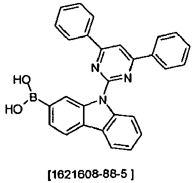 | 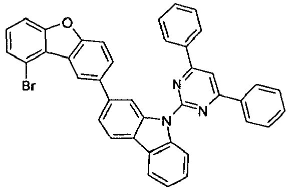 | 61% |
| e32 | 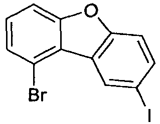 | 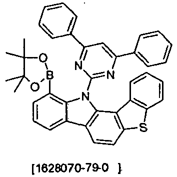 | 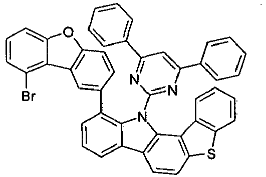 | 48% |
| e33 | 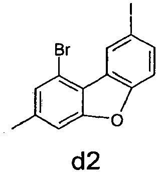 | 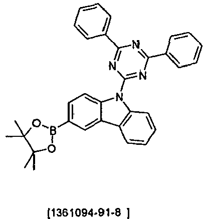 | 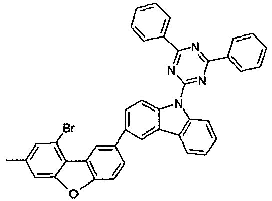 | 46% |
| e34 | 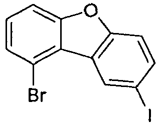 | 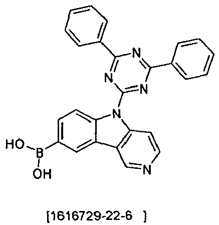 | 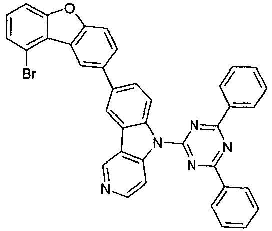 | 50% |
| e35 | 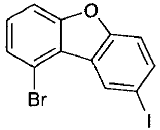 | 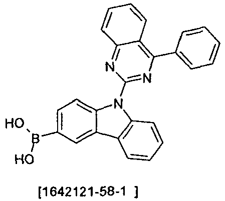 | 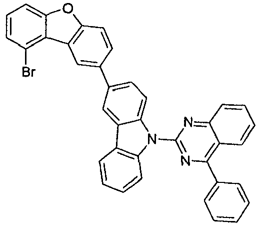 | 66% |

### f) 3-[1-[9-[(4,6-Diphenyl-1,3,5-triazin-2-yl)-9H-carbazol-3-yl]-8-dibenzofuranyl]-9-phenyl-9H-carbazol

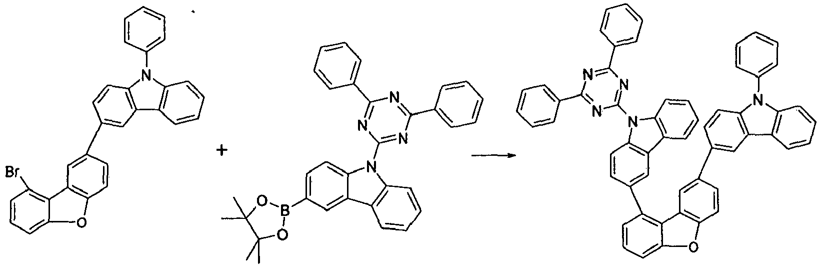

76 g (156 mmol) 3-(9-Bromo-dibenzofuran-2-yl)-9-phenyl-9H-carbazol, 90 g (172 mmol) 9-(4,6-Diphenyl-[1,3,5]triazin-2-yl)-3-(4,4,5,5-tetramethyl-[1,3,2]dioxaborolan-2-yl)-9H-carbazol und 36 g (340 mmol) Natriumcarbonat werden in 1000 mL Ethylenglycoldimethylether und 280 mL Wasser suspendiert. Zu dieser Suspension werden 1.8 g (1.5 mmol) Tetrakis(triphenylphosphin)-palladium(0) zugegeben, und die Reaktionsmischung wird 16 h unter Rückfluss erhitzt. Nach Erkalten wird die organische Phase abgetrennt, über Kieselgel filtriert, dreimal mit 200 mL Wasser gewaschen und anschließend zur Trockene eingeengt Der Rückstand wird fünfmal aus DMF umkristallisiert und abschließend zweimal fraktioniert sublimiert (p ca. 10⁻⁶ mbar, T = 350 - 370 °C). Ausbeute: 81g (110 mmol), 65 % der Theorie: 99.9 % n. HPLC.

Analog dazu werden die folgenden Verbindungen hergestellt:

| | Edukt | Edukt | Produkt | Ausbeute |
|---|---|---|---|---|
| f1 | 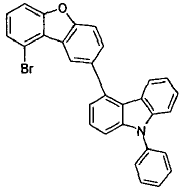 | 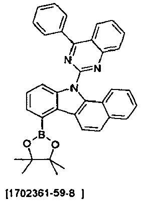 | 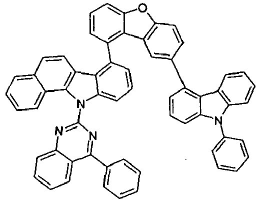 | 61% |
| f2 | 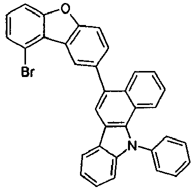 | 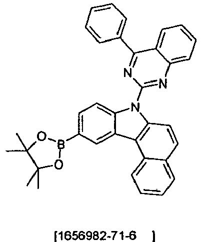 | 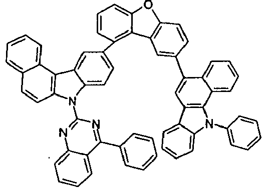 | 53% |
| f3 | 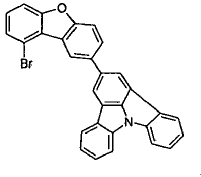 | 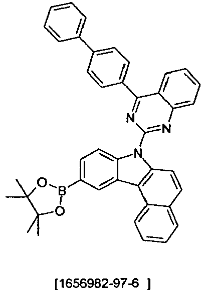 | 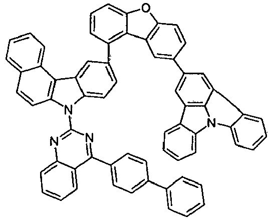 | 54% |
| f4 | 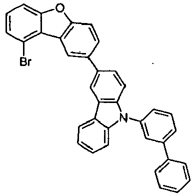 | 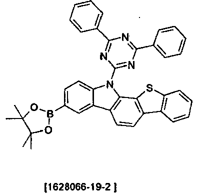 | 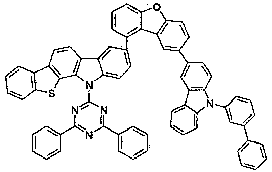 | 65% |
| f5 | 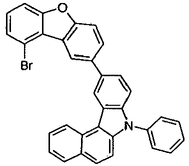 | 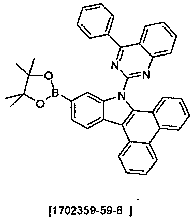 | 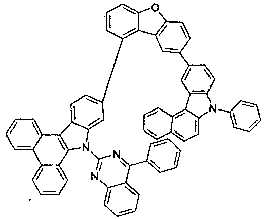 | 67% |
| f6 | 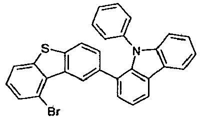 | 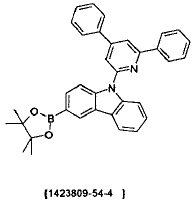 | 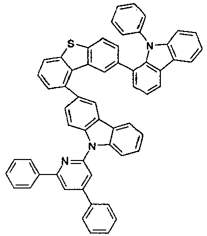 | 57% |
| f7 | 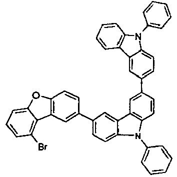 | 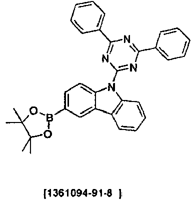 | 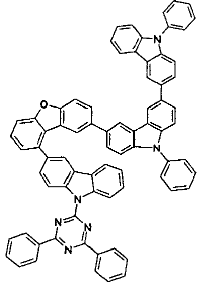 | 63% |
| f8 | 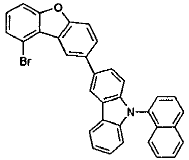 | 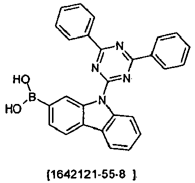 | 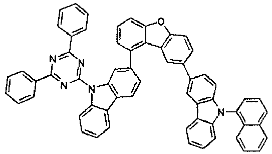 | 63% |
| f9 | 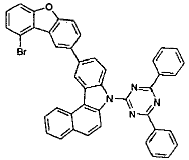 | 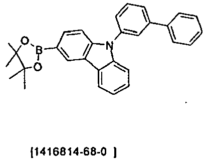 | 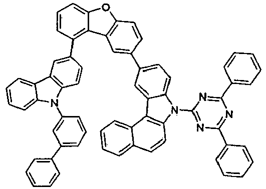 | 68% |
| f10 | 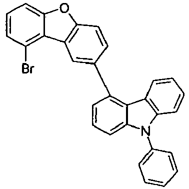 | 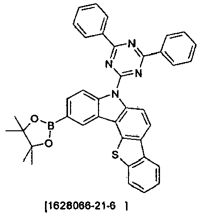 | 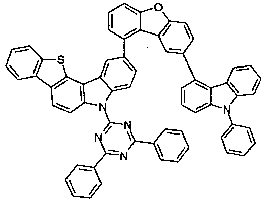 | 64% |
| f11 | 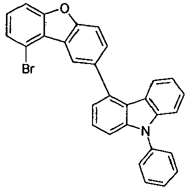 | 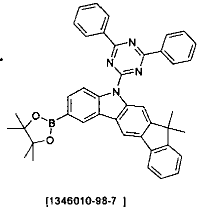 | 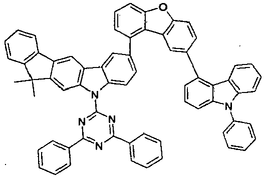 | 66% |
| f12 | 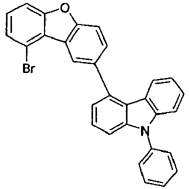 | 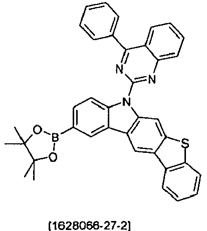 | 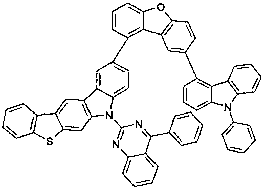 | 68% |
| f13 | 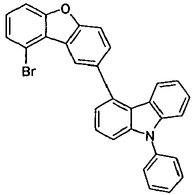 | 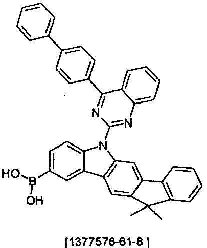 | 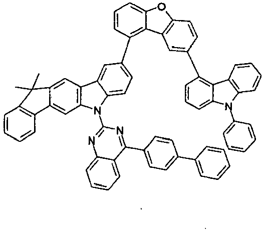 | 60% |
| f14 | 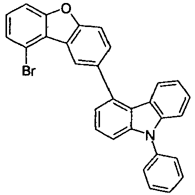 | 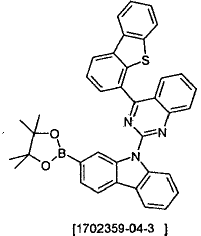 | 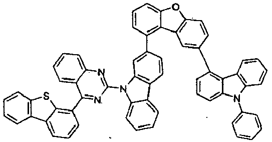 | 58% |
| f15 | 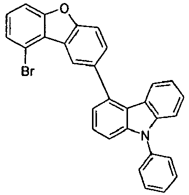 | 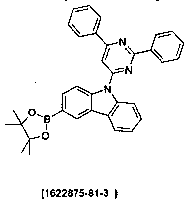 | 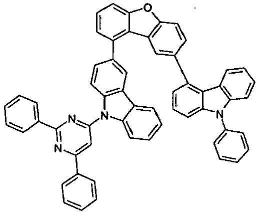 | 61% |
| f16 | 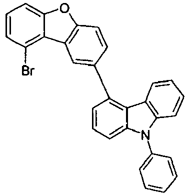 | 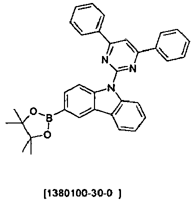 | 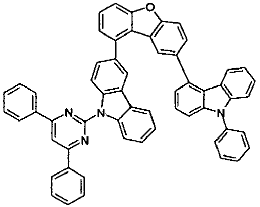 | 63% |
| f17 | 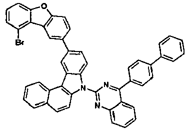 | 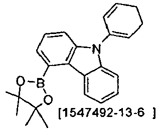 | 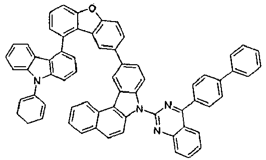 | 63% |
| f18 | 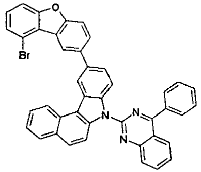 | 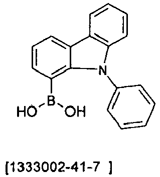 | 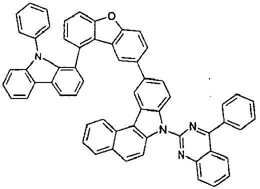 | 65% |
| f19 | 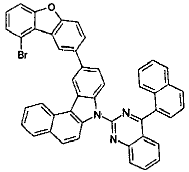 | 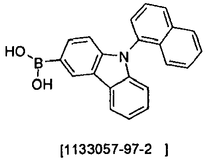 | 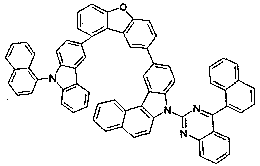 | 60% |
| f20 | 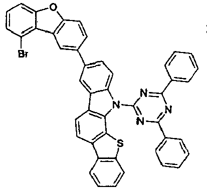 | 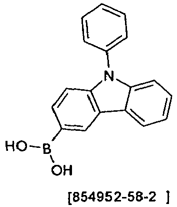 | 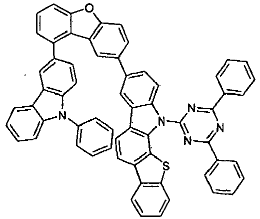 | 58% |
| f21 | 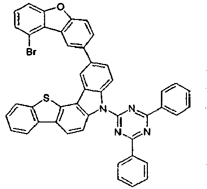 | 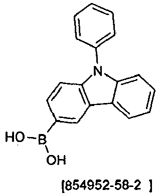 | 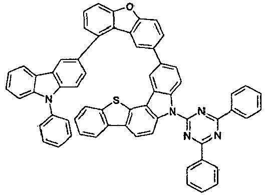 | 57% |
| f22 | 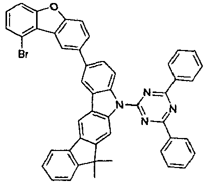 | 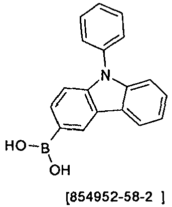 | 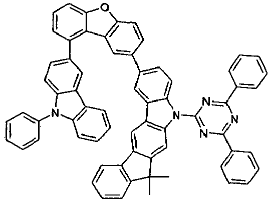 | 56% |
| f23 | 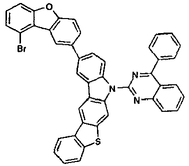 | 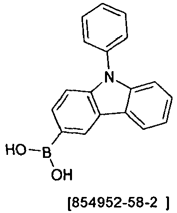 | 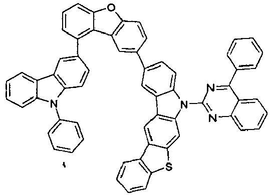 | 51% |
| f24 | 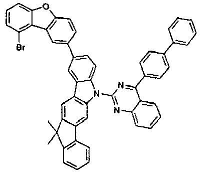 | 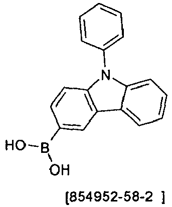 | 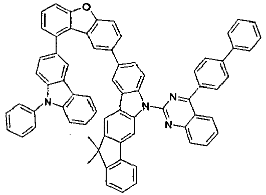 | 53% |
| f25 | 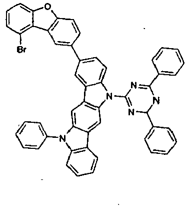 | 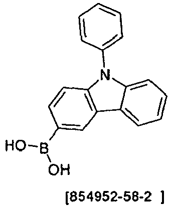 | 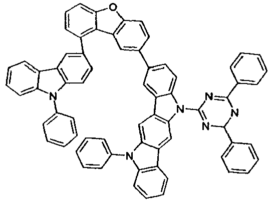 | 56% |
| f26 | 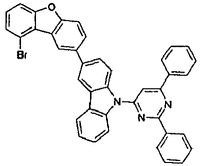 | 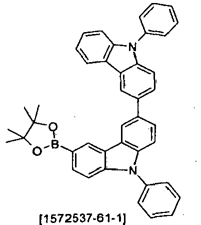 | 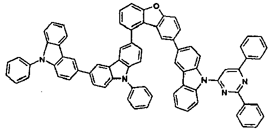 | 56% |
| f27 | 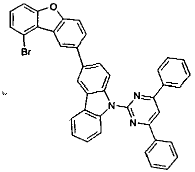 | 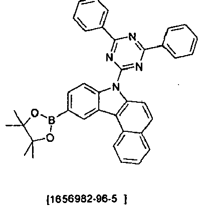 | 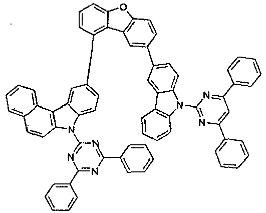 | 70% |
| f28 | 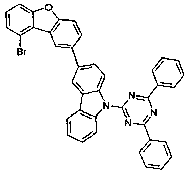 | 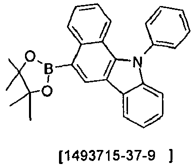 | 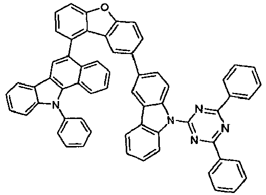 | 73% |
| f29 | 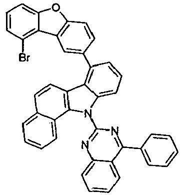 | 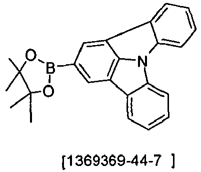 | 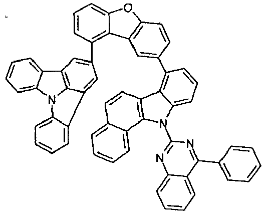 | 72% |
| f30 | 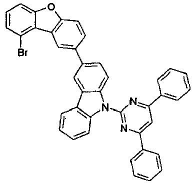 | 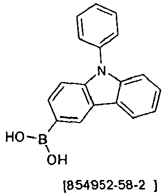 | 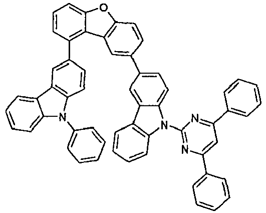 | 73% |
| f31 | 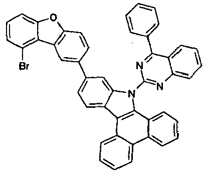 | 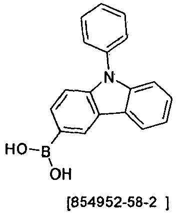 | 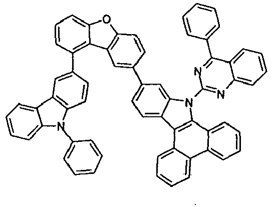 | 56% |
| f32 | 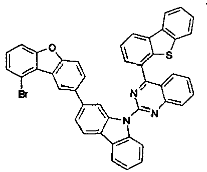 | 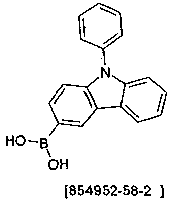 | 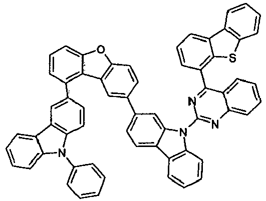 | 55% |
| f33 | 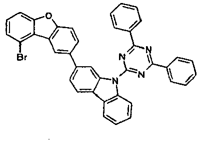 | 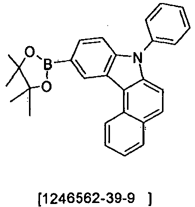 | 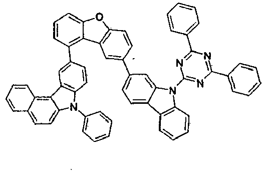 | 62% |
| f34 | 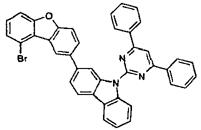 | 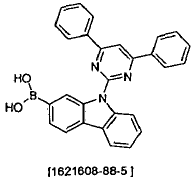 | 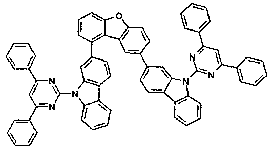 | 60% |
| f35 | 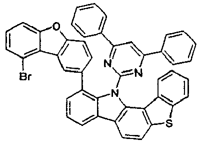 | 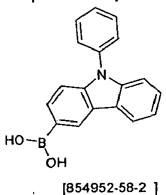 | 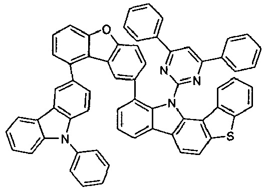 | 45% |
| f36 | 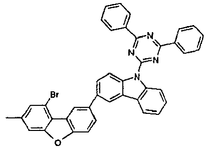 | 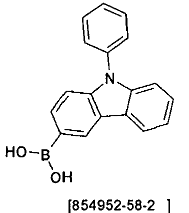 | 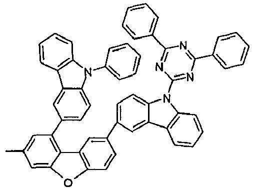 | 57% |
| f37 | 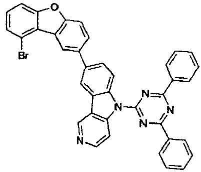 | 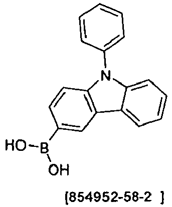 | 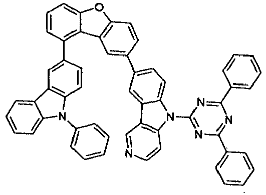 | 62% |
| f38 | 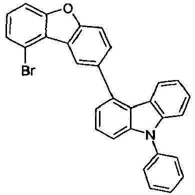 | 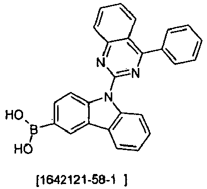 | 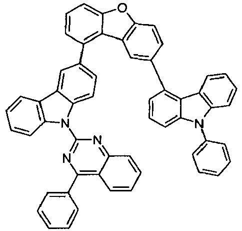 | 65% |
| f39 | 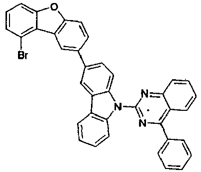 | 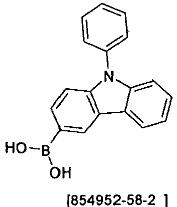 | 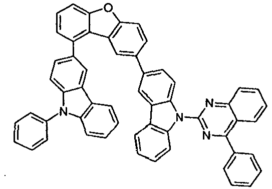 | 67% |
| f40 | 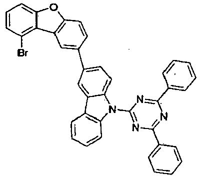 | 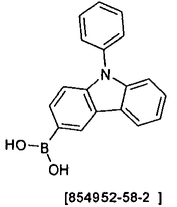 | 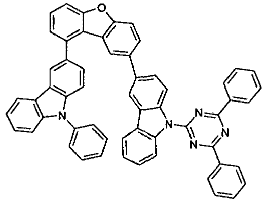 | 72% |

### Herstellung der OLEDs

In den folgenden Beispielen V1 bis E26(siehe Tabellen 1 und 2) werden die Daten verschiedener OLEDs vorgestellt.

**Vorbehandlung für die Beispiele V1-E26:** Glasplättchen, die mit strukturiertem ITO (Indium Zinn Oxid) der Dicke 50 nm beschichtet sind, werden vor der Beschichtung mit einem Sauerstoffplasma, gefolgt von einem Argonplasma, behandelt. Diese mit Plasma behandelten Glasplättchen bilden die Substrate, auf welche die OLEDs aufgebracht werden

Die OLEDs haben prinzipiell folgenden Schichtaufbau: Substrat / Lochinjektionsschicht (HIL) / Lochtransportschicht (HTL) / Elektronenblockierschicht (EBL) / Emissionsschicht (EML) / optionale Lochblockierschicht (HBL) / Elektronentransportschicht (ETL) / optionale Elektroneninjektionsschicht (EIL) und abschließend eine Kathode. Die Kathode wird durch eine 100 nm dicke Aluminiumschicht gebildet. Der genaue Aufbau der OLEDs ist Tabelle 1 zu entnehmen. Die zur Herstellung der OLEDs verwendeten Materialien sind in Tabelle 3 gezeigt.

Alle Materialien werden in einer Vakuumkammer thermisch aufgedampft. Dabei besteht die Emissionsschicht immer aus mindestens einem Matrixmaterial (Hostmaterial, Wirtsmaterial) und einem emittierenden Dotierstoff (Dotand, Emitter), der dem Matrixmaterial bzw. den Matrixmaterialien durch Coverdampfung in einem bestimmten Volumenanteil beigemischt wird. Eine Angabe wie IC5:SdT1:TEG2 (30%:55%:15%) bedeutet hierbei, dass das Material IC5 in einem Volumenanteil von 30%, SdT1 in einem Volumenanteil von 55% und TEG2 in einem Volumenanteil von 15% in der Schicht vorliegt. Analog kann auch die Elektronentransportschicht aus einer Mischung von zwei Materialien bestehen.

Die OLEDs werden standardmäßig charakterisiert. Hierfür werden die Elektrolumineszenzspektren, die Stromeffizienz (gemessen in cd/A) und die externe Quanteneffizienz (EQE, gemessen in Prozent) in Abhängigkeit der Leuchtdichte, berechnet aus Strom-Spannungs-Leuchtdichte-Kennlinien (IUL-Kennlinien) unter Annahme einer lambertschen Abstrahlcharakteristik bestimmt. Die Elektrolumineszenzspektren werden bei einer Leuchtdichte von 1000 cd/m² bestimmt und daraus die CIE 1931 x und y Farbkoordinaten berechnet. Die Angabe U1000 in Tabelle 2 bezeichnet die Spannung, die für eine Leuchtdichte von 1000 cd/m² benötigt wird. SE1000 bezeichnet die Stromeffizienz, die bei 1000 cd/m² erreicht werden. EQE1000 schließlich bezeichnet die externe Quanteneffizienz bei einer Betriebsleuchtdichte von 1000 cd/m².

Die Daten der verschiedenen OLEDs sind in Tabelle 2 zusammengefasst. Die Beispiele V1-V6 sind Vergleichsbeispiele gemäß dem Stand der Technik, die Beispiele E1-E26 zeigen Daten von erfindungsgemäßen OLEDs.

Im Folgenden werden einige der Beispiele näher erläutert, um die Vorteile der erfindungsgemäßen OLED zu verdeutlichen.

### Verwendung von erfindungsgemäßen Verbindungen als Matrixmaterial in phosphoreszenten OLEDs

Die erfindungsgemäßen Materialien ergeben beim Einsatz als Matrixmaterial in Kombination mit einer elektronenleitenden Verbindung (wie z. B. Verbindung IC5 in den unten aufgeführten Beispielen) in der Emissionsschicht (EML) in phosphoreszierenden OLEDs wesentliche Verbesserungen gegenüber dem Stand der Technik, vor allem bezüglich der externen Quanteneffizienz der OLEDs. Durch Einsatz der erfindungsgemäßen Verbindungen f40, f, f30, f16 und f21 (gemäß den oben angegebenen Beispielen) lässt sich eine Verbesserung der EQE um ca. 10 bis 50 % gegenüber den Verbindungen aus dem Stand der Technik SdT1, SdT2, SdT3, SdT4, SdT5 und SdT6 beobachten (Vergleich der Beispiele V1, V2, V3, V4, V5 und V6 mit Beispielen E1, E2, E3, E15 und E16).

**Tabelle 1: Aufbau der OLEDs**

| Bsp | HIL Dicke | HTL Dicke | EBL Dicke | EML Dicke | HBL Dicke | ETL Dicke | EIL Dicke |
|---|---|---|---|---|---|---|---|
| V1 | HATCN | SpMA1 | SpMA3 | IC5:SdT1:TEG2 | --- | ST2:LiQ | --- |
| | 5nm | 230nm | 20nm | (30%:55%:15%) 30nm | | (50%:50%) 40nm | |
| V2 | HATCN | SpMA1 | SpMA3 | IC5:SdT2:TEG2 | --- | ST2:LiQ | --- |
| | 5nm | 230nm | 20nm | (30%:55%:15%) 30nm | | (50%:50%) 40nm | |
| V3 | HATCN | SpMA1 | SpMA3 | IC5:SdT3:TEG2 | --- | ST2:LiQ | --- |
| | 5nm | 230nm | 20nm | (30%:55%:15%) 30nm | | (50%:50%) 40nm | |
| V4 | HATCN | SpMA1 | SpMA3 | IC5:SdT4:TEG2 | --- | ST2:LiQ | --- |
| | 5nm | 230nm | 20nm | (30%:55%:15%) 30nm | | (50%:50%) 40nm | |
| V5 | HATCN | SpMA1 | SpMA3 | IC5:SdT5:TEG2 | --- | ST2:LiQ | --- |
| | 5nm | 230nm | 20nm | (30%:55%:15%) 30nm | | (50%:50%) 40nm | |
| V6 | HATCN | SpMA1 | SpMA3 | IC5:SdT6:TEG2 | --- | ST2:LiQ | --- |
| | 5nm | 230nm | 20nm | (30%:55%:15%) 30nm | | (50%:50%) 40nm | |
| E1 | HATCN | SpMA1 | SpMA3 | IC5:f40:TEG2 | --- | ST2:LiQ | --- |
| | 5nm | 230nm | 20nm | (30%:55%:15%) 30nm | | (50%:50%) 40nm | |
| E2 | HATCN | SpMA1 | SpMA3 | IC5:f:TEG2 | --- | ST2:LiQ | --- |
| | 5nm | 230nm | 20nm | (30%:55%:15%) 30nm | | (50%:50%) 40nm | |
| E3 | HATCN | SpMA1 | SpMA3 | IC5:f30:TEG2 | --- | ST2:LiQ | --- |
| | 5nm | 230nm | 20nm | (30%:55%:15%) 30nm | | (50%:50%) 40nm | |
| E4 | HATCN | SpMA1 | SpMA3 | f1:TER5 | --- | ST2:LiQ | --- |
| | 5nm | 125nm | 10nm | (95%:5%) 40nm | | (50%:50%) 35nm | |
| E5 | HATCN | SpMA1 | SpMA3 | f2:TER5 | --- | ST2:LiQ | --- |
| | 5nm | 125nm | 10nm | (95%:5%) 40nm | | (50%:50%) 35nm | |
| E6 | HATCN | SpMA1 | SpMA3 | f4:TEG2 | --- | ST2:LiQ | --- |
| | 5nm | 230nm | 20nm | (90%:10%) 30nm | | (50%:50%) 40nm | |
| E7 | HATCN | SpMA1 | SpMA3 | f5:TER5 | --- | ST2:LiQ | --- |
| | 5nm | 125nm | 10nm | (95%:5%) 40nm | | (50%:50%) 35nm | |
| E8 | HATCN | SpMA1 | SpMA3 | f7:TEG2 | --- | ST2:LiQ | --- |
| | 5nm | 230nm | 20nm | (90%:10%) 30nm | | (50%:50%) 40nm | |
| E9 | HATCN | SpMA1 | SpMA3 | f8:TEG2 | --- | ST2:LiQ | --- |
| | 5nm | 230nm | 20nm | (90%:10%) 30nm | | (50%:50%) 40nm | |
| E10 | HATCN | SpMA1 | SpMA3 | f9:TER5 | --- | ST2:LiQ | --- |
| | 5nm | 125nm | 10nm | (95%:5%) 40nm | | (50%:50%) 35nm | |
| E11 | HATCN | SpMA1 | SpMA3 | f10:TEG2 | --- | ST2:LiQ | --- |
| | 5nm | 230nm | 20nm | (90%:10%) 30nm | | (50%:50%) 40nm | |
| E12 | HATCN | SpMA1 | SpMA3 | f11:TEG2 | --- | ST2:LiQ | --- |
| | 5nm | 230nm | 20nm | (90%:10%) 30nm | | (50%:50%) 40nm | |
| E13 | HATCN | SpMA1 | SpMA3 | f12:TER5 | --- | ST2:LiQ | --- |
| | 5nm | 125nm | 10nm | (95%:5%) 40nm | | (50%:50%) 35nm | |
| E14 | HATCN | SpMA1 | SpMA3 | f13:TER5 | --- | ST2:LiQ | --- |
| | 5nm | 125nm | 10nm | (95%:5%) 40nm | | (50%:50%) 35nm | |
| E15 | HATCN | SpMA1 | SpMA3 | IC5:f16:TEG2 | --- | ST2:LiQ | --- |
| | 5nm | 230nm | 20nm | (30%:55%:15%) 30nm | | (50%:50%) 40nm | |
| E16 | HATCN | SpMA1 | SpMA3 | f17:TER5 | --- | ST2:LiQ | --- |
| | 5nm | 125nm | 10nm | (95%:5%) 40nm | | (50%:50%) 35nm | |
| E17 | HATCN | SpMA1 | SpMA3 | f19:TER5 | --- | ST2:LiQ | --- |
| | 5nm | 125nm | 10nm | (95%:5%) 40nm | | (50%:50%) 35nm | |
| E18 | HATCN | SpMA1 | SpMA3 | IC5:f21:TEG2 | --- | ST2:LiQ | --- |
| | 5nm | 230nm | 20nm | (30%:55%:15%) 30nm | | (50%:50%) 40nm | |
| E19 | HATCN | SpMA1 | SpMA3 | f24:TER5 | --- | ST2:LiQ | --- |
| | 5nm | 125nm | 10nm | (95%:5%) 40nm | | (50%:50%) 35nm | |
| E20 | HATCN | SpMA1 | SpMA3 | f25:TEG2 | --- | ST2:LiQ | --- |
| | 5nm | 230nm | 20nm | (90%:10%) 30nm | | (50%:50%) 40nm | |
| E21 | HATCN | SpMA1 | SpMA3 | f26:TEG2 | --- | ST2:LiQ | --- |
| | 5nm | 230nm | 20nm | (90%:10%) 30nm | | (50%:50%) 40nm | |
| E22 | HATCN | SpMA1 | SpMA3 | f27:TER5 | --- | ST2:LiQ | --- |
| | 5nm | 125nm | 10nm | (95%:5%) 40nm | | (50%:50%) 35nm | |
| E23 | HATCN | SpMA1 | SpMA3 | f28:TER5 | --- | ST2:LiQ | --- |
| | 5nm | 125nm | 10nm | (95%:5%) 40nm | | (50%:50%) 35nm | |
| E24 | HATCN | SpMA1 | SpMA3 | f31:TER5 | --- | ST2:LiQ | --- |
| | 5nm | 125nm | 10nm | (95%:5%) 40nm | | (50%:50%) 35nm | |
| E25 | HATCN | SpMA1 | SpMA3 | f33:TER5 | --- | ST2:LiQ | --- |
| | 5nm | 125nm | 10nm | (95%:5%) 40nm | | (50%:50%) 35nm | |
| E26 | HATCN | SpMA1 | SpMA3 | f39:TER5 | --- | ST2:LiQ | --- |
| | 5nm | 125nm | 10nm | (95%:5%) 40nm | | (50%:50%) 35nm | |

**Tabelle 2: Daten der OLEDs**

| Bsp. | U1000 (V) | SE1000 (cd/A) | EQE 1000 | CIE x/y bei 1000 cd/m² |
|---|---|---|---|---|
| V1 | 3.6 | 44 | 12.2% | 0.34/0.62 |
| V2 | 3.7 | 44 | 12.1% | 0.34/0.62 |
| V3 | 3.5 | 45 | 12.5% | 0.34/0.62 |
| V4 | 3.6 | 44 | 12.3% | 0.34/0.62 |
| V5 | 3.5 | 63 | 16.9% | 0.32/0.64 |
| V6 | 3.6 | 60 | 16.7% | 0.33/0.63 |
| E1 | 3.5 | 68 | 18.3% | 0.34/0.62 |
| E2 | 3.5 | 67 | 18.2% | 0.33/0.63 |
| E3 | 3.6 | 69 | 18.5% | 0.33/0.63 |
| E4 | 3.6 | 23 | 22.4% | 0.67/0.33 |
| E5 | 3.5 | 24 | 22.7% | 0.67/0.33 |
| E6 | 3.5 | 69 | 18.5% | 0.34/0.63 |
| E7 | 3.5 | 23 | 22.5% | 0.67/0.33 |
| E8 | 3.4 | 66 | 17.9% | 0.32/0.64 |
| E9 | 3.6 | 66 | 18.0% | 0.33/0.63 |
| E10 | 3.3 | 25 | 22.9% | 0.67/0.33 |
| E11 | 3.6 | 69 | 18.7% | 0.33/0.63 |
| E12 | 3.5 | 68 | 18.6% | 0.32/0.63 |
| E13 | 3.6 | 24 | 22.7% | 0.67/0.33 |
| E14 | 3.7 | 27 | 23.0% | 0.67/0.33 |
| E15 | 3.6 | 67 | 18.4% | 0.32/0.63 |
| E16 | 3.5 | 23 | 22.5% | 0.67/0.33 |
| E17 | 3.6 | 22 | 22.2% | 0.67/0.33 |
| E18 | 3.6 | 67 | 18.2% | 0.31/0.64 |
| E19 | 3.6 | 27 | 23.1% | 0.67/0.33 |
| E20 | 3.4 | 68 | 18.7% | 0.31/0.64 |
| E21 | 3.5 | 65 | 18.1% | 0.31/0.64 |
| E22 | 3.3 | 22 | 21.9% | 0.67/0.33 |
| E23 | 3.4 | 23 | 22.5% | 0.67/0.33 |
| E24 | 3.5 | 23 | 22.4% | 0.67/0.33 |
| E25 | 3.4 | 24 | 22.8% | 0.67/0.33 |
| E26 | 3.5 | 28 | 23.1% | 0.67/0.33 |

**Tabelle 3: Strukturformeln der Materialien für die OLEDs**

| | |
|---|---|
| 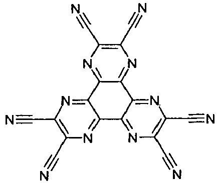 | 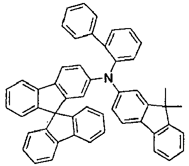 |
| HATCN | SpMA1 |
| 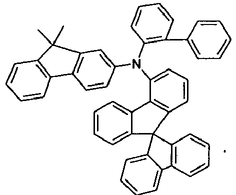 | 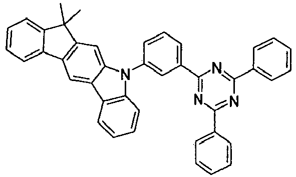 |
| SpMA3 | IC5 |
| 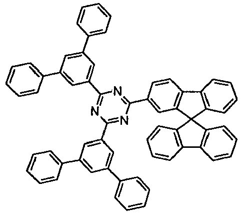 | 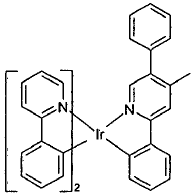 |
| ST2 | TEG2 |
| 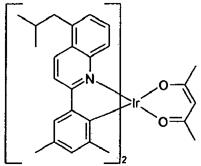 | 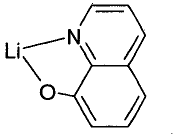 |
| TER5 | LiQ |
| 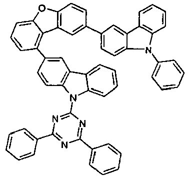 | 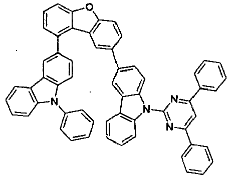 |
| f | f30 |
| 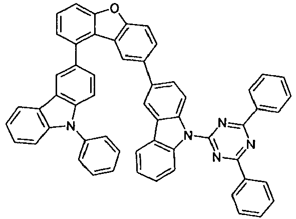 | 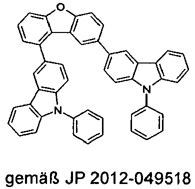 |
| f40 | SdT1 |
| 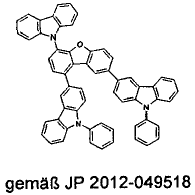 | 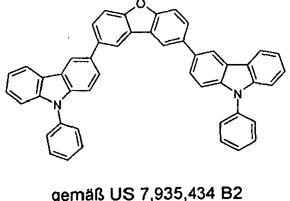 |
| SdT2 | SdT3 |
| 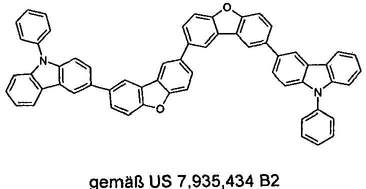 | 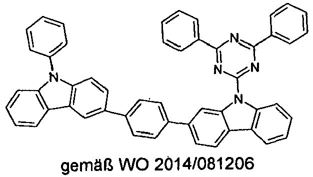 |
| SdT4 | SdT5 |
| 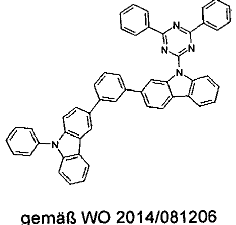 | 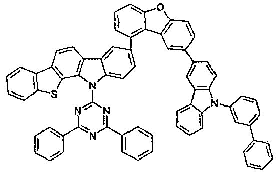 |
| SdT6 | f4 |
| 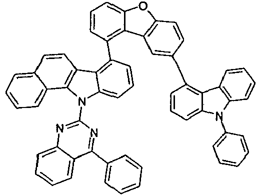 | 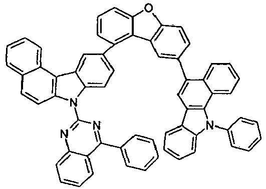 |
| f1 | f2 |
| 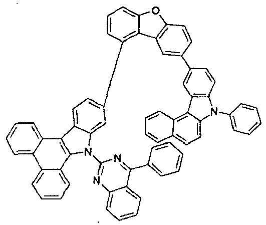 | 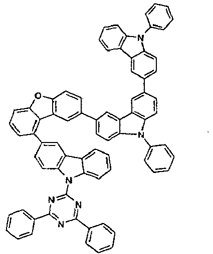 |
| f5 | f7 |
| 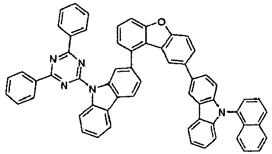 | 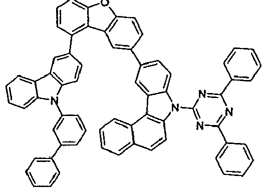 |
| f8 | f9 |
| 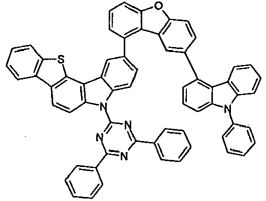 | 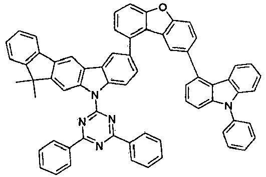 |
| f10 | f11 |
| 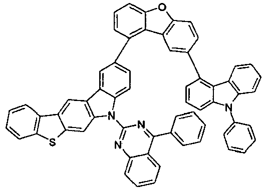 | 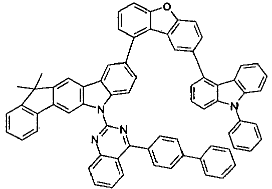 |
| f12 | f13 |
| 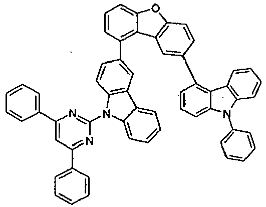 | 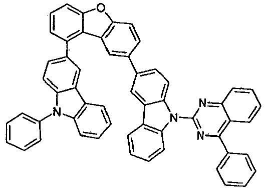 |
| f16 | f39 |
| 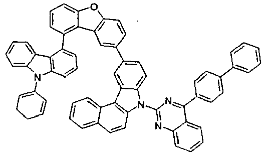 | 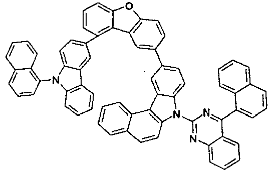 |
| f17 | f19 |
| 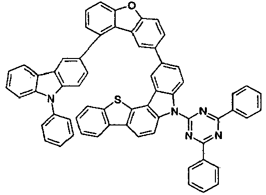 | 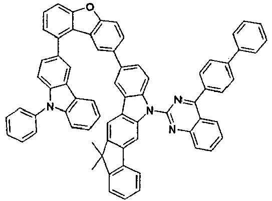 |
| f21 | f24 |
| 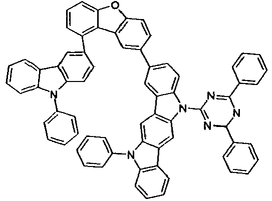 | 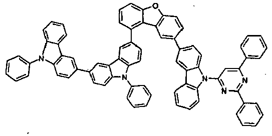 |
| f25 | f26 |
| 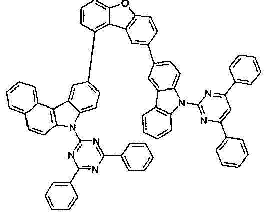 | 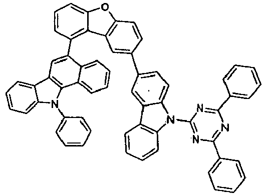 |
| f27 | f28 |
| 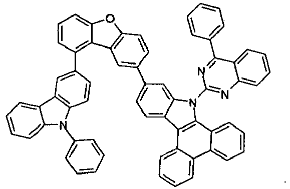 | 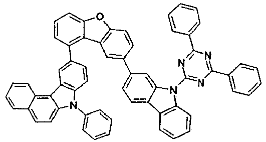 |
| f31 | f33 |

## Patentansprüche

1. Verbindung gemäß Formel (1), 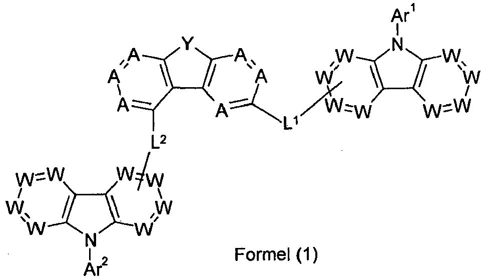 wobei für die verwendeten Symbole gilt:
A ist bei jedem Auftreten gleich oder verschieden CR oder N, wobei maximal zwei Gruppen A pro Cyclus für N stehen;
Y ist O oder S;
W ist bei jedem Auftreten gleich oder verschieden CR oder N, wobei maximal zwei Gruppen W pro Cyclus für N stehen und wobei W für C steht, wenn an diese Position eine Gruppe L¹ bzw. L² gebunden ist, oder zwei benachbarte Gruppen W stehen zusammen für eine Gruppe der folgenden Formel (2) oder (3), wobei jede der beiden Carbazolylderivat-Gruppen in der Verbindung der Formel (1) maximal zwei Gruppen der Formel (2) bzw. Formel (3) aufweist, 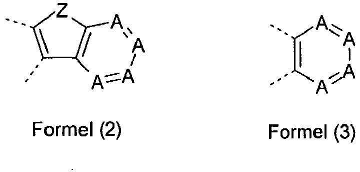 wobei die gestrichelten Bindungen die Verknüpfung dieser Gruppe andeuten, A die oben genannten Bedeutungen aufweist und Z für NR, CR₂, O oder S steht;
Ar¹, Ar² ist ein aromatisches Ringsystem mit 5 bis 30 aromatischen Ringatomen oder eine Dibenzofuran- oder Dibenzothiophengruppe, wobei das aromatische Ringsystem bzw. die Dibenzofuran- oder Dibenzothiophengruppe jeweils durch einen oder mehrere nicht-aromatische Reste R substituiert sein kann, oder ist eine Gruppe gemäß einer der folgenden Formeln (4) oder (5), 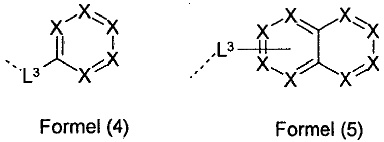 wobei die gestrichelte Bindung die Bindung an das Stickstoffatom darstellt;
mit der Maßgabe, dass genau eine der Gruppen Ar¹ oder Ar² für eine Gruppe gemäß einer der Formeln (4) oder (5) steht;
X ist bei jedem Auftreten gleich oder verschieden CR oder N, wobei X in Formel (5) für C steht, wenn die Gruppe der Formel (5) in dieser Position mit L³ verknüpft ist, mit der Maßgabe, dass in Formel (4) zwei oder drei Gruppen X für N stehen und in Formel (5) eine, zwei oder drei Gruppen X für N stehen;
L¹, L², L³ ist bei jedem Auftreten gleich oder verschieden eine Einfachbindung oder ein aromatisches oder heteroaromatisches Ringsystem mit 5 bis 30 aromatischen Ringatomen, das durch einen oder mehrere Reste R substituiert sein kann;
R ist bei jedem Auftreten gleich oder verschieden ausgewählt aus der Gruppe bestehend aus H, D, F, Cl, Br, I, CN, NO₂, N(R¹)₂, C(=O)R¹, P(=O)(R¹)₂ P(R¹)₂, B(R¹)₂, Si(R¹)₃, einer geradkettigen Alkyl-, Alkoxy- oder Thioalkylgruppe mit 1 bis 20 C-Atomen oder einer verzweigten oder cyclischen Alkyl-, Alkoxy- oder Thioalkylgruppe mit 3 bis 20 C-Atomen oder einer Alkenylgruppe mit 2 bis 20 C-Atomen, die jeweils mit einem oder mehreren Resten R¹ substituiert sein kann, wobei eine oder mehrere nicht-benachbarte CH₂-Gruppen durch R¹C=GR¹, Si(R¹)₂, C=O, C=S, C=NR¹, P(=O)(R¹), SO, SO₂, NR¹, O, S oder CONR¹ ersetzt sein können und wobei ein oder mehrere H-Atome durch D, F, Cl, Br, I, CN oder NO₂ ersetzt sein können, einem aromatischen oder heteroaromatischen Ringsystem mit 5 bis 40 aromatischen Ringatomen, das jeweils mit einem oder mehreren Resten R¹ substituiert sein kann, oder einer Aryloxy- oder Heteroaryloxygruppe mit 5 bis 40 aromatischen Ringatomen, die mit einem oder mehreren Resten R¹ substituiert sein kann; dabei können optional zwei Substituenten R, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten R¹ substituiert sein kann;
R¹ ist bei jedem Auftreten gleich oder verschieden ausgewählt aus der Gruppe bestehend aus H, D, F, Cl, Br, I, CN, N(R²)₂, C(=O)R¹, einer geradkettigen Alkylgruppe mit 1 bis 10 C-Atomen oder einer verzweigten oder cyclischen Alkylgruppe mit 3 bis 10 C-Atomen oder einer Alkenylgruppe mit 2 bis 10 C-Atomen, die jeweils mit einem oder mehreren Resten R² substituiert sein kann, wobei ein oder mehrere H-Atome durch D, F, Cl, Br, I oder CN ersetzt sein können, oder einem aromatischen oder heteroaromatischen Ringsystem mit 5 bis 40 aromatischen Ringatomen, das jeweils mit einem oder mehreren Resten R² substituiert sein kann; dabei können optional zwei Substituenten R¹, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten R² substituiert sein kann;
R² ist bei jedem Auftreten gleich oder verschieden ausgewählt aus der Gruppe bestehend aus H, D, F, CN, einem aliphatischen Kohlenwasserstoffrest mit 1 bis 20 C-Atomen oder einem aromatischen oder heteroaromatischen Ringsystem mit 5 bis 30 aromatischen Ringatomen, in dem ein oder mehrere H-Atome durch D, F, CN ersetzt sein können und das durch ein oder mehrere Alkylgruppen mit jeweils 1 bis 4 Kohlenstoffatomen substituiert sein kann; dabei können zwei oder mehr benachbarte Substituenten R² miteinander ein mono- oder polycyclisches, aliphatisches Ringsystem bilden.

2. Verbindung nach Anspruch 1, ausgewählt aus den Verbindungen der Formeln (7a), (7b), (7c), (7d) und (7e), 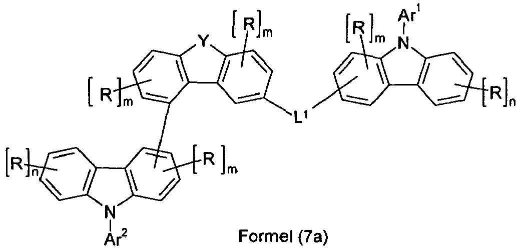 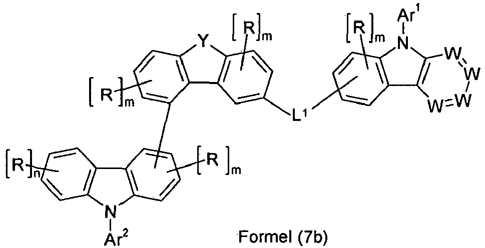 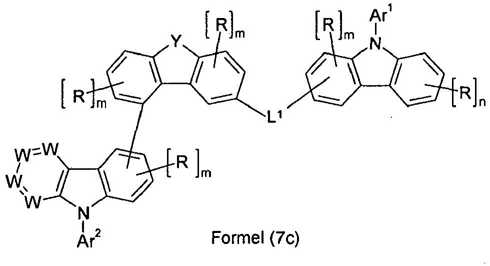 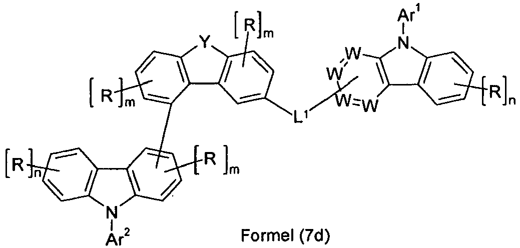 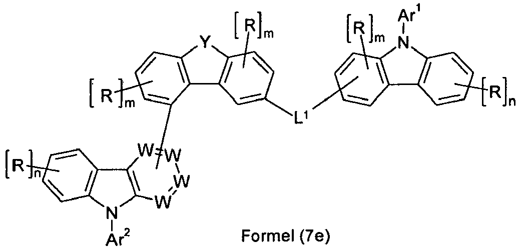 wobei gilt:
W zwei benachbarte Gruppen W stehen zusammen für eine Gruppe der folgenden Formel (2a) oder (3a) und die anderen beiden Gruppen W stehen für CR, wobei W in Formel (7d) für C steht, wenn an diese Position eine Gruppe L¹ gebunden ist, und in Formel (7e) für C steht, wenn an diese Position das Dibenzofuran bzw. Dibenzothiophen gebunden ist, 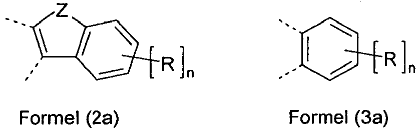 wobei die gestrichelten Bindungen die Verknüpfung dieser Gruppe andeuten;
n ist bei jedem Auftreten gleich oder verschieden 0, 1, 2, 3 oder 4;
m ist bei jedem Auftreten gleich oder verschieden 0, 1, 2 oder 3;
die weiteren verwendeten Symbole weisen die in Anspruch 1 genannten Bedeutungen auf.

3. Verbindung nach Anspruch 1 oder 2, **dadurch gekennzeichnet, dass** L¹ und L² für Einfachbindungen stehen.

4. Verbindung nach einem oder mehreren der Ansprüche 1 bis 3, ausgewählt aus den Verbindungen der Formeln (9a), (9b), (9c), (9d) und (9e), 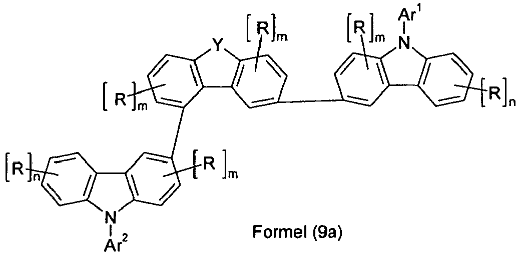 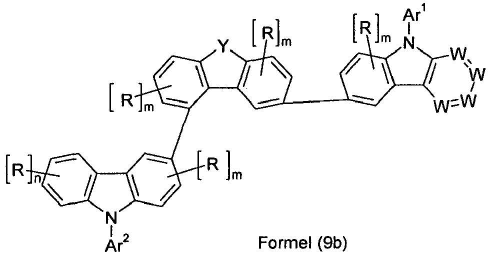 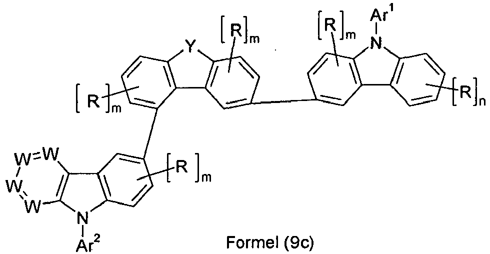 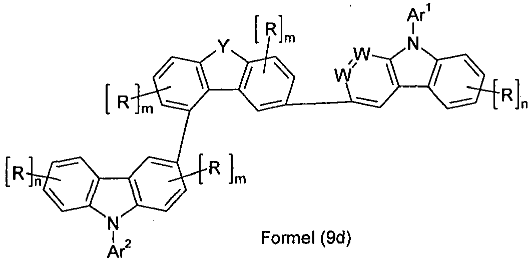 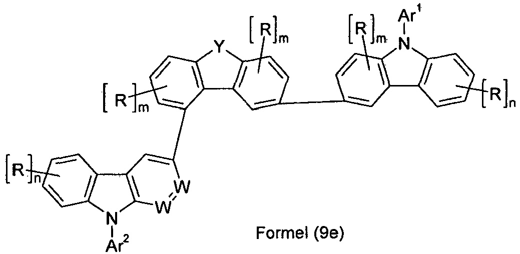 wobei W, n und m die in Anspruch 2 und die weiteren verwendeten Symbole die in Anspruch 1 genannten Bedeutungen aufweisen.

5. Verbindung nach einem oder mehreren der Ansprüche 1 bis 4, **dadurch gekennzeichnet, dass** die Gruppe der Formel (4) ausgewählt ist aus den Strukturen der Formeln (4-1) bis (4-3) und dass die Gruppe der Formel (5) ausgewählt ist aus den Strukturen der Formeln (5-1) bis (5-18), 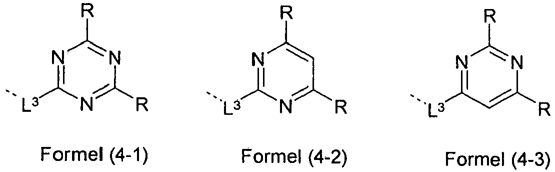 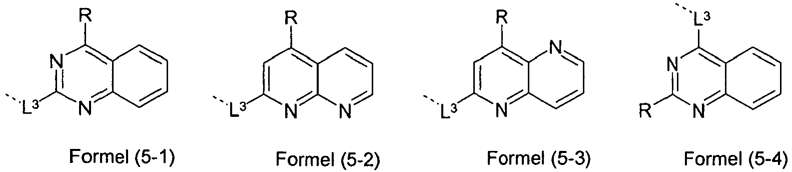 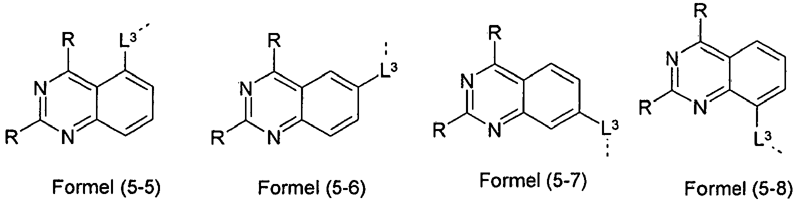 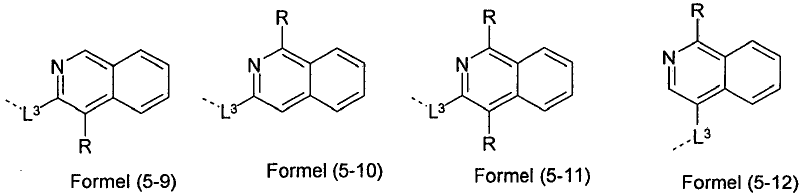 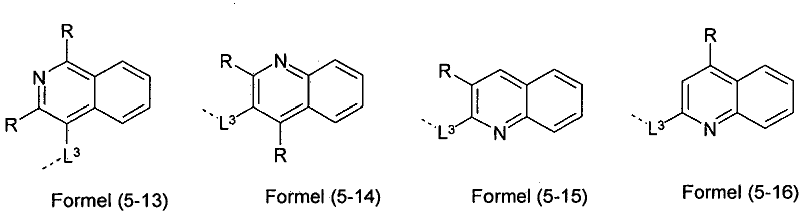 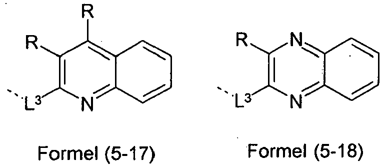 wobei die gestrichelte Bindung die Bindung an das Stickstoffatom darstellt und weiterhin gilt:
R ist bei jedem Auftreten gleich oder verschieden H, eine Alkylgruppe mit 1 bis 10 C-Atomen, die durch eine oder mehrere Gruppen R¹ substituiert sein kann, oder ein aromatisches oder heteroaromatisches Ringsystem mit 5 bis 40 aromatischen Ringatomen, das jeweils mit einem oder mehreren Resten R¹ substituiert sein kann.

6. Verbindung nach einem oder mehreren der Ansprüche 1 bis 5, **dadurch gekennzeichnet, dass** L³ für eine Einfachbindung oder für ein aromatisches Ringsystem mit 6 bis 12 aromatischen Ringatomen steht.

7. Verbindung nach einem oder mehreren der Ansprüche 1 bis 6, **dadurch gekennzeichnet, dass** die Gruppe Ar¹ bzw. Ar², die nicht für eine Gruppe der Formel (4) oder (5) steht, ausgewählt ist aus der Gruppe bestehend aus Phenyl, ortho-, meta- oder para-Biphenyl, Terphenyl, Quaterphenyl, 1-, 2-, 3- oder 4-Fluorenyl, 1-, 2-, 3- oder 4-Spirobifluorenyl, 1-, 2-, 3- oder 4-Dibenzofuranyl und 1-, 2-, 3- oder 4-Dibenzothienyl, die jeweils durch einen oder mehrere nicht-aromatische Reste R substituiert sein können.

8. Verbindung nach einem oder mehreren der Ansprüche 2 bis 7, **dadurch gekennzeichnet, dass** der Index n gleich oder verschieden bei jedem Auftreten 0, 1, 2 oder 3 ist, bevorzugt 0 oder 1, und dass der Index m gleich oder verschieden bei jedem Auftreten 0, 1 oder 2 ist, bevorzugt 0.

9. Verbindung nach einem oder mehreren der Ansprüche 1 bis 8, **dadurch gekennzeichnet, dass** R ausgewählt ist aus der Gruppe bestehend aus H, D, F, CN, N(R¹)₂, C(=O)R¹, P(=O)(R¹)₂, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R¹ substituiert sein kann, wobei eine oder mehrere nicht-benachbarte CH₂-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten R¹ substituiert sein kann; dabei können optional zwei Substituenten R, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten R¹ substituiert sein kann.

10. Verbindung nach einem oder mehreren der Ansprüche 1 bis 9, ausgewählt aus den Verbindungen gemäß den Formeln (10) und (11), 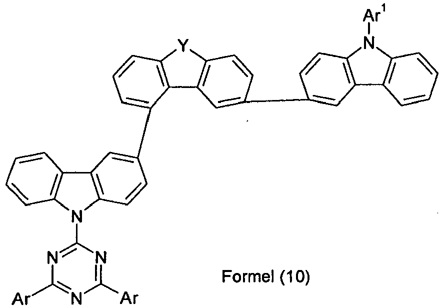 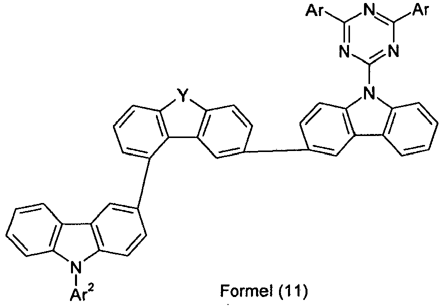 wobei die verwendeten Symbole die in Anspruch 1 genannten Bedeutungen aufweisen und Ar gleich oder verschieden bei jedem Auftreten für ein aromatisches oder heteroaromatisches Ringsystem mit 5 bis 40 aromatischen Ringatomen steht, welches mit einem oder mehreren Resten R¹ substituiert sein kann.

11. Verfahren zur Herstellung einer Verbindung nach einem oder mehreren der Ansprüche 1 bis 10 durch Umsetzung eines gegebenenfalls substituierten 1,8-Dihalogendibenzofurans bzw. 1,8-Dihalogendibenzothiophens mit einem Carbazolderivat, gefolgt durch Umsetzung mit dem anderen Carbazolderivat, wobei die Umsetzungen mit den Carbazolderivaten jeweils C-C-Kupplungen darstellen.

12. Formulierung, enthaltend mindestens eine Verbindung nach einem oder mehreren der Ansprüche 1 bis 10 und ein Lösemittel und/oder mindestens eine weitere organische oder anorganische Verbindung.

13. Verwendung einer Verbindung nach einem oder mehreren der Ansprüche 1 bis 10 in einer elektronischen Vorrichtung.

14. Elektronische Vorrichtung, bevorzugt ausgewählt aus der Gruppe bestehend aus organischen Elektrolumineszenzvorrichtungen, organischen integrierten Schaltungen, organischen Feld-Effekt-Transistoren, organischen Dünnfilmtransistoren, organischen lichtemittierenden Transistoren, organischen Solarzellen, organischen farbstoff-sensibilisierten Solarzellen, organischen optischen Detektoren, organischen Photorezeptoren, organischen Feld-Quench-Devices, lichtemittierenden elektrochemischen Zellen, organischen Laserdioden und "organic plasmon emitting devices" enthaltend mindestens eine Verbindung nach einem oder mehreren der Ansprüche 1 bis 10.

15. Elektronische Vorrichtung nach Anspruch 14, **dadurch gekennzeichnet, dass** es sich um eine organische Elektrolumineszenzvorrichtung handelt und die Verbindung nach einem oder mehreren der Ansprüche 1 bis 10 als Matrixmaterial für fluoreszierende oder phosphoreszierende Emitter in einer emittierenden Schicht und/oder als Elektronentransport- oder Lochblockiermaterial in einer Elektronentransportschicht und/oder in einer lochblockierenden Schicht eingesetzt wird.

## Claims

1. Compound of formula (1) 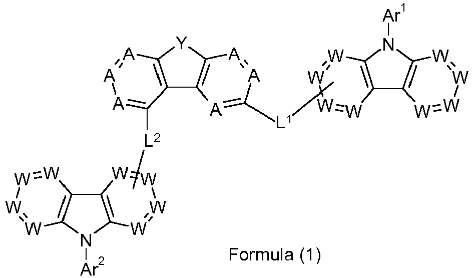 where the symbols used are as follows:
A is the same or different at each instance and is CR or N, where not more than two A symbols per cycle are N;
Y is O or S;
W is the same or different at each instance and is CR or N, where not more than two W groups per cycle are N and where W is C when an L¹ or L² group is bonded to this position, or two adjacent W groups together are a group of the following formula (2) or (3), where each of the two carbazolyl derivative groups in the compound of the formula (1) have not more than two groups of the formula (2) or formula (3): 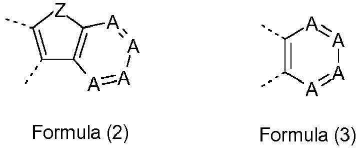 where the dotted bonds indicate the linkage of this group, A has definitions given above and Z is NR, CR₂, O or S;
Ar¹, Ar² is an aromatic ring system having 5 to 30 aromatic ring atoms or a dibenzofuran or dibenzothiophene group, where the aromatic ring system or the dibenzofuran or dibenzothiophene group may be substituted in each case by one or more nonaromatic R radicals, or is a group of one of the following formulae (4) and (5): 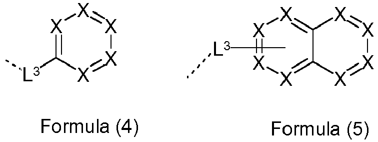 where the dotted bond represents the bond to the nitrogen atom;
with the proviso that exactly one of the Ar¹ and Ar² groups is a group of one of the formulae (4) and (5);
X is the same or different at each instance and is CR or N, where X in formula (5) is C when the group of the formula (5) in this position is joined to L³, with the proviso that, in formula (4), two or three X groups are N and, in formula (5), one, two or three X groups are N;
L¹, L², L³ is the same or different at each instance and is a single bond or an aromatic or heteroaromatic ring system which has 5 to 30 aromatic ring atoms and may be substituted by one or more R radicals;
R is the same or different at each instance and is selected from the group consisting of H, D, F, Cl, Br, I, CN, NO₂, N(R¹)₂, C(=O)R¹, P(=O)(R¹)₂ P(R¹)₂, B(R¹)₂, Si(R¹)₃, a straight-chain alkyl, alkoxy or thioalkyl group having 1 to 20 carbon atoms or a branched or cyclic alkyl, alkoxy or thioalkyl group having 3 to 20 carbon atoms or an alkenyl group having 2 to 20 carbon atoms, each of which may be substituted by one or more R¹ radicals, where one or more nonadjacent CH₂ groups may be replaced by R¹C=CR¹, Si(R¹)₂, C=O, C=S, C=NR¹, P(=O)(R¹), SO, SO₂, NR¹, O, S or CONR¹ and where one or more hydrogen atoms may be replaced by D, F, Cl, Br, I, CN or NO₂, an aromatic or heteroaromatic ring system which has 5 to 40 aromatic ring atoms and may be substituted in each case by one or more R¹ radicals, or an aryloxy or heteroaryloxy group which has 5 to 40 aromatic ring atoms and may be substituted by one or more R¹ radicals; at the same time, it is optionally possible for two R substituents bonded to the same carbon atom or to adjacent carbon atoms to form a monocyclic or polycyclic, aliphatic, aromatic or heteroaromatic ring system which may be substituted by one or more R¹ radicals;
R¹ is the same or different at each instance and is selected from the group consisting of H, D, F, Cl, Br, I, CN, N(R²)₂, C(=O)R¹, a straight-chain alkyl group having 1 to 10 carbon atoms or a branched or cyclic alkyl group having 3 to 10 carbon atoms or an alkenyl group having 2 to 10 carbon atoms, each of which may be substituted by one or more R² radicals, where one or more hydrogen atoms may be replaced by D, F, Cl, Br, I or CN, or an aromatic or heteroaromatic ring system which has 5 to 40 aromatic ring atoms and may be substituted in each case by one or more R² radicals; at the same time, it is optionally possible for two R¹ substituents bonded to the same carbon atom or to adjacent carbon atoms to form a monocyclic or polycyclic, aliphatic, aromatic or heteroaromatic ring system which may be substituted by one or more R² radicals;
R² is the same or different at each instance and is selected from the group consisting of H, D, F, CN, an aliphatic hydrocarbyl radical having 1 to 20 carbon atoms, or an aromatic or heteroaromatic ring system having 5 to 30 aromatic ring atoms in which one or more hydrogen atoms may be replaced by D, F, CN and which may be substituted by one or more alkyl groups each having 1 to 4 carbon atoms; at the same time, it is possible for two or more adjacent R² substituents together to form a mono- or polycyclic, aliphatic ring system.

2. Compound according to Claim 1, selected from the compounds of the formulae (7a), (7b), (7c), (7d) and (7e) 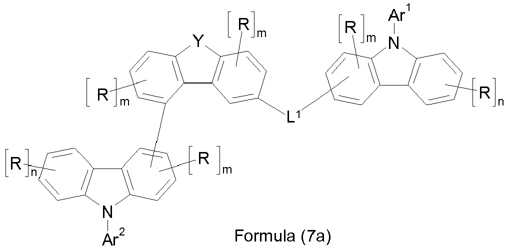 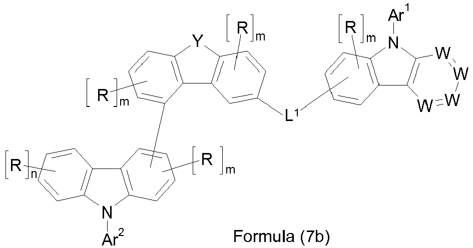 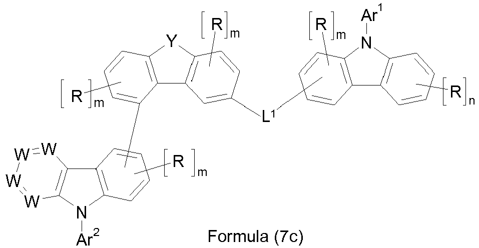 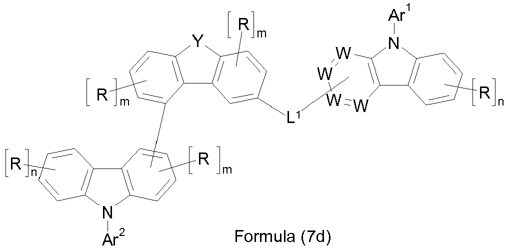 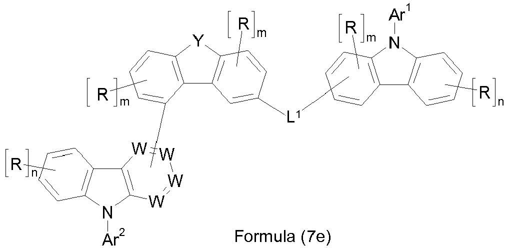 where:
W two adjacent W groups together are a group of the following formula (2a) or (3a) and the two other W groups are CR, where W in formula (7d) is C when an L¹ group is bonded to this position, and in formula (7e) is C when the dibenzofuran or dibenzothiophene is bonded to this position: 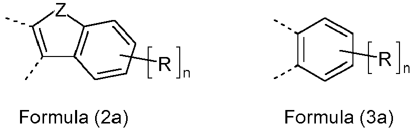 where the dotted bonds indicate the linkage of this group;
n is the same or different at each instance and is 0, 1, 2, 3 or 4;
m is the same or different at each instance and is 0, 1, 2 or 3;
the further symbols used have the definitions given in Claim 1.

3. Compound according to Claim 1 or 2, **characterized in that** L¹ and L² are single bonds.

4. Compound according to one or more of Claims 1 to 3, selected from the compounds of the formulae (9a), (9b), (9c), (9d) and (9e) 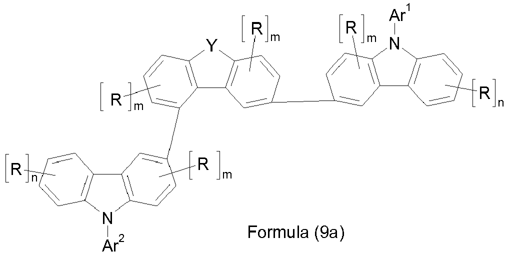 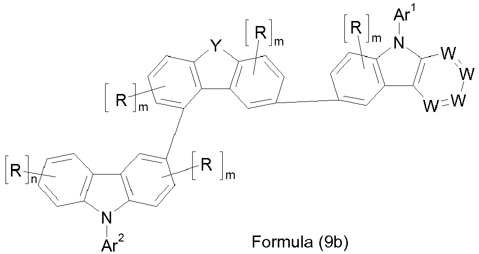 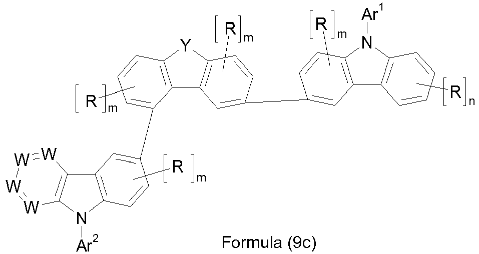 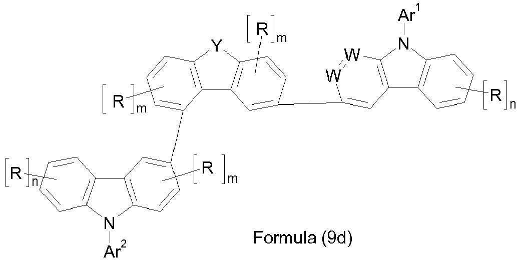 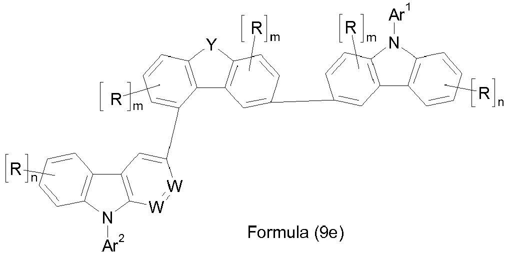 where W, n and m have the definitions given in Claim 2 and the further symbols used those given in Claim 1.

5. Compound according to one or more of Claims 1 to 4, **characterized in that** the group of the formula (4) is selected from the structures of the formulae (4-1) to (4-3) and that the group of the formula (5) is selected from the structures of the formulae (5-1) to (5-18) 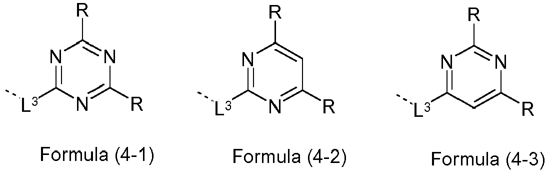 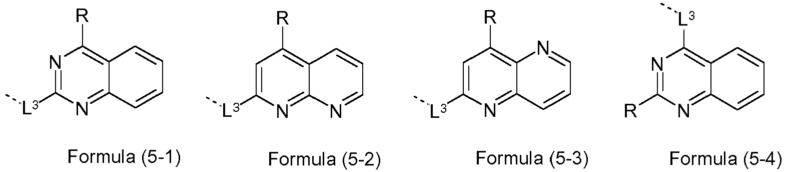 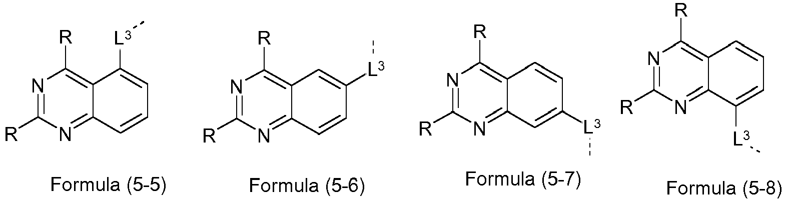 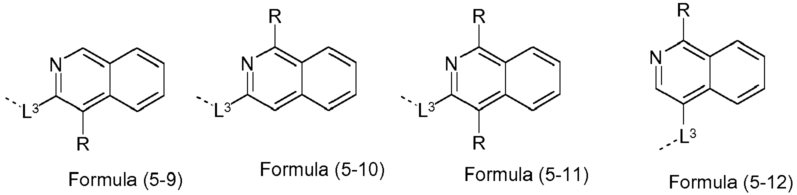 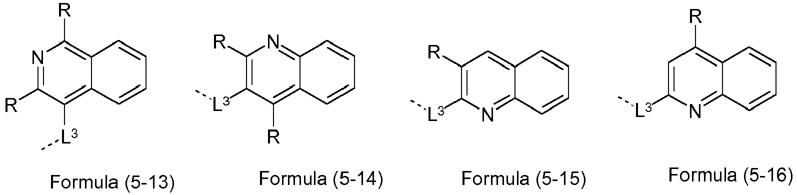 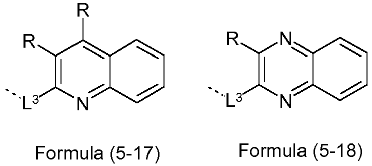 where the dotted bond represents the bond to the nitrogen atom and in addition:
R is the same or different at each instance and is H, an alkyl group which has 1 to 10 carbon atoms and may be substituted by one or more R¹ groups, or an aromatic or heteroaromatic ring system which has 5 to 40 aromatic ring atoms and may be substituted in each case by one or more R¹ radicals.

6. Compound according to one or more of Claims 1 to 5, **characterized in that** L³ is a single bond or an aromatic ring system having 6 to 12 aromatic ring atoms.

7. Compound according to one or more of Claims 1 to 6, **characterized in that** the Ar¹ or Ar² group which is not a group of the formula (4) or (5) is selected from the group consisting of phenyl, ortho-, meta- or para-biphenyl, terphenyl, quaterphenyl, 1-, 2-, 3- or 4-fluorenyl, 1-, 2-, 3- or 4-spirobifluorenyl, 1-, 2-, 3- or 4-dibenzofuranyl and 1-, 2-, 3- or 4-dibenzothienyl, each of which may be substituted by one or more nonaromatic R radicals.

8. Compound according to one or more of Claims 2 to 7, **characterized in that** the index n is the same or different at each instance and is 0, 1, 2 or 3, preferably 0 or 1, and **in that** the index m is the same or different at each instance and is 0, 1 or 2, preferably 0.

9. Compound according to one or more of Claims 1 to 8, **characterized in that** R is selected from the group consisting of H, D, F, CN, N(R¹)₂, C(=O)R¹, P(=O)(R¹)₂, a straight-chain alkyl or alkoxy group having 1 to 10 carbon atoms or a branched or cyclic alkyl or alkoxy group having 3 to 10 carbon atoms or an alkenyl group having 2 to 10 carbon atoms, each of which may be substituted by one or more R¹ radicals, where one or more nonadjacent CH₂ groups may be replaced by O and where one or more hydrogen atoms may be replaced by D or F, an aromatic or heteroaromatic ring system which has 5 to 24 aromatic ring atoms and may be substituted in each case by one or more R¹ radicals; at the same time, it is optionally possible for two R substituents bonded to the same carbon atom or to adjacent carbon atoms to form a monocyclic or polycyclic, aliphatic, aromatic or heteroaromatic ring system which may be substituted by one or more R¹ radicals.

10. Compound according to one or more of Claims 1 to 9, selected from the compounds of the formulae (10) and (11) 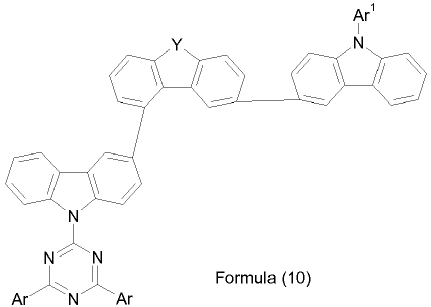 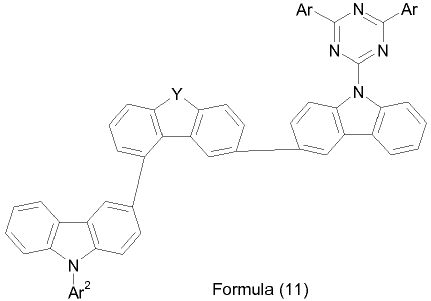 where the symbols used have the definitions given in Claim 1 and Ar is the same or different at each instance and is an aromatic or heteroaromatic ring system which has 5 to 40 aromatic ring atoms and may be substituted by one or more R¹ radicals.

11. Process for preparing a compound according to one or more of Claims 1 to 10 by reacting an optionally substituted 1,8-dihalodibenzofuran or 1,8-dihalodibenzothiophene with a carbazole derivative, followed by reaction with the other carbazole derivative, where the reactions with the carbazole derivatives are each C-C couplings.

12. Formulation comprising at least one compound according to one or more of Claims 1 to 10 and a solvent and/or at least one further organic or inorganic compound.

13. Use of a compound according to one or more of Claims 1 to 10 in an electronic device.

14. Electronic device, preferably selected from the group consisting of organic electroluminescent devices, organic integrated circuits, organic field-effect transistors, organic thin-film transistors, organic light-emitting transistors, organic solar cells, organic dye-sensitized solar cells, organic optical detectors, organic photoreceptors, organic field-quench devices, light-emitting electrochemical cells, organic laser diodes and organic plasmon emitting devices, comprising at least one compound according to one or more of Claims 1 to 10.

15. Electronic device according to Claim 14, **characterized in that** it is an organic electroluminescent device and the compound according to one or more of Claims 1 to 10 is used as matrix material for fluorescent or phosphorescent emitters in an emitting layer and/or as electron transport or hole blocker material in an electron transport layer and/or in a hole-blocking layer.

## Revendications

1. Composé selon la formule (1), 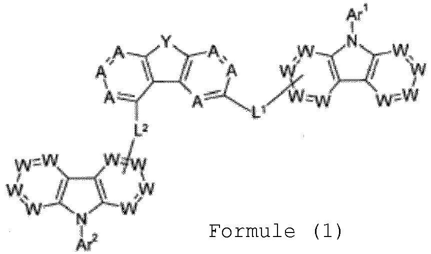 où, pour les symboles utilisés, ce qui suit est d'application :
A identique ou différent en chaque occurrence, représente CR ou N, au maximum 2 groupes A par cycle représentant N ;
Y représente O ou S ;
W identique ou différent en chaque occurrence, représente CR ou N, au maximum 2 groupes W par cycle représentant N et W représentant C lorsqu'un groupe L¹ ou, selon le cas, L² est lié à cette position, ou deux groupes W adjacents représentant ensemble un groupe de la formule suivante (2) ou (3), chacun des deux groupes de dérivé de carbazolyle dans le composé de la formule (1) présentant au maximum deux groupes de la formule (2) ou, selon le cas, de la formule (3), 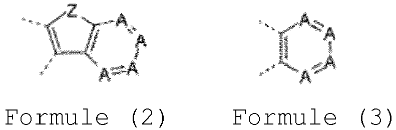 les liaisons en pointillé indiquant la liaison de ce groupe, A présentant les significations mentionnées ci-dessus et Z représentant NR, CR₂, O ou S ;
Ar¹, Ar² représente un système cyclique aromatique comportant 5 jusqu'à 30 atomes de cycle aromatiques ou un groupe dibenzofuranne ou un groupe dibenzothiophène, le système cyclique aromatique ou, selon le cas, le groupe dibenzofuranne ou le groupe dibenzothiophène pouvant à chaque fois être substitué par un ou plusieurs radical/radicaux R non aromatique(s), ou représente un groupe selon l'une des formules suivantes (4) ou (5), 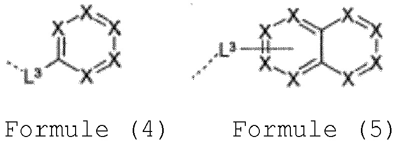 la liaison en pointillé représentant la liaison à l'atome d'azote ;
étant entendu que précisément un des groupes Ar¹ ou Ar² représente un groupe selon l'une des formules (4) ou (5) ;
X identique ou différent en chaque occurrence, représente CR ou N, X représentant C dans la formule (5) lorsque le groupe de la formule (5) est lié à L³ en cette position, étant entendu que deux ou trois groupes X représentent N dans la formule (4) et qu'un, deux ou trois groupe(s) X représente(nt) N dans la formule (5) ;
L¹, L², L³, identiques ou différents en chaque occurrence, représentent une simple liaison ou un système cyclique aromatique ou hétéroaromatique comprenant 5 jusqu'à 30 atomes de cycle aromatiques, qui peut être substitué par un ou plusieurs radical/radicaux R ;
R identique ou différent en chaque occurrence, est choisi dans le groupe constitué par H, D, F, Cl, Br, I, CN, NO₂, N(R¹)₂, C(=O)R¹, P(=O)(R¹)₂P(R¹)₂, B(R¹)₂, Si(R¹)₃, un groupe linéaire alkyle, alcoxyle ou thioalkyle comprenant 1 jusqu'à 20 atome(s) de C ou un groupe alkyle, alcoxyle ou thioalkyle, ramifié ou cyclique, comprenant 3 jusqu'à 20 atomes de C ou un groupe alcényle comprenant 2 jusqu'à 20 atomes de C, qui peut à chaque fois être substitué par un ou plusieurs radical/radicaux R¹, un ou plusieurs groupe(s) CH₂ non adjacents pouvant être remplacé(s) par R¹C=CR¹, Si(R¹)₂, C=O, C=S, C=NR¹, P(=O)(R¹), SO, SO₂, NR¹, O, S ou CONR¹ et un ou plusieurs atome (s) de H pouvant être remplacé(s) par D, F, Cl, Br, I, CN ou NO₂, un système cyclique aromatique ou hétéroaromatique comprenant 5 jusqu'à 40 atomes de cycle aromatiques, qui peut à chaque fois être substitué par un ou plusieurs radical/radicaux R¹, ou un groupe aryloxyle ou hétéroaryloxyle comprenant 5 jusqu'à 40 atomes de cycle aromatiques, qui peut à chaque fois être substitué par un ou plusieurs radical/radicaux R¹ ; deux substituants R, qui sont liés au même atome de carbone ou à des atomes de carbone adjacents, pouvant éventuellement former un système cyclique monocyclique ou polycyclique, aliphatique, aromatique ou hétéroaromatique, qui peut être substitué par un ou plusieurs radical/radicaux R¹ ;
R¹ identique ou différent en chaque occurrence, est choisi dans le groupe constitué par H, D, F, Cl, Br, I, CN, N(R²)₂, C(=O)R¹, un groupe alkyle linéaire comprenant 1 jusqu'à 10 atome(s) de C ou un groupe alkyle ramifié ou cyclique comprenant 3 jusqu'à 10 atomes de C ou un groupe alcényle comprenant 2 jusqu'à 10 atomes de C, qui peut à chaque fois être substitué par un ou plusieurs radical/radicaux R², un ou plusieurs atome(s) de H pouvant être remplacé(s) par D, F, Cl, Br, I ou CN, ou un système cyclique aromatique ou hétéroaromatique comprenant 5 jusqu'à 40 atomes de cycle aromatiques, qui peut à chaque fois être substitué par un ou plusieurs radical/radicaux R² ; deux substituants R¹, qui sont liés au même atome de carbone ou à des atomes de carbone adjacents, pouvant éventuellement former un système cyclique monocyclique ou polycyclique, aliphatique, aromatique ou hétéroaromatique, qui peut être substitué par un ou plusieurs radical/radicaux R² ;
R² identique ou différent en chaque occurrence, est choisi dans le groupe constitué par H, D, F, CN, un radical hydrocarboné aliphatique comprenant 1 jusqu'à 20 atome(s) de C ou un système cyclique aromatique ou hétéroaromatique comprenant 5 jusqu'à 30 atomes de cycle aromatiques, dans lequel un ou plusieurs atome(s) de H peut/peuvent être substitué(s) par D, F, CN et qui peut être substitué par un ou plusieurs groupe(s) alkyle comprenant à chaque fois 1 jusqu'à 4 atomes(s) de carbone ; deux substituants R² adjacents ou plus pouvant former ensemble un système cyclique aliphatique monocyclique ou polycyclique.

2. Composé selon la revendication 1, choisi parmi les composés des formules (7a), (7b), (7c), (7d) et (7e), 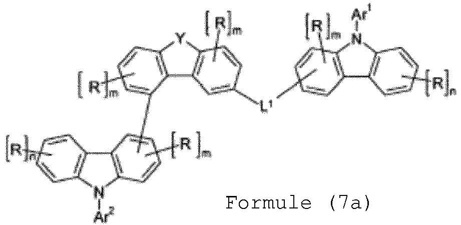 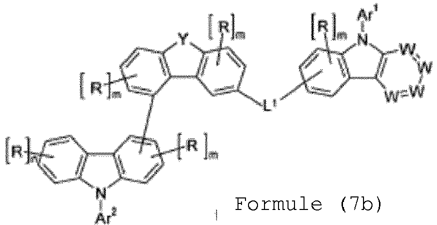 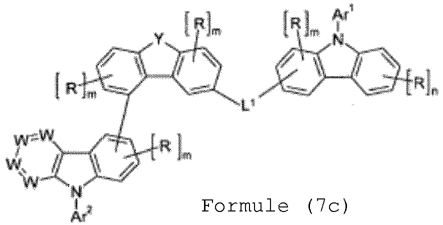 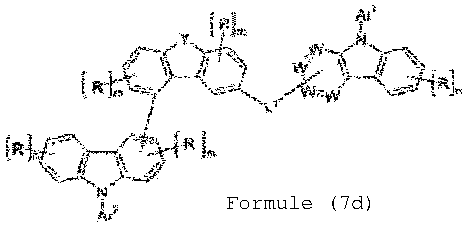 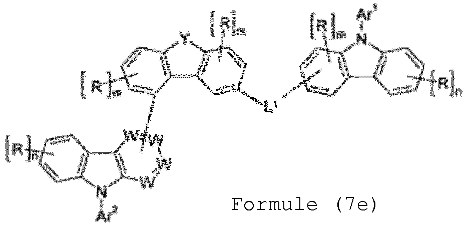 où ce qui suit est d'application
W deux groupes W adjacents représentent ensemble un groupe de la formule suivante (2a) ou (3a) et les deux autres groupes W représentent CR, W représentant C dans la formule (7d), lorsqu'un groupe L¹ est lié à cette position, et représente C dans la formule (7e), lorsque le dibenzofuranne ou, selon le cas, le dibenzothiophène est lié à cette position, 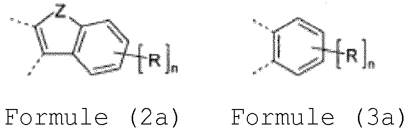
n les liaisons en pointillé indiquant la liaison de ce groupe ; identique ou différent en chaque occurrence, est 0, 1, 2, 3 ou 4 ;
m identique ou différent en chaque occurrence, est 0, 1, 2 ou 3 ;
les autres symboles utilisés présentant les significations mentionnées dans la revendication 1.

3. Composé selon la revendication 1 ou 2, **caractérisé en ce que** L¹ et L² représentent des simples liaisons.

4. Composé selon l'une ou plusieurs des revendications 1 à 3, choisi parmi les composés des formules (9a), (9b), (9c), (9d) et (9e), 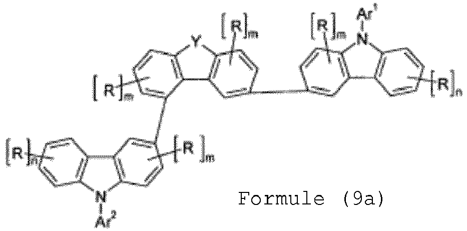 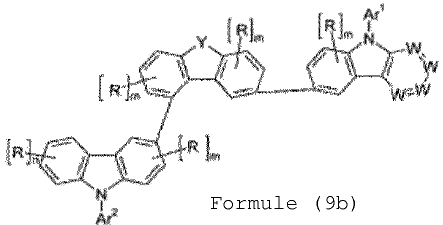 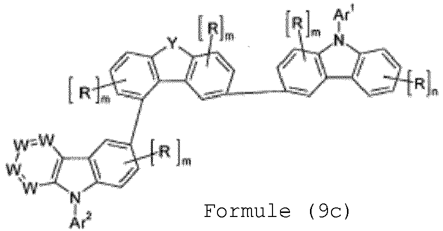 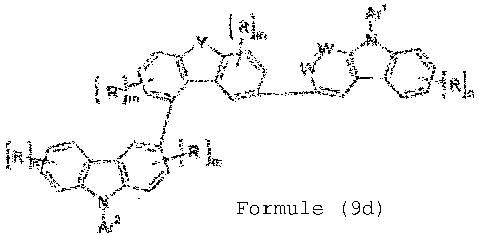 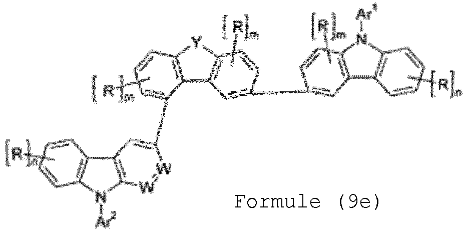 W, n et m présentant les significations mentionnées dans la revendication 2 et les autres symboles utilisés présentant les significations mentionnées dans la revendication 1.

5. Composé selon l'une ou plusieurs des revendications 1 à 4, **caractérisé en ce que** le groupe de la formule (4) est choisi parmi les structures des formules (4-1) jusqu'à (4-3) et **en ce que** le groupe de la formule (5) est choisi parmi les structures des formules (5-1) jusqu'à (5-18), 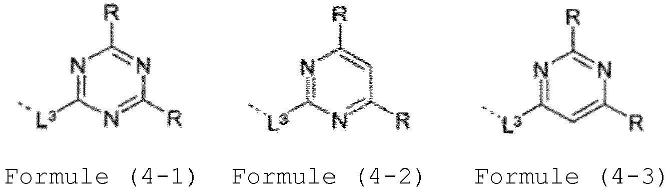 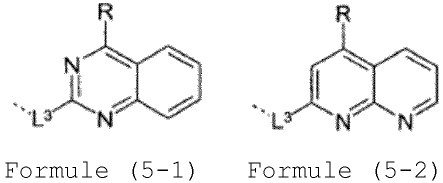 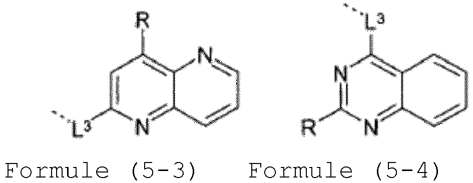 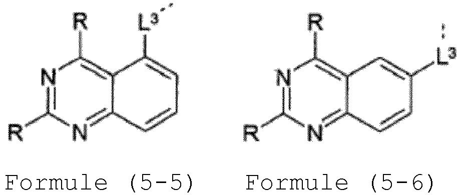 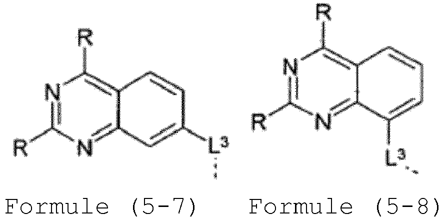 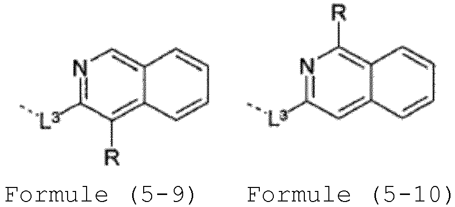 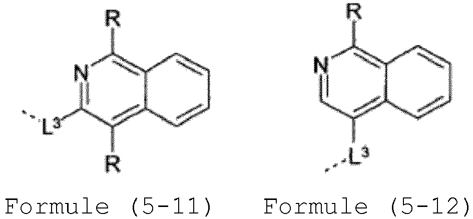 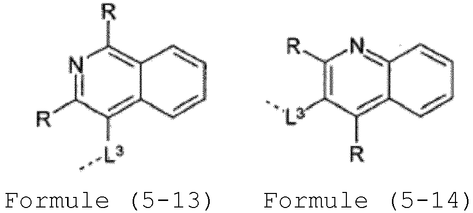 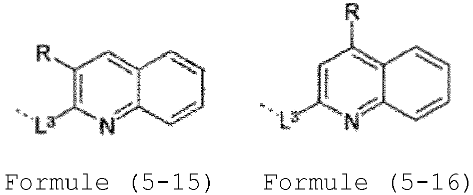 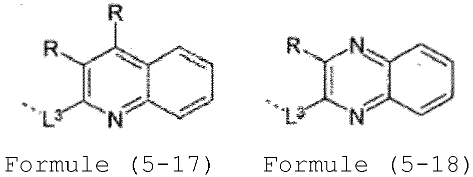 la liaison en pointillé représentant la liaison à l'atome d'azote et ce qui suit étant d'application :
R identique ou différent en chaque occurrence, représente H, un groupe alkyle comprenant 1 jusqu'à 10 atome(s) de C, qui peut être substitué par un ou plusieurs groupe(s) R¹, ou un système cyclique aromatique ou hétéroaromatique comprenant 5 jusqu'à 40 atomes de cycle aromatiques, qui peut à chaque fois être substitué par un ou plusieurs radical/radicaux R¹.

6. Composé selon l'une ou plusieurs des revendications 1 à 5, **caractérisé en ce que** L³ représente une simple liaison ou un système cyclique aromatique comprenant 6 jusqu'à 12 atomes de cycle aromatiques.

7. Composé selon l'une ou plusieurs des revendications 1 à 6, **caractérisé en ce que** le groupe Ar¹ ou, selon le cas, Ar², qui ne représente pas un groupe de la formule (4) ou (5), est choisi dans le groupe constitué par phényle, ortho-biphényle, méta-biphényle ou para-biphényle, terphényle, quaterphényle, 1-fluorényle, 2-fluorényle, 3-fluorényle ou 4-fluorényle, 1-spirobifluorényle, 2-spirobifluorényle, 3-spirobifluorényle ou 4-spirobifluorényle, 1-dibenzofurannyle, 2-dibenzofurannyle , 3-dibenzofurannyle ou 4-dibenzofurannyle et 1-dibenzothiényle, 2-dibenzothiényle, 3-dibenzothiényle ou 4-dibenzothiényle, qui peuvent être à chaque fois substitués par un ou plusieurs radical/radicaux R non aromatique(s).

8. Composé selon l'une ou plusieurs des revendications 2 à 7, **caractérisé en ce que** l'indice n, identique ou différent en chaque occurrence, est 0, 1, 2 ou 3, préférablement 0 ou 1, et **en ce que** l'indice m, identique ou différent en chaque occurrence, est 0, 1 ou 2, préférablement 0.

9. Composé selon l'une ou plusieurs des revendications 1 à 8, **caractérisé en ce que** R est choisi dans le groupe constitué par H, D, F, CN, N(R¹)₂, C(=O)R¹, P(=O)(R¹)₂, un groupe linéaire alkyle ou alcoxyle comprenant 1 jusqu'à 10 atome(s) de C ou un groupe ramifié ou cyclique alkyle ou alcoxyle comprenant 3 jusqu'à 10 atomes de C ou un groupe alcényle comprenant 2 jusqu'à 10 atomes de C, qui peut être à chaque fois substitué par un ou plusieurs radical/radicaux R¹, un ou plusieurs groupe(s) CH₂ non adjacents pouvant être remplacé(s) par O, et un ou plusieurs atome(s) de H pouvant être remplacé(s) par D ou F, un système cyclique aromatique ou hétéroaromatique comprenant 5 jusqu'à 24 atomes de cycle aromatiques, qui peut à chaque fois être substitué par un ou plusieurs radical/radicaux R¹ ; deux substituants R, qui sont liés au même atome de carbone ou à des atomes de carbone adjacents, pouvant éventuellement former un système cyclique monocyclique ou polycyclique, aliphatique, aromatique ou hétéroaromatique, qui peut être substitué par un ou plusieurs radical/radicaux R¹.

10. Composé selon l'une ou plusieurs des revendications 1 à 9, choisi parmi les composés selon les formules (10) et (11), 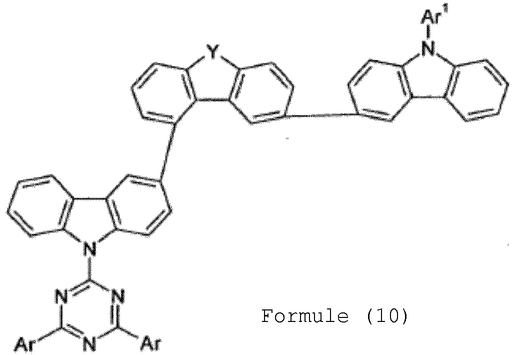 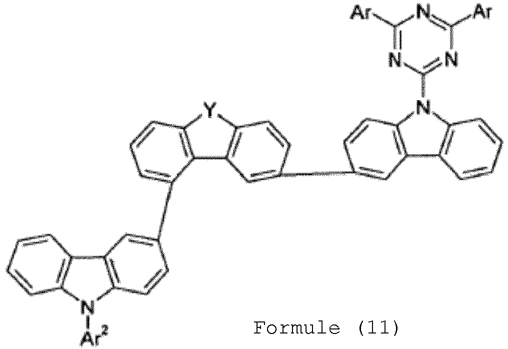 les symboles utilisés présentant les significations mentionnées dans la revendication 1 et Ar, identique ou différent en chaque occurrence, représentant un système cyclique aromatique ou hétéroaromatique comprenant 5 jusqu'à 40 atomes de cycle aromatiques, qui peut être substitué par un ou plusieurs radical/radicaux R¹.

11. Procédé pour la préparation d'un composé selon l'une ou plusieurs des revendications 1 à 10 par la transformation d'un 1,8-dihalogénodibenzofuranne ou, selon le cas, d'un 1,8-dihalogénodibenzothiophène, éventuellement substitués, avec un dérivé de carbazole, suivie par la transformation avec l'autre dérivé de carbazole, les transformations avec les dérivés de carbazole représentant à chaque fois des couplages C-C.

12. Formulation, contenant au moins un composé selon l'une ou plusieurs des revendications 1 à 10 et un solvant et/ou au moins un autre composé organique ou inorganique.

13. Utilisation d'un composé selon l'une ou plusieurs des revendications 1 à 10 dans un dispositif électronique.

14. Dispositif électronique, préférablement choisi dans le groupe constitué par les dispositifs organiques électroluminescents, les circuits organiques intégrés, les transistors organiques à effet de champ, les transistors organiques à film mince, les transistors organiques électroluminescents, les piles solaires organiques, les piles solaires organiques sensibilisées par un colorant, les détecteurs organiques optiques, les photorécepteurs organiques, les dispositifs organiques à extinction de champ, les cellules électrochimiques électroluminescentes, les diodes laser organiques et les dispositifs organiques émettant un plasmon (« organic plasmon emitting devices ») contenant au moins un composé selon l'une ou plusieurs des revendications 1 à 10.

15. Dispositif électronique selon la revendication 14, **caractérisé en ce qu'**il s'agit d'un dispositif organique électroluminescent et le composé selon l'une ou plusieurs des revendications 1 à 10 est utilisé en tant que matériau de matrice pour des émetteurs fluorescents ou phosphorescents dans une couche émettrice et/ou en tant que matériau de transport d'électrons ou de blocage de trous dans une couche de transport d'électrons et/ou dans une couche de blocage de trous.
